# EUROPEAN PATENT SPECIFICATION

(11) **EP 0 545 912 B1**
(45) Date of publication and mention of the grant of the patent: **25.04.2001**
(21) Application number: 93200195.1
(22) Date of filing: 21.02.1992
(51) Int. Cl.: C07D 233/90, A61K 31/415, C07D 405/12, C07D 405/14, C07D 403/10, C07F 7/18

(54) **1-Biphenylmethylimidazole derivatives, their preparation and their therapeutic use**
1-Biphenylimidazolderivate, Verfahren zu deren Herstellung und deren therapeutische Anwendung
Dérivés de 1-biphénylimidazole, leur préparation et leur utilisation thérapeutique

(30) Priority: 21.02.1991 JP 2709891; 26.04.1991 JP 9658891; 06.06.1991 JP 13488991; 08.07.1991 JP 16713891; 24.07.1991 JP 18484191; 15.07.1991 JP 17397291
(43) Date of publication of application: 09.06.1993
(62) Divisional of application: 92301449.2
(73) Proprietor: Sankyo Company Limited, Tokyo (JP)
(72) Inventor: Yanagisawa, Hiroaki, Shinagawa-ku, Tokyo 140 (JP); Shimoji, Yasuo, Shinagawa-ku, Tokyo 140 (JP); Fujimoto, Koichi, Shinagawa-ku, Tokyo 140 (JP); Kanazaki, Takuro, Shinagawa-ku, Tokyo 140 (JP); Amemiya, Yoshiya, Shinagawa-ku, Tokyo 140 (JP); Koike, Hiroyuki, Shinagawa-ku, Tokyo 140 (JP); Sada, Toshio, Shinagawa-ku, Tokyo 140 (JP)
(74) Representative: Gibson, Christian John Robert

(56) References cited:
- EP-A- 0 028 833
- EP-A- 0 028 834
- EP-A- 0 253 310
- EP-A- 0 323 841
- EP-A- 0 324 377
- EP-A- 0 401 030
- : "", J.MED.CHEM., WASHINGTON, 1990, vol. 33, no. , pages 1330 to

## Description

The present invention provides a series of novel 1-(biphenylmethyl)imidazole derivatives which have valuable hypotensive activities, and which may, therefore, be used in the treatment and prophylaxis of hypertension, including diseases of the heart and circulatory system. We also provide methods and compositions using these compounds, as well as processes for their preparation.

It is known that the renin-angiotension system provides one of the important mechanisms for maintaining the homeostasis of blood pressure in living animals. When blood pressure is reduced or the sodium ion concentration of the body fluids falls, this system is activated. As a result, the enzyme renin and angiotensin converting enzyme (hereinafter abbreviated, as is conventional, to "ACE") are activated and act on angiotensinogen, which is first decomposed by the renin to produce angiotensin I (hereinafter abbreviated to "AI"). This AI is then converted by ACE to angiotensin II (hereinafter abbreviated to "AII"). Since All induces strong contractions of blood vessels and accelerates the secretion of aldosterone, the activation of the system results in an elevation of blood pressure. Inhibitors or suppressors of the renin-angiotension system, such as renin inhibitors, ACE inhibitors and All antagonists, dilate blood vessels, cause lower blood pressure and improve the circulatory function, which is the basis for the use of these agents in the treatment of heart diseases.

At present only ACE inhibitors are used clinically, although renin inhibitors and All antagonists are under investigation for such use. Of these, some peptide type All antagonists, such as saralasin, have been known for many years, whilst certain non-peptide type antagonists have recently been discovered (for example, those compounds disclosed in European Patent Publications No. 28 833, 28 834, 245 637, 253 310, 323 841, 324 377, 380 959, 399 732, 399 731, 400 835 and 401030, in Japanese Patent Application Kokai No. Sho 57-98270)and in Carini et al., J. Med. Chem. 1990, 33, 1330-1336. Of these, the closest prior art is believed to be European Patent Publications No. 253 310 and 324 377.

European Patent Publication No. 253 310 discloses a series of 1-phenyl-, 1-phenethylor 1-benzylimidazole derivatives which are said to have the ability to inhibit the activity of AII. Included in the scope of these prior art compounds are a number of 1-biphenylmethylimidazole derivatives, which, however, differ from the compounds of the present invention in the nature of the substituent at the 4- or 5- position of the imidazole ring.

European Patent Publication No. 324 377 also discloses a series of such compounds. The activities of all of these prior art compounds, however, including those of European Patent Publications No. 253 310 and 324 377, are not thought to be sufficient and more potent All antagonists are sought for better clinical results.

We have now discovered a limited series of 1-(biphenylmethyl)imidazole-5-carboxylic acid derivatives having an excellent All receptor antagonist activity, and which are therefore useful as antihypertensive drugs and for the therapy and prophylaxis of heart diseases.

Thus, the present invention provides compounds of formula (I): 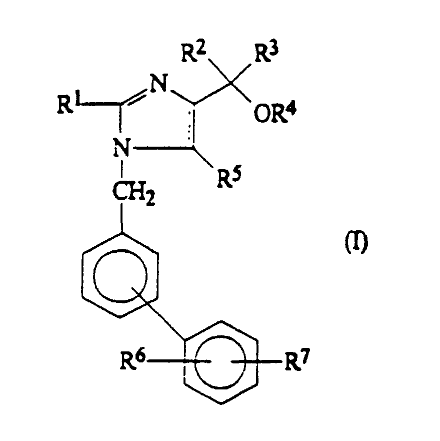 in which:
R¹ represents an alkyl group having from 2 to 5 carbon atoms;
R² and R³ each represent a hydrogen atom or one of R² and R³ represents a hydrogen atom, and the other represents an alkyl group having from 1 to 4 carbon atoms;
R⁴ represents a hydrogen atom, a methyl group, an ethyl group or an alkanoyl group having from 1 to 5 carbon atoms;
R⁵ represents a group of formula -C00R^{5a} or a group of formula -CONR⁸R⁹, in which:
   R^{5a} represents
   a hydrogen atom,
   a methyl, ethyl or benzyl group,
   an alkanoyloxymethyl group, in which the alkanoyl part has from 1 to 5 carbon atoms,
   a 1-(alkanoyloxy)ethyl group, in which the alkanoyl part has from 1 to 5 carbon atoms,
   an alkoxycarbonyloxymethyl group, in which the alkoxy part has from 1 to 4 carbon atoms,
   a 1-(alkoxycarbonyloxy)ethyl group, in which the alkoxy part has from 1 to 4 carbon atoms,
   a cycloalkanoyloxyalkyl group in which the cycloalkyl part has 5 or 6 carbon atoms and the alkyl part has 1 or 2 carbon atoms,
   a cycloalkoxycarbonyloxyalkyl group in which the cycloalkyl part has 5 or 6 carbon atoms and the alkyl part has 1 or 2 carbon atoms,
   a [5-(phenyl- or methyl-)-2-oxo-1,3-dioxolen-4-yl]methyl group, or
   a phthalidyl group;
R⁸ and R⁹ are the same or different and each represents a hydrogen atom, a methyl group, an ethyl group, a methoxycarbonylmethyl group, an ethoxy carbonylmethyl group or a carboxymethyl group; or R⁸ and R⁹ together represent a tetramethylene, pentamethylene, 1-carboxytetramethylene or 1-carboxypentamethylene group;
R⁶ represents a hydrogen atom, or it represents a methyl group, an methoxy group, a fluorine atom or a chlorine atom at the 6-position of the benzene ring;
R⁷ represents a carboxy group or a tetrazol-5-yl group at the 2-position of the benzene ring; and
the benzene ring which bears the substituents represented by R⁶ and R⁷ is at the 4-position of the benzyl group to which it is attached;
and pharmaceutically acceptable salts and esters thereof.

The invention also provides a pharmaceutical composition for the treatment or prophylaxis of hypertension, which comprises an effective amount of an anti-hypertensive agent in admixture with a pharmaceutically acceptable carrier or diluent, in which the anti-hypertensive agent is at least one compound of formula (I) or a pharmaceutically acceptable salt or ester thereof.

The invention further provides compounds of formula (I) and pharmaceutically acceptable salts and esters thereof for use in therapy, and still further provides the use of compounds of formula (I) and pharmaceutically acceptable salts and esters thereof for the manufacture of a medicament for the treatment or prophylaxis of hypertension in a mammal, e.g. a human being.

The invention still further provides processes for the preparation of compounds of formula (I) and pharmaceutically acceptable salts and esters thereof, which are described in more detail hereafter.

In the compounds of the present invention, where R¹ is an alkyl group, this is an alkyl group having from 2 to 5 carbon atoms, and where R² or R³ is an alkyl group having group having from 1 to 4 carbon atoms they may be straight or branched chain groups examples include the methyl, ethyl, propyl, isopropyl, butyl, isobutyl, sec-butyl, t-butyl, pentyl, t-pentyl, 2-methylbutyl, 3-methylbutyl and 1-ethylpropyl groups. R¹ preferably represents a straight chain group, i.e. most preferably an ethyl, propyl or butyl group. Each of R² and R³, which may be the same or different, more preferably represent a methyl, ethyl, propyl, isopropyl or t-butyl group, and most preferably a methyl or ethyl group when R⁵ represents a carboxy group, or an isopropyl or t-butyl group when R⁵ represents a group of formula -CONR⁸R⁹.

R⁴ can represent an alkanoyl group; such a group may be a straight or branched chain group and has from 1 to 5 carbon atoms. Examples of such groups include the formyl, acetyl, propionyl, butyryl, isobutyryl, pivaloyl, valeryl and isovaleryl groups, of which the formyl and acetyl groups are preferred.

Where R⁸ and R⁹ together represent a tetramethylene or pentamethylene group which may be substituted by a carboxy group, in such cases, the group of formula -R⁸R⁹ is a nitrogen-containing heterocyclic group having 5 or 6 ring atoms (one being the nitrogen atom), i.e. it is a 1-pyrrolidinyl or piperidino group, respectively.

R⁵ may represent a group of formula -COOR^{5a}, in which R^{5a} represents an ester residue as defined above (in the case of the carboxylic acid, R^{5a} represents a hydrogen atom). Where R⁵ represents a carboxy group, it can also form salts, examples of which are as exemplified below in relation to R⁷. It is well known in the art that certain ester residues confer advantages on compounds incorporating them, for example easier or better absorption in vivo.

Examples of the ester group R^{5a} include:
alkanoyloxymethyl and 1-(alkanoyloxy)ethyl groups in which the alkanoyl part has from 1 to 5 carbon atoms which groups include formoyloxymethyl, acetoxymethyl, propionyloxymethyl, butyryloxymethyl, pivaloyloxymethyl, valeryloxymethyl, isovaleryloxymethyl, 1-(formoyloxy)ethyl, 1-(acetoxy)ethyl, 1-(propionyloxy) ethyl, 1-(butyryloxy)ethyl, 1-(pivaloyloxy)ethyl, 1-(valeryloxy)ethyl and 1-(isovaleryloxy)ethyl groups; and more preferably the acetoxymethyl, propionyloxymethyl, butyryloxymethyl, pivaloyloxymethyl, 1-(acetoxy)ethyl, 1-(propionyloxy) ethyl, 1-(butyryloxy) ethyl and 1-(pivaloyloxy)ethyl; and most preferably the pivaloyloxymethyl and 1-(pivaloyloxy)ethyl groups;
cycloalkanoyloxyalkyl groups in which the cycloalkyl part has 5 or 6 carbon atoms and the alkyl part has 1 or 2 carbon atoms, which groups include cyclopentanoyloxymethyl, cyclohexanoyloxymethyl, 1-(cyclopentanoyloxy)ethyl and 1-(cyclohexanoyloxy)ethyl groups;
alkoxycarbonyloxymethyl and 1-(alkoxycarbonyloxy)ethyl groups in which the alkyl part has from 1 to 4 carbon atoms, as exemplified above in relation to R¹, and preferably the alkyl part has 1 or 2 carbon atoms; preferred examples include the methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, 1-(methoxycarbonyloxy) ethyl, 1-(ethoxycarbonyloxy)ethyl, 1-(propoxycarbonyloxy) ethyl, 1- (isopropoxycarbonyloxy) ethyl, 1-(butoxycarbonyloxy)ethyl, and 1-(isobutoxycarbonyloxy)ethyl groups; and most preferably the methoxycarbonyloxymethyl, ethoxycarbonyloxymethyl, isopropoxycarbonyloxymethyl, 1-(methoxycarbonyloxy) ethyl, 1-(ethoxycarbonyloxy) ethyl, and 1-(isopropoxycarbonyloxy)ethyl groups;
cycloalkoxycarbonyloxyalkyl groups in which the cycloalkyl part has 5 or 6 carbon atoms and the alkyl part has 1 or 2 carbon atoms, which include cyclopentoxycarbonyloxymethyl, cyclohexyloxycarbonyloxymethyl, 1-(cyclopentoxy-carbonyloxy)ethyl, and 1-(cyclohexyloxycarbonyloxy)ethyl groups.

The benzene ring which bears the substituents represented by R⁶ and R⁷ is at the 4- position of the benzyl group to which it attaches, i.e. the compounds of the invention have the formula (Ia):

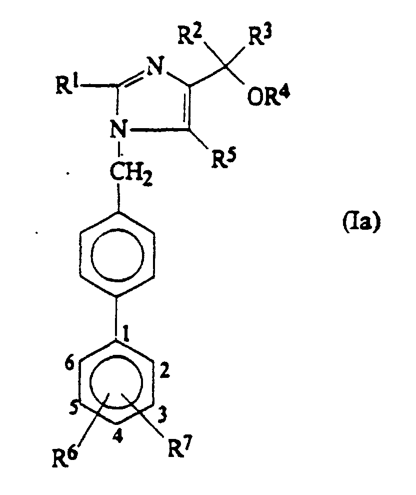

R⁷ may represent a carboxy group or a tetrazol-5- yl group. When it represents a carboxy group, the resulting compounds may form salts or esters. There is no particular restriction on the nature of these salts or esters, provided that, where they are intended for therapeutic use, they are pharmaceutically acceptable i.e. it should not have increased, or unacceptably increased, toxicity or reduced, or unacceptably reduced, activity, as compared with the parent acid. Where they are intended for non-therapeutic uses, e.g. as intermediates in the preparation of other, and possibly more active, compounds, even this restriction does not apply. Examples of such salts include: salts with an alkali metal, such as sodium, potassium or lithium; salts with an alkaline earth metal, such as barium or calcium; salts with another metal, such as magnesium and aluminium; organic base salts, such as a salt with guanidine, triethylamine, dicyclohexylamine; and salts with a basic amino acid, such as lysine or arginine. Examples of ester groups may be those ester groups defined above in relation to R^{5a}.

Preferably R⁷ represents a carboxy group or a tetrazol-5-yl group, and, where R⁷ represents a carboxy group, salts of these compounds are also preferred. R⁷ is at the 2- position of the phenyl group.

The compounds of the present invention necessarily contain at least one basic nitrogen atom in the imidazole ring and can therefore form acid addition salts. Examples of such acid addition salts include: addition salts with inorganic acids, such as hydrochloric acid, hydrobromic acid, sulphuric acid or phosphoric acid; and addition salts with organic acids such as maleic acid, fumaric acid, tartaric acid or citric acid.

Preferred classes of compounds of the present invention are those compounds of formula (I) and salts and esters thereof, in which:
either
R¹ represents an ethyl, propyl or butyl group;
R² represents a hydrogen atom or a methyl group;
R³ represents a hydrogen atom;
R⁴ represents a hydrogen atom or a methyl group;
R⁵ represents a group of formula -COOR^{5a}, in which R^{5a} represents a hydrogen atom, a pivaloyloxymethyl group, an ethoxycarbonyloxymethyl group, a 1-(ethoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group, or a phthalidyl group;
R⁶ represents a hydrogen atom;
R⁷ represents a carboxy group or a tetrazol-5-yl group at the 2-position of the benzene ring; and
the benzene ring which bears the substituents represented by R⁶ and R⁷ is at the 4-position of the benzyl group to which it is attached.
or
R¹ represents an ethyl, propyl or butyl group;
R² represents an isopropyl group or a t-butyl group;
R³ represents a hydrogen atom;
R⁴ represents a hydrogen atom or a methyl group;
R⁵ represents a group of formula -CONR⁸R⁹, in which R⁸ and R⁹ are the same or different and each represents a hydrogen atom, a methyl group, a methoxycarbonylmethyl group, an ethoxycarbonylmethyl group, or a carboxymethyl group;
R⁶ represents a hydrogen atom;
R⁷ represents a carboxy group or a tetrazol-5-yl group at the 2-position of the benzene ring; and
the benzene ring which bears the substituents represented by R⁶ and R⁷ is at the 4-position of the benzyl group to which it is attached.

The compounds of the present invention may contain one or more asymmetric carbon atoms in their molecules, and can thus form optical isomers. Although these are all represented herein by a single molecular formula, the present invention includes both the individual, isolated isomers and mixtures, including racemates thereof. Where stereospecific synthesis techniques are employed or optically active compounds are employed as starting materials, individual isomers may be prepared directly; on the other hand, if a mixture of isomers is prepared, the individual isomers may be obtained by conventional resolution techniques.

Specific examples of individual compounds of the present invention are shown in the following formulae (I-1), (I-4) and (I-5):

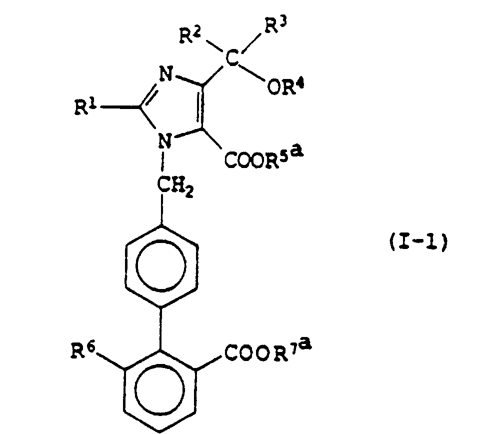

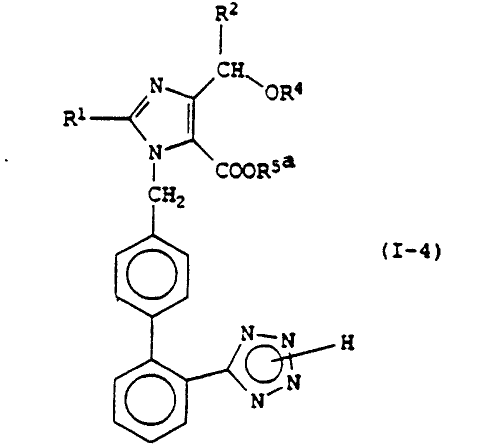

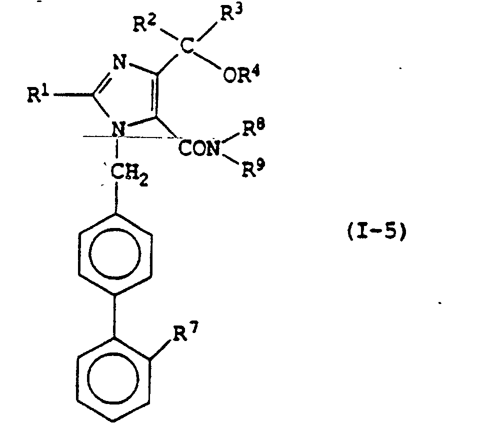

In these formulae, the meanings of the various substituent groups are as given in the following Tables 1, 4 and 5 in which Table 1 relates to formula (I-1), Table 4 relates to formula (I-4) and Table 5 relates to formula (I-5). In the Tables, the following abbreviations are used:

| | |
|---|---|
| Ac | acetyl |
| Bu | butyl |
| iBu | isobutyl |
| tBu | t-butyl |
| Bz | benzyl |
| Et | ethyl |
| Etc | ethoxycarbonyl |
| Fo | formyl |
| Fu | 2-furyl |
| Me | methyl |
| Mec | methoxycarbonyl |
| Mod | (5-methyl-2-oxo-1,3-dioxolen-4-yl)methyl |
| Ph | phenyl |
| Phth | phthalidyl |
| Piv | pivaloyl |
| Pn | pentyl |
| iPn | isopentyl |
| Pr | propyl |
| iPr | isopropyl |
| iPrc | isopropoxycarbonyl |
| Prn | propionyl |
| Tz | tetrazol-5-yl |

**Table 1**

| Cpd. No. | R¹ | R² | R³ | R⁴ | R^{5a} | R⁶ | R^{7a} |
|---|---|---|---|---|---|---|---|
| 1-1 | Pr | H | H | H | H | H | H |
| 1-2 | Bu | H | H | H | H | H | H |
| 1-4 | Pn | H | H | H | H | H | H |
| 1-5 | Bu | H | H | H | Me | H | H |
| 1-6 | Bu | H | H | H | Et | H | H |
| 1-7 | Bu | H | H | H | Bu | H | H |
| 1-8 | Bu | H | H | H | Bz | H | H |
| 1-9 | Bu | H | H | Me | H | H | H |
| 1-10 | Bu | H | H | Et | H | H | H |
| 1-11 | Bu | H | H | Fo | H | H | H |
| 1-12 | Bu | H | H | Ac | H | H | H |
| 1-14 | Bu | H | H | Me | Et | H | H |
| 1-15 | Bu | H | H | Me | PivOCH₂- | H | H |
| 1-16 | Bu | H | H | H | H | Cl | H |
| 1-17 | Bu | H | H | H | Et | Cl | H |
| 1-18 | Bu | H | H | H | H | OMe | H |
| 1-19 | Bu | H | H | H | Et | OMe | H |
| 1-22 | Bu | H | H | H | Mod | H | H |
| 1-23 | Bu | H | H | H | EtcOCH₂- | H | H |
| 1-24 | Bu | H | H | H | 1-(EtcO)Et | H | H |
| 1-25 | Bu | Me | H | H | H | H | H |
| 1-26 | Bu | Me | H | H | Et | H | H |
| 1-27 | Bu | Me | H | H | PivOCH₂- | H | H |
| 1-28 | Bu | Me | H | H | Mod | H | H |
| 1-29 | Bu | Me | H | Ac | H | H | H |
| 1-30 | Bu | Me | H | Ac | Et | H | H |
| 1-56 | Et | Me | H | H | H | H | H |
| 1-57 | Et | Me | H | H | Et | H | H |
| 1-58 | Et | Me | H | H | PivOCH₂- | H | H |
| 1-59 | Et | Me | H | H | Mod | H | H |
| 1-60 | Et | Me | H | H | EtcOCH₂- | H | H |
| 1-61 | Et | Me | H | H | 1-(EtcO)Et | H | H |
| 1-62 | Bu | Et | H | H | H | H | H |
| 1-63 | Bu | Et | H | H | Et | H | H |
| 1-64 | Bu | Et | H | H | H | Cl | H |
| 1-65 | Bu | Et | H | H | Et | Cl | H |
| 1-66 | Bu | Et | H | H | H | OMe | H |
| 1-67 | Bu | Et | H | H | Et | OMe | H |
| 1-68 | Bu | iPr | H | H | H | H | H |
| 1-69 | Bu | iPr | H | H | Et | H | H |
| 1-70 | Bu | iPr | H | H | H | Cl | H |
| 1-71 | Bu | iPr | H | H | Et | Cl | H |
| 1-72 | Bu | iPr | H | H | H | OMe | H |
| 1-73 | Bu | iPr | H | H | Et | OMe | H |
| 1-74 | Bu | tBu | H | H | H | H | H |
| 1-75 | Bu | tBu | H | H | Et | H | H |
| 1-76 | Bu | tBu | H | H | H | Cl | H |
| 1-77 | Bu | tBu | H | H | Et | Cl | H |
| 1-78 | Bu | tBu | H | H | H | OMe | H |
| 1-79 | Bu | tBu | H | H | Et | OMe | H |
| 1-90 | Bu | Pr | H | H | H | H | H |
| 1-91 | Bu | Pr | H | H | Et | H | H |
| 1-92 | Pr | Pr | H | H | H | H | H |
| 1-93 | Pr | Pr | H | H | Et | H | H |
| 1-94 | Bu | H | H | H | Me | H | tBu |
| 1-95 | Bu | H | H | H | Et | H | tBu |
| 1-96 | Bu | H | H | H | H | H | tBu |
| 1-97 | Bu | H | H | H | PivOCH₂- | H | tBu |
| 1-98 | Bu | H | H | H | PivOCH₂- | H | H |
| 1-99 | Bu | H | H | Me | Me | H | tBu |
| 1-100 | Pr | H | H | H | Et | H | H |
| 1-101 | Pr | H | H | H | Bu | H | H |
| 1-102 | Pr | H | H | H | PivOCH₂- | H | H |
| 1-103 | Pr | H | H | H | Mod | H | H |
| 1-104 | Pr | H | H | H | H | Cl | H |
| 1-105 | Pr | H | H | H | Et | Cl | H |
| 1-106 | Pr | H | H | H | H | OMe | H |
| 1-107 | Pr | H | H | H | Et | OMe | H |
| 1-121 | Bu | H | H | Me | Me | H | H |
| 1-133 | Pr | Me | H | H | PivOCH₂- | H | H |
| 1-134 | Pr | Me | H | H | Mod | H | H |
| 1-135 | Pr | Me | H | H | EtcOCH₂- | H | H |
| 1-136 | Pr | Me | H | H | 1-(EtcO)Et | H | H |
| 1-137 | Pr | Me | H | H | Phth | H | H |
| 1-138 | Et | H | H | H | H | H | H |
| 1-139 | Et | H | H | H | PivOCH₂- | H | H |
| 1-140 | Et | H | H | H | Mod | H | H |
| 1-141 | Et | H | H | H | EtcOCH₂- | H | H |
| 1-142 | Et | H | H | H | 1-(EtcO)Et | H | H |
| 1-143 | Et | H | H | H | Phth | H | H |

**Table 4**

| Cpd. No. | R¹ | R² | R⁴ | R^{5a} |
|---|---|---|---|---|
| 4-1 | Pr | H | H | H |
| 4-2 | Pr | H | H | Me |
| 4-3 | Pr | H | H | Et |
| 4-4 | Pr | H | H | PivOCH₂- |
| 4-5 | Pr | H | H | Mod |
| 4-6 | Pr | H | H | EtcOCH₂- |
| 4-7 | Pr | H | H | iPrcOCH₂- |
| 4-8 | Pr | H | H | 1-(EtcO)Et |
| 4-9 | Pr | H | H | 1-(iPrcO)Et |
| 4-10 | Pr | H | H | Phth |
| 4-11 | Pr | H | Me | H |
| 4-12 | Pr | H | Me | Me |
| 4-13 | Pr | H | Me | Et |
| 4-14 | Pr | H | Me | PivOCH₂- |
| 4-15 | Pr | H | Me | Mod |
| 4-16 | Pr | H | Me | EtcOCH₂- |
| 4-17 | Pr | H | Me | iPrcOCH₂- |
| 4-18 | Pr | H | Me | 1-(EtcO)Et |
| 4-19 | Pr | H | Me | 1-(iPrcO)Et |
| 4-20 | Pr | H | Me | Phth |
| 4-21 | Pr | H | Fo | H |
| 4-22 | Pr | H | Fo | PivOCH₂- |
| 4-23 | Pr | H | Fo | Mod |
| 4-24 | Pr | H | Fo | Phth |
| 4-25 | Pr | H | Ac | H |
| 4-26 | Pr | H | Ac | PivOCH₂- |
| 4-27 | Pr | H | Ac | Mod |
| 4-28 | Pr | H | Ac | Phth |
| 4-29 | Pr | Me | H | H |
| 4-30 | Pr | Me | H | Et |
| 4-31 | Pr | Me | H | PivOCH₂- |
| 4-32 | Pr | Me | H | Mod |
| 4-33 | Pr | Me | H | EtcOCH₂- |
| 4-34 | Pr | Me | H | iPrcOCH₂- |
| 4-35 | Pr | Me | H | Phth |
| 4-36 | Pr | Me | Me | H |
| 4-37 | Pr | Me | Me | Et |
| 4-38 | Pr | Me | Me | PivOCH₂- |
| 4-39 | Pr | Me | Me | Mod |
| 4-40 | Pr | Me | Me | Phth |
| 4-41 | Pr | Et | H | H |
| 4-42 | Pr | Et | H | Et |
| 4-43 | Pr | Et | H | PivOCH₂- |
| 4-44 | Pr | Et | H | Mod |
| 4-45 | Pr | Et | H | Phth |
| 4-46 | Bu | H | H | H |
| 4-47 | Bu | H | H | Me |
| 4-48 | Bu | H | H | Et |
| 4-49 | Bu | H | H | PivOCH₂- |
| 4-50 | Bu | H | H | Mod |
| 4-51 | Bu | H | H | EtcOCH₂- |
| 4-52 | Bu | H | H | iPrcOCH₂- |
| 4-53 | Bu | H | H | 1-(EtcO) Et |
| 4-54 | Bu | H | H | 1-(iPrcO) Et |
| 4-55 | Bu | H | H | Phth |
| 4-56 | Bu | H | Me | H |
| 4-57 | Bu | H | Me | Me |
| 4-58 | Bu | H | Me | Et |
| 4-59 | Bu | H | Me | PivOCH₂- |
| 4-60 | Bu | H | Me | Mod |
| 4-61 | Bu | H | Me | EtcOCH₂- |
| 4-62 | Bu | H | Me | iPrcOCH₂- |
| 4-63 | Bu | H | Me | 1-(EtcO)Et |
| 4-64 | Bu | H | Me | 1-(iPrcO)Et |
| 4-65 | Bu | H | Me | Phth |
| 4-66 | Bu | H | Fo | H |
| 4-67 | Bu | H | Fo | PivOCH₂- |
| 4-68 | Bu | H | Fo | Mod |
| 4-69 | Bu | H | Fo | Phth |
| 4-70 | Bu | H | Ac | H |
| 4-71 | Bu | H | Ac | PivOCH₂- |
| 4-72 | Bu | H | Ac | Mod |
| 4-73 | Bu | H | Ac | Phth |
| 4-74 | Bu | Me | H | H |
| 4-75 | Bu | Me | H | Et |
| 4-76 | Bu | Me | H | PivOCH₂- |
| 4-77 | Bu | Me | H | Mod |
| 4-78 | Bu | Me | H | EtcOCH₂- |
| 4-79 | Bu | Me | H | iPrcOCH₂- |
| 4-80 | Bu | Me | H | Phth |
| 4-81 | Bu | Me | Me | H |
| 4-82 | Bu | Me | Me | Me |
| 4-83 | Bu | Me | Me | PivOCH₂- |
| 4-84 | Bu | Me | Me | Mod |
| 4-85 | Bu | Me | Me | Phth |
| 4-86 | Bu | Et | H | H |
| 4-87 | Bu | Et | H | Me |
| 4-88 | Bu | Et | H | PivOCH₂- |
| 4-89 | Bu | Et | H | Mod |
| 4-90 | Bu | Et | H | Phth |
| 4-91 | Et | H | H | H |
| 4-92 | Et | H | Et | H |
| 4-93 | Et | H | Et | PivOCH₂- |
| 4-94 | Et | H | Et | Mod |
| 4-95 | Et | H | Et | Phth |
| 4-96 | Pn | H | H | H |
| 4-97 | Pn | H | H | Et |
| 4-98 | Pn | H | H | PivOCH₂- |
| 4-99 | Pn | H | H | Mod |
| 4-100 | Pn | H | H | Phth |
| 4-101 | Pr | iPr | H | H |
| 4-102 | Pr | iPr | H | PivOCH₂- |
| 4-103 | Pr | iPr | H | Mod |
| 4-104 | Pr | tBu | H | H |
| 4-105 | Pr | tBu | H | PivOCH₂- |
| 4-106 | Pr | tBu | H | Mod |
| 4-107 | Et | Me | H | H |
| 4-108 | Et | Me | H | Et |
| 4-109 | Et | Me | H | PivOCH₂- |
| 4-110 | Et | Me | H | Mod |
| 4-111 | Et | Me | H | Phth |
| 4-112 | Et | H | H | PivOCH₂- |
| 4-113 | Et | H | H | Mod |
| 4-114 | Et | Me | H | PivOCH₂- |
| 4-115 | Et | Me | H | Mod |

**Table 5**

| Cpd. No. | R¹ | R² | R³ | R⁴ | R⁷ | R⁸ | R⁹ |
|---|---|---|---|---|---|---|---|
| 5-1 | Pr | H | H | H | COOH | H | H |
| 5-2 | Pr | Me | H | H | COOH | H | H |
| 5-3 | Pr | Et | H | H | COOH | H | H |
| 5-4 | Pr | Pr | H | H | COOH | H | H |
| 5-5 | Pr | iPr | H | H | COOH | H | H |
| 5-6 | Pr | tBu | H | H | COOH | H | H |
| 5-9 | Pr | H | H | Me | COOH | H | H |
| 5-10 | Pr | H | H | Et | COOH | H | H |
| 5-11 | Pr | Me | H | Me | COOH | H | H |
| 5-12 | Pr | Et | H | Me | COOH | H | H |
| 5-13 | Pr | iPr | H | Me | COOH | H | H |
| 5-14 | Pr x | tBu | H | Me | COOH | H | H |
| 5-15 | Pr | H | H | Fo | COOH | H | H |
| 5-16 | Pr | Me | H | Fo | COOH | H | H |
| 5-17 | Pr | Et | H | Fo | COOH | H | H |
| 5-18 | Pr | iPr | H | Fo | COOH | H | H |
| 5-19 | Pr | tBu | H | Fo | COOH | H | H |
| 5-20 | Pr | H | H | Ac | COOH | H | H |
| 5-21 | Pr | Me | H | Ac | COOH | H | H |
| 5-22 | Pr | Et | H | Ac | COOH | H | H |
| 5-23 | Pr | iPr | H | Ac | COOH | H | H |
| 5-24 | Pr | tBu | H | Ac | COOH | H | H |
| 5-25 | Pr | H | H | H | COOH | H | Me |
| 5-26 | Pr | H | H | H | COOH | H | Et |
| 5-31 | Pr | H | H | H | COOH | Me | Me |
| 5-32 | Pr | H | H | H | Tz | H | H |
| 5-33 | Pr | Me | H | H | Tz | H | H |
| 5-34 | Pr | Et | H | H | Tz | H | H |
| 5-35 | Pr | Pr | H | H | Tz | H | H |
| 5-36 | Pr | iPr | H | H | Tz | H | H |
| 5-37 | Pr | tBu | H | H | Tz | H | H |
| 5-40 | Pr | H | H | Me | Tz | H | H |
| 5-41 | Pr | H | H | Et | Tz | H | H |
| 5-42 | Pr | Me | H | Me | Tz | H | H |
| 5-43 | Pr | Et | H | Me | Tz | H | H |
| 5-44 | Pr | iPr | H | Me | Tz | H | H |
| 5-45 | Pr | tBu | H | Me | Tz | H | H |
| 5-46 | Pr | H | H | Fo | Tz | H | H |
| 5-47 | Pr | Me | H | Fo | Tz | H | H |
| 5-48 | Pr | Et | H | Fo | Tz | H | H |
| 5-49 | Pr | iPr | H | Fo | Tz | H | H |
| 5-50 | Pr | tBu | H | Fo | Tz | H | H |
| 5-51 | Pr | H | H | Ac | Tz | H | H |
| 5-52 | Pr | Me | H | Ac | Tz | H | H |
| 5-53 | Pr | Et | H | Ac | Tz | H | H |
| 5-54 | Pr | iPr | H | Ac | Tz | H | H |
| 5-55 | Pr | tBu | H | Ac | Tz | H | H |
| 5-56 | Pr | H | H | H | Tz | H | Me |
| 5-57 | Pr | H | H | H | Tz | H | Et |
| 5-62 | Pr | H | H | H | Tz | Me | Me |
| 5-63 | Bu | H | H | H | COOH | H | H |
| 5-64 | Bu | Me | H | H | COOH | H | H |
| 5-65 | Bu | Et | H | H | COOH | H | H |
| 5-66 | Bu | Pr | H | H | COOH | H | H |
| 5-67 | Bu | iPr | H | H | COOH | H | H |
| 5-68 | Bu | tBu | H | H | COOH | H | H |
| 5-71 | Bu | H | H | Me | COOH | H | H |
| 5-72 | Bu | H | H | Et | COOH | H | H |
| 5-73 | Bu | Me | H | Me | COOH | H | H |
| 5-74 | Bu | Et | H | Me | COOH | H | H |
| 5-75 | Bu | iPr | H | Me | COOH | H | H |
| 5-76 | Bu | tBu | H | Me | COOH | H | H |
| 5-77 | Bu | H | H | Fo | COOH | H | H |
| 5-78 | Bu | Me | H | Fo | COOH | H | H |
| 5-79 | Bu | Et | H | Fo | COOH | H | H |
| 5-80 | Bu | iPr | H | Fo | COOH | H | H |
| 5-81 | Bu | tBu | H | Fo | COOH | H | H |
| 5-82 | Bu | H | H | Ac | COOH | H | H |
| 5-83 | Bu | Me | H | Ac | COOH | H | H |
| 5-84 | Bu | Et | H | Ac | COOH | H | H |
| 5-85 | Bu | iPr | H | Ac | COOH | H | H |
| 5-86 | Bu | tBu | H | Ac | COOH | H | H |
| 5-87 | Bu | H | H | H | COOH | H | Me |
| 5-88 | Bu | H | H | H | COOH | H | Et |
| 5-93 | Bu | H | H | H | COOH | Me | Me |
| 5-94 | Bu | H | H | H | Tz | H | H |
| 5-95 | Bu | Me | H | H | Tz | H | H |
| 5-96 | Bu | Et | H | H | Tz | H | H |
| 5-97 | Bu | Pr | H | H | Tz | H | H |
| 5-98 | Bu | iPr | H | H | Tz | H | H |
| 5-99 | Bu | tBu | H | H | Tz | H | H |
| 5-102 | Bu | H | H | Me | Tz | H | H |
| 5-103 | Bu | H | H | Et | Tz | H | H |
| 5-104 | Bu | Me | H | Me | Tz | H | H |
| 5-105 | Bu | Et | H | Me | Tz | H | H |
| 5-106 | Bu | iPr | H | Me | Tz | H | H |
| 5-107 | Bu | tBu | H | Me | Tz | H | H |
| 5-108 | Bu | H | H | Fo | Tz | H | H |
| 5-109 | Bu | Me | H | Fo | Tz | H | H |
| 5-110 | Bu | Et | H | Fo | Tz | H | H |
| 5-111 | Bu | iPr | H | Fo | Tz | H | H |
| 5-112 | Bu | tBu | H | Fo | Tz | H | H |
| 5-113 | Bu | H | H | Ac | Tz | H | H |
| 5-114 | Bu | Me | H | Ac | Tz | H | H |
| 5-115 | Bu | Et | H | Ac | Tz | H | H |
| 5-116 | Bu | iPr | H | Ac | Tz | H | H |
| 5-117 | Bu | tBu | H | Ac | Tz | H | H |
| 5-118 | Bu | H | H | H | Tz | H | Me |
| 5-119 | Bu | H | H | H | Tz | H | Et |
| 5-124 | Bu | H | H | H | Tz | Me | Me |
| 5-125 | Bu | H | H | H | COOH | H | CH₂COOH |
| 5-126 | Bu | H | H | H | COOH | H | CH₂COOEt |
| 5-138 | Bu | Me | H | H | COOH | H | CH₂COOH |
| 5-139 | Bu | Me | H | H | COOH | H | CH₂COOEt |
| 5-151 | Bu | iPr | H | H | COOH | H | CH₂COOH |
| 5-152 | Bu | iPr | H | H | COOH | H | CH₂COOEt |
| 5-164 | Bu | tBu | H | H | COOH | H | CH₂COOH |
| 5-165 | Bu | tBu | H | H | COOH | H | CH₂COOEt |
| 5-177 | Bu | H | H | H | Tz | H | CH₂COOH |
| 5-178 | Bu | H | H | H | Tz | H | CH₂COOEt |
| 5-190 | Bu | Me | H | H | Tz | H | CH₂COOH |
| 5-191 | Bu | Me | H | H | Tz | H | CH₂COOEt |
| 5-203 | Bu | iPr | H | H | Tz | H | CH₂COOH |
| 5-204 | Bu | iPr | H | H | Tz | H | CH₂COOEt |
| 5-216 | Bu | tBu | H | H | Tz | H | CH₂COOH |
| 5-217 | Bu | tBu | H | H | Tz | H | CH₂COOEt |
| 5-229 | Pr | H | H | H | COOH | H | CH₂COOH |
| 5-230 | Pr | H | H | H | COOH | H | CH₂COOEt |
| 5-242 | Pr | Me | H | H | COOH | H | CH₂COOH |
| 5-243 | Pr | Me | H | H | COOH | H | CH₂COOEt |
| 5-255 | Pr | iPr | H | H | COOH | H | CH₂COOH |
| 5-256 | Pr | iPr | H | H | COOH | H | CH₂COOEt |
| 5-268 | Pr | tBu | H | H | COOH | H | CH₂COOH |
| 5-269 | Pr | tBu | H | H | COOH | H | CH₂COOEt |
| 5-281 | Pr | H | H | H | Tz | H | CH₂COOH |
| 5-282 | Pr | H | H | H | Tz | H | CH₂COOEt |
| 5-294 | Pr | Me | H | H | Tz | H | CH₂COOH |
| 5-295 | Pr | Me | H | H | Tz | H | CH₂COOEt |
| 5-307 | Pr | iPr | H | H | Tz | H | CH₂COOH |
| 5-308 | Pr | iPr | H | H | Tz | H | CH₂COOEt |
| 5-320 | Pr | tBu | H | H | Tz | H | CH₂COOH |
| 5-321 | Pr | tBu | H | H | Tz | H | CH₂COOEt |
| 5-334 | Bu | H | H | H | COOH | -(CH₂)₃CH(COOH)- | |
| 5-335 | Bu | H | H | H | COOH | - (CH₂)₃CH (COOMe)- | |
| 5-336 | Pr | H | H | H | -COOCH₂-OPiv | H | H |
| 5-337 | Pr | Me | H | H | -COOCH₂OPiv | H | H |
| 5-339 | Pr | H | H | H | -COOMod | H | H |
| 5-340 | Pr | Me | H | H | -COOMod | H | H |
| 5-342 | Bu | H | H | H | -COOCH₂OPiv | H | H |
| 5-343 | Bu | Me | H | H | -COOCH₂OPiv | H | H |
| 5-345 | Bu | H | H | H | -COOMod | H | H |
| 5-346 | Bu | Me | H | H | -COOMod | H | H |
| 5-348 | Et | iPr | H | H | Tz | H | H |
| 5-349 | Et | iPr | H | H | COOH | H | H |
| 5-350 | Et | tBu | H | H | Tz | H | H |
| 5-351 | Et | tBu | H | H | COOH | H | H |

Of the compounds listed above, the following are preferred, that is to say Compounds No. 1-1, 1-2, 1-9, 1-11, 1-12, 1-15, 1-22, 1-23, 1-24, 1-25, 1-27, 1-28, 1-56, 1-58, 1-59, 1-60, 1-61, 1-62, 1-98, 1-102, 1-103, 1-133, 1-134, 1-138, 1-139, 1-140, 4-1, 4-4, 4-5, 4-6, 4-7, 4-8, 4-9, 4-10, 4-11, 4-14, 4-15, 4-16, 4-17, 4-18, 4-19, 4-20, 4-21, 4-22, 4-23, 4-25, 4-26, 4-27, 4-29, 4-31, 4-32, 4-33, 4-34, 4-35, 4-36, 4-38, 4-39, 4-41, 4-43, 4-44, 4-46, 4-49, 4-50, 4-51, 4-52, 4-53, 4-54, 4-55, 4-56, 4-59, 4-60, 4-61, 4-62, 4-63, 4-64, 4-65, 4-66, 4-67, 4-68, 4-70, 4-71, 4-72, 4-74, 4-76, 4-77, 4-78, 4-79, 4-80, 4-81, 4-83, 4-84, 4-85, 4-91, 4-96, 4-98, 4-99, 4-107, 4-109, 4-110, 4-112, 4-113, 4-114, 4-115, 5-1, 5-2, 5-3, 5-5, 5-6, 5-13, 5-14, 5-18, 5-19, 5-23, 5-24, 5-32, 5-33, 5-34, 5-36, 5-37, 5-44, 5-45, 5-49, 5-50, 5-54, 5-55, 5-63, 5-64, 5-65, 5-67, 5-68, 5-75, 5-76, 5-80, 5-81, 5-85, 5-86, 5-94, 5-95, 5-96, 5-98, 5-99, 5-106, 5-107, 5-111, 5-112, 5-116, 5-117, 5-125, 5-138, 5-151, 5-164, 5-177, 5-190, 5-203, 5-216, 5-229, 5-242, 5-255, 5-268, 5-281, 5-294, 5-307, 5-320, 5-348, 5-349, 5-350 and 5-351, of which Compounds No. 1-22, 1-25, 1-27, 1-28, 1-56, 1-58, 1-59, 1-132, 1-133, 1-134, 4-4, 4-5, 4-6, 4-7, 4-11, 4-14, 4-15, 4-16, 4-17, 4-20, 4-29, 4-31, 4-32, 4-33, 4-34, 4-35, 4-36, 4-38, 4-39, 4-41, 4-43, 4-44, 4-46, 4-49, 4-50, 4-51, 4-52, 4-55, 4-56, 4-59, 4-60, 4-61, 4-62, 4-65, 4-74, 4-76, 4-77, 4-78, 4-79, 4-80, 4-81, 4-83, 4-84, 4-91, 4-96, 4-107, 4-109, 4-110, 4-114, 4-115, 5-5, 5-6, 5-13, 5-14, 5-32, 5-36, 5-37, 5-44, 5-45, 5-63, 5-67, 5-68, 5-75, 5-76, 5-80, 5-81, 5-94, 5-98, 5-99, 5-106, 5-107, 5-348, 5-349, 5-350 and 5-351 are more preferred, and Compounds No. 1-28, 1-56, 1-58, 1-59, 1-133, 1-134, 4-29, 4-31, 4-32, 5-36 and 5-37 are still more preferred. The most preferred compounds are Compounds No.:
4-29. 4-(1-Hydroxyethyl)-2-propyl-1-{4-[2-(tetrazol5-yl)phenyl]phenyl}methylimidazole-5-carboxylic acid;
4-31. Pivaloyloxymethyl 4-(1-hydroxyethyl)-2-propyl-1-{4-[2-(tetrazol-5-yl)phenyl]phenyl}methylimidazole-5-carboxylate; and
4-32. (5-Methyl-2-oxo-l,3-dioxolen-4-yl)methyl 4-(1-hydroxyethyl)-2-propyl-1-{4-[2-(tetrazol-5-yl)phenyl]-phenyl}methylimidazole-5-carboxylate;
and pharmaceutically acceptable salts thereof.

The compounds of the present invention may be prepared by a variety of methods well known in the art for the preparation of compounds of this type.

For example, in general terms, the compounds may be prepared by reacting a compound of formula (II): 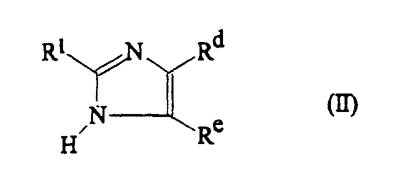 in which:
R¹ is as defined above and R^{d} represents a group of formula 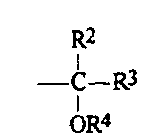 wherein R², R³ and R⁴ are as defined above,
or R^{d} represents a group of formula -COOR^{f} wherein R^{f} represents a carboxy-protecting group, R^{d} represents a group of formula -COR², wherein R² is as defined above, or R^{d} represents a cyano group; and
R^{e} represents a cyano group, a carboxy group or a group of formula -COOR^{f}, wherein R^{f} is as defined above,
with a compound of formula (III) : 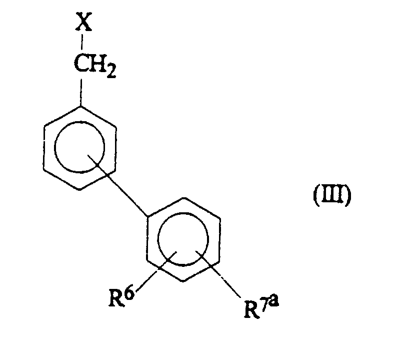 in which: R⁶ is as defined above; R^{7a} represents a protected carboxy group, a cyano group, a protected tetrazol-5-yl group, a carbamoyl group or an alkylcarbamoyl group; and X represents a halogen atom;
to give a compound of formula (IV): 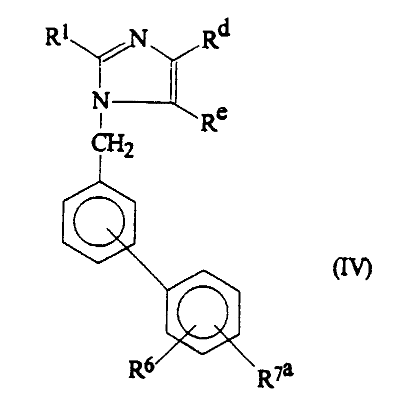 wherein R^{d}, R^{e}, R¹, R⁶ and R^{7a} are as defined above; and
in any order, removing protecting groups, and, if necessary, converting said group R^{d} to a group of formula 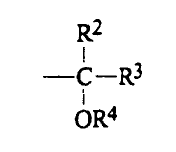 wherein R², R³ and R⁴ are as defined above,
and, if necessary, converting said group R^{e} to a group R⁵, converting said group R^{7a} to a group R⁷, or alkylating or acylating a hydroxy group in R⁴, to give a compound of formula (I); and
optionally salifying or esterifying the product.

Preferably, R^{e} represents a protected carboxy group, when R^{7a} represents a protected carboxy group, a cyano group, a protected tetrazolyl group, a carbamoyl group or an alkylcarbamoyl group, and R^{e} represents a cyano group when R^{7a} represents a protected carboxy group or a protected tetrazolyl group.

In more detail, the compounds of the present invention may be prepared as described below in Reaction Schemes A to F.

### Reaction Scheme A:

In this Reaction Scheme, a compound of formula (I) is prepared by reacting an imidazole-5-carboxylic acid or ester thereof of formula (V) with a biphenylmethyl halide of formula (III), and then, if desired, removing protecting groups, converting the group of formula - COOR^{5a} to any other group represented by R⁵, converting the group represented by R^{7a} to any other group represented by R⁷ and/or alkylating or acylating a hydroxy group in R⁴, as shown below:

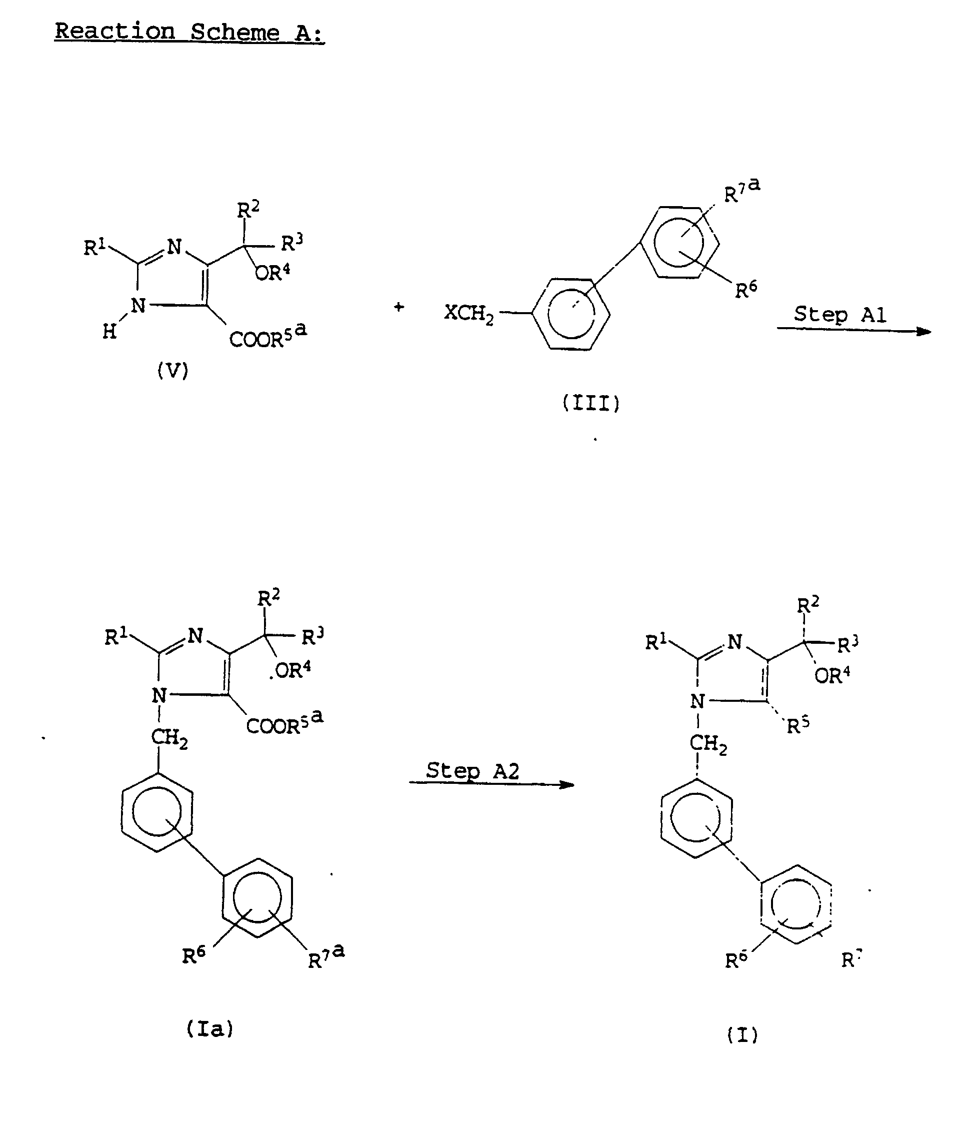

In the above reaction scheme, R¹, R², R³, R⁴, R⁵, R^{5a}, R⁶, R⁷, R^{7a} and X are as defined above, and R^{5a} preferably represents a group other than a hydrogen atom.

Where R^{7a} represents a protected carboxy group, the protecting group may be any of the ester residues illustrated above in relation to R^{5a}. Alternatively, R^{7a} may be a carbamoyl group or a substituted carbamoyl group of formula -CONHR, where R represents a hydrogen atom or an alkyl group having from 1 to 6 carbon atoms, for example any of those illustrated above in relation to R¹. Examples of such carbamoyl groups which may be represented by R^{7a} include the carbamoyl, methylcarbamoyl, ethylcarbamoyl, propylcarbamoyl, butylcarbamoyl, t-butylcarbamoyl, pentylcarbamoyl, t-pentylcarbamoyl and hexylcarbamoyl groups, of which the carbamoyl, t-butylcarbamoyl and t-pentylcarbamoyl groups are preferred. Where R^{7a} represents a protected tetrazolyl group, the protecting group may be any protecting group commonly used to protect tetrazolyl groups in conventional compounds of this type. Examples of suitable protecting groups include aralkyl groups comprising an alkyl group having from 1 to 6 carbon atoms which is substituted by an aromatic carbocyclic group having from 6 to 14 ring atoms, but it is preferably a benzyl, diphenylmethyl (benzhydryl) or triphenylmethyl (trityl group), most preferably a trityl group.

X represents a halogen atom, preferably a chlorine, bromine or iodine atom).

In Step Al of this Reaction Scheme, a compound of formula (Ia) is prepared by reacting an imidazole-5-carboxylate compound of formula (V) with a biphenylmethyl compound of formula (III). The reaction normally and preferably takes place in an inert solvent and preferably in the presence of a base.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, preferably aromatic hydrocarbons, such as benzene or toluene; ethers, such as tetrahydrofuran or dioxane; alcohols, such as methanol, ethanol or t-butanol; amides, such as N,N-dimethylacetamide, N,N-dimethylformamide or N-methyl-2-pyrrolidinone; ketones, such as acetone or methyl ethyl ketone; nitriles, such as acetonitrile; and sulphoxides, such as dimethyl sulphoxide. Of these, we prefer the amides, ketones, nitriles and sulphoxides.

The nature of the base employed in the reaction is likewise not critical, and any base capable of reacting with the acid H-X can be used in this reaction. Preferred examples of bases which may be used include: alkali metal carbonates, such as sodium carbonate or potassium carbonate; alkali metal hydrides, such as sodium hydride, potassium hydride or lithium hydride; alkali metal alkoxides, such as sodium methoxide, sodium ethoxide, potassium t-butoxide or lithium methoxide; and alkali metal hydrogencarbonates, such as sodium hydrogencarbonate or potassium hydrogencarbonate. Of these, we prefer the alkali metal carbonates, alkalimetal hydrides or alkali metal alkoxides.

The reaction can take place over a wide range of temperatures, and the precise reaction temperature is not critical to the invention. In general, we find it convenient to carry out the reaction at a temperature of from -10°C to 100°C, more preferably from 0°C to 80°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the desired compound of formula (Ia) can be recovered from the reaction mixture by conventional means. For example, one suitable recovery procedure comprises: removing the solvent by distillation under reduced pressure; mixing the residue with water; extracted the residue with a water-immiscible solvent, such as ethyl acetate; drying the extract over, for example, anhydrous sodium sulphate; and freeing the product from the solvent by distillation. The resulting product can, if necessary, be purified by conventional means, for example, by recrystallization, or the various chromatography techniques, notably preparative thin layer chromatography or column chromatography.

Step A2 may comprise any one or (if appropriate) more of the following reactions:
(i) removing the carboxy-protecting groups either selectively or non-selectively from the group of formula -COOR^{5a} and/or the group R^{7a}, to convert it or them to a free carboxy group as represented by R⁵ or R⁷, respectively;
(ii) esterifying any such free carboxy group to provide an ester of the group, for example as illustrated above in relation to R^{5a};
(iii) converting such a free carboxy group represented by R⁵ to a group of formula -CONR⁸R⁹;
(iv) removing the tetrazolyl-protecting group;
(v) converting a cyano group represented by R^{7a} to a tetrazolyl group;
(vi) converting a monoalkylcarbamoyl group or a carbamoyl group represented by R^{7a} first to a cyano group and then to a tetrazolyl group;
(vii) where R⁴ represents an aliphatic acyl group, which can be regarded as hydroxy-protecting group, removing the protecting group to produce a compound in which R⁴ represents a hydrogen atom; and
(viii) where R⁴ represents a hydroxy group, alkylating or acylating this group.

### (i) Removal of carboxy-protecting groups:

The nature of the reaction employed to remove the carboxy-protecting group will, of course, depend on the nature of the group to be removed and such reactions are well known in the field of organic synthesis.

For example, where the carboxy-protecting group is an aralkyl group, such as a benzyl or p-nitrobenzyl group, the protecting group may be removed by catalytic reduction, in the presence of hydrogen, which may be under atmospheric pressure or superatmospheric pressure, for example up to 5 atmospheres pressure. The reaction normally and preferably takes place in an inert solvent (preferably an alcohol, such as methanol or ethanol, or a carboxylic acid, such as acetic acid) and in the presence of a catalyst. Any catalyst commonly used for catalytic hydrogenation or reduction may equally be employed here, preferably palladium-on-charcoal or platinum oxide.

Where the carboxy-protecting group is a t-butyl or diphenylmethyl group, it may be removed by reacting the protected compound with an acid (preferably a mineral acid, such as hydrogen chloride or sulphuric acid, or an organic acid, such as trifluoroacetic acid, methanesulphonic acid or p-toluenesulphonic acid) in an inert solvent (preferably an alcohol, such as methanol or ethanol; an ether, such as tetrahydrofuran or dioxane; water; or a mixture of water and one or more of the above organic solvents).

Where the carboxy-protecting group is a silyl group, this may be a group of formula -SiR^{a}R^{b}R^{c}, in which R^{a}, R^{b} and R^{c} are as defined above. In this case, the protecting group may be removed by reacting the protected compound with an acid (preferably a mineral acid, such as hydrogen chloride, or an organic acid, such as acetic acid, trifluoroacetic acid, methanesulphonic acid or p-toluenesulphonic acid) or with a fluorine salt, such as tetrabutylammonium fluoride. The reaction normally and preferably takes place in an inert solvent (preferably: an ether, such as tetrahydrofuran or dioxane; an alcohol, such as methanol or ethanol; an amide, such as N,N-dimethylformamide or N,N-dimethylacetamide; water; or a mixture of water and one or more of the above organic solvents).

Where the carboxy-protecting group is an ester residue, the protecting group may be removed by hydrolysis using a base (preferably an alkali metal hydroxide, such as lithium hydroxide, sodium hydroxide or potassium hydroxide, or an alkali metal carbonate, such as sodium carbonate or potassium carbonate) in an inert solvent (preferably an alcohol, such as methanol or ethanol; an ether, such as tetrahydrofuran or dioxane; water; or a mixture of water and one or more of the above organic solvents). Where R⁴ represents an acyl group, it is removed simultaneously in the course of this reaction.

The reaction can take place over a wide range of temperatures, and the precise reaction temperature is not critical to the invention. In general, we find it convenient to carry out the reaction at a temperature of from 0°C to 100°C, more preferably from about room temperature to 60°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the desired compound may be recovered by conventional means, the nature of which will depend on the nature of the deprotection reaction. For example, where the deprotection is carried out by catalytic reduction, the desired product can be recovered by filtering off the catalyst and then distilling off the solvent. Where the deprotection is carried out using an acid, the desired product can be recovered by collecting the precipitate in the reaction system by filtration or by concentration of the reaction mixture. Where the deprotection is carried out by alkaline hydrolysis, the desired product can be recovered by distilling off the solvent and then neutralizing the residue with an aqueous acid, after which the precipitate in the aqueous solvent may be collected by filtration; alternatively, it may be recovered by neutralizing the aqueous layer obtained by extracting the reaction mixture with a water-immiscible organic solvent (such as ethyl acetate or diethyl ether), extracting the neutralized solution with a water-immiscible organic solvent (such as ethyl acetate), and then distilling off the solvent. The reaction product may, if necessary, be further purified by conventional means, for example by recrystallization or by the various chromatography techniques, notably preparative thin layer chromatography or column chromatography.

Each of the protecting groups represented by R^{5a} and R^{7a} can be selectively eliminated by appropriate choice of the protecting groups and the specific reaction conditions employed to remove them, as is well known to those skilled in the art.

### (ii) Esterification

Where a compound containing one or more free carboxy groups is produced, this group or these groups may be esterified, by methods well known in organic chemistry. For example, the reaction may be carried out by reacting the corresponding carboxylic acid with a compound of formula, R^{5b}-Y [in which R^{5b} may represent any of the groups defined above for R^{5a} other than a hydrogen atom, and Y represents a halogen atom, such as a chlorine, bromine or iodine atom, a group of formula -OSO₃R^{5b} (in which R^{5b} is as defined above) or a sulphonyloxy group, such as a methanesulphonyloxy or p-toluenesulphonyloxy group]. The reaction is carried out in the presence of a base, for example: an organic amine, such as triethylamine, pyridine or N-methylmorpholine; an alkali metal carbonate, such as sodium carbonate or potassium carbonate; or an alkali metal hydrogencarbonate, such as sodium hydrogencarbonate or potassium hydrogencarbonate. It is also normally and preferably carried out in an inert solvent (preferably: an amide, such as N,N-dimethylformamide or N,N-dimethylacetamide; a halogenated hydrocarbon, preferably a halogenated aliphatic hydrocarbon, such as methylene chloride; a ketone, such as acetone or methyl ethyl ketone; or an ether, such as tetrahydrofuran or dioxane). Where the desired ester group is an alkyl group, the reaction may be carried out by reacting the carboxylic acid with the corresponding dialkyl sulphate.

The reaction can take place over a wide range of temperatures, and the precise reaction temperature is not critical to the invention. In general, we find it convenient to carry out the reaction at a temperature of from 0°C to 120°C, more preferably from 20°C to 80°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

Where the carboxy-protecting group is a C₁ - C₆ alkyl group, the esterification reaction may be carried out by reacting the corresponding carboxylic acid with a C₁ - C₆ alcohol, such as methanol, ethanol, propanol or hexanol, in the presence of an acid catalyst, such as hydrogen chloride or sulphuric acid, in an inert solvent (for example: one of the C₁ - C₆ alcohols which may be used as the starting material described above; a halogenated hydrocarbon, such as methylene chloride; or an ether, such as tetrahydrofuran or dioxane) at a temperature of from 0°C to 100°C for a period of from 1 to 24 hours, or by reacting the corresponding carboxylic acid with a halogenating agent (e.g. phosphorus pentachloride, thionyl chloride or oxalyl chloride) in an inert solvent (for example: a halogenated hydrocarbon, such as methylene chloride; an ether, such as tetrahydrofuran or dioxane; or an aromatic hydrocarbon, such as benzene or toluene) at a temperature of about room temperature for a period of from 30 minutes to 5 hours to yield the corresponding acyl halide, which is then reacted with the corresponding alcohol in an inert solvent (e.g. benzene or methylene chloride) in the presence of a base (for example triethylamine; however, in the case of the t-butyl ester, potassium t-butoxide is used as the preferred base) at a temperature of about room temperature for a period of from 30 minutes to 10 hours. The desired compound can be recovered by conventional means, for example, by a similar method to that described in Step A1.

### (iii) Formation of a carbamoyl group

Conversion of a carboxy group represented by R⁵ to a group of formula -CONR⁸R⁹, in which R⁸ and R⁹ are as defined above, may be carried out using well known methods, for example by reacting the carboxylic acid compound, in which the group R⁷ is protected, with a compound of formula (VI):

R⁸R⁹NH (VI)

in which R⁸ and R⁹ are as defined above).

This reaction consists of the formation of a peptide bond and is generally well known in organic synthetic chemistry. It may be carried out in an inert solvent (preferably a halogenated hydrocarbon, more preferably a halogenated aliphatic hydrocarbon, such as methylene chloride or chloroform; an ester, such as ethyl acetate; an ether, such as tetrahydrofuran or dioxane; or an amide, such as N,N-dimethylacetamide or N,N-dimethylformamide) in the presence of a condensing agent.

Examples of condensing agents which may be used in this reaction include: carbodiimides, such as N,N-dicyclohexylcarbodiimide or 1-(3-dimethylaminopropyl)-3-ethylcarbodiimide hydrochloride; phosphoryl compounds, such as diphenylphosphoryl azide or diethylphosphoryl cyanide; carbonyldiimidazole; and triphenylphosphinediethyl azodicarboxylate. Of these, we prefer the carbodiimides and diphenylphosphoryl azide. Where a phosphoryl compound is used, the reaction is preferably carried out in the presence of a tertiary amine, such as triethylamine or N-methylmorpholine.

Alternatively, the reaction in this step can be accomplished by reacting the carboxylic acid with a lower alkyl chloroformate, such as ethyl chloroformate or isobutyl chloroformate, in the presence of a tertiary amine, such as triethylamine or N-methylmorpholine, to produce a mixed acid anhydride, or by reacting the carboxylic acid with N-hydroxysuccinimide, N-hydroxybenzotriazole or p-nitrophenol or the like in the presence of a carbodiimide, such as N,N-dicyclohexylcarbodiimide, to produce the corresponding active ester, and subsequently reacting the mixed acid anhydride or the active ester with the amine compound of formula (VI).

As a further alternative, the reaction in this step can be carried out by reacting the carboxylic acid with a halogenating agent, such as phosphorus pentachloride, oxalyl chloride or thionyl chloride, in an inert solvent (for example: a halogenated hydrocarbon, such as methylene chloride; an ether, such as tetrahydrofuran or dioxane; or an aromatic hydrocarbon, such as benzene or toluene) to give the corresponding acyl halide, and then reacting the acyl halide with the amine compound of formula (VI).

All of these reactions can take place over a wide range of temperatures, and the precise reaction temperature is not critical to the invention. In general, we find it convenient to carry out the reaction at a temperature of from -20°C to 100°C, more preferably from -5°C to 50°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the reaction product can be recovered from the reaction mixture by conventional means. For example, insoluble materials in the reaction system are filtered off; a water-immiscible organic solvent, such as ethyl acetate, and water are added to the filtrate; the organic solvent layer is separated and dried over a drying agent, such as anhydrous magnesium sulphate; and then the solvent is distilled off to leave the desired product. The reaction product may, if necessary, be further purified by conventional means, for example by recrystallization or by the various chromatography techniques, notably preparative thin layer chromatography or column chromatography.

### (iv) Removal of tetrazolyl-protecting groups

This may be accomplished by reacting the protected compound with an acid. The reaction is normally and preferably effected in an inert solvent.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: water; an organic acid, such as acetic acid; an ether, such as tetrahydrofuran or dioxane; an alcohol, such as methanol, ethanol or t-butanol; a ketone, such as acetone or methyl ethyl ketone; or a mixture of any two or more of these solvents. Of these, we prefer water, an organic acid, an alcohol or a mixture thereof.

There is no particular limitation upon the nature of the acid used in the reaction, provided that it can normally function as a Bronsted acid. Preferred examples of such acids include: organic acids, such as acetic acid, formic acid, oxalic acid, methanesulphonic acid, p-toluenesulphonic acid or trifluoroacetic acid; and inorganic acids, such as hydrochloric acid, hydrobromic acid, sulphuric acid or phosphoric acid. Of these, we prefer acetic acid, formic acid, trifluoroacetic acid or hydrochloric acid.

The reaction can take place over a wide range of temperatures, and the precise reaction temperature is not critical to the invention. In general, we find it convenient to carry out the reaction at a temperature of from -10°C to 120°C, more preferably from 0°C to 100°C. The time required for the reaction may also vary widely, depending on many factors, notably the reaction temperature and the nature of the reagents and solvent employed. However, provided that the reaction is effected under the preferred conditions outlined above, a period of from from 0.5 to 24 hours, more preferably from 1 to 16 hours, will usually suffice.

After completion of the reaction, the desired product of this reaction can be recovered from the reaction mixture by conventional means. For example, after distilling off the solvent, the residue is dissolved in water and a water-immiscible organic solvent. The organic layer containing the desired compound is separated and dried over anhydrous magnesium sulphate. After distilling off the solvent, the desired compound can be obtained. The reaction product may, if necessary, be further purified by conventional means, for example by recrystallization or the various chromatography techniques, notably preparative thin layer chromatography or column chromatography.

### (v) Conversion of a cyano group to a tetrazolyl group

In this step, a cyano group is converted to a tetrazolyl group by reacting the cyano compound with an alkali metal azide.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: amides, such as N,N-dimethylformamide or N,N-dimethylacetamide; ethers, such as dioxane or 1,2-dimethoxyethane; and sulphoxides, such as dimethyl sulphoxide.

Examples of suitable alkali metal azides include lithium azide, sodium azide and potassium azide, of which sodium azide is preferred. There is no particular restriction on the amount of alkali metal azide eployed, but we generally prefer to use from 1 to 5 equivalents, more preferably from 1 to 3 equivalents, of the alkali metal azide per equivalent of the cyano compound.

We also prefer to carry out the reaction in the presence of an ammonium halide, for example ammonium fluoride, ammonium chloride or ammonium bromide, of which ammonium chloride is preferred. There is no particular restriction on the amount of ammonium halide employed, but we generally prefer to use from 0.5 to 2 equivalents, more preferably from 1 to 1.2 equivalents, of the ammonium halide per equivalent of the cyano compound.

The reaction can take place over a wide range of temperatures, and the precise reaction temperature is not critical to the invention. In general, we find it convenient to carry out the reaction at a temperature of from 70 to 150°C, more preferably from 80 to 120°C. The time required for the reaction may also vary widely, depending on many factors, notably the reaction temperature and the nature of the reagents and solvent employed. However, provided that the reaction is effected under the preferred conditions outlined above, a period of from 10 hours to 7 days, more preferably from 1 to 5 days, will usually suffice.

Alternatively, the cyano group may be converted to a tetrazolyl group by reacting the cyano compound with a trialkyltin azide or triaryltin azide, and then treating the resulting tin compound with an acid, a base or an alkali metal fluoride.

The reaction of the cyano compound with the trialkyltin azide or triaryltin azide is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic hydrocarbons, such as benzene, toluene, xylene or heptane; halogenated hydrocarbons, especially halogenated aliphatic hydrocarbons, such as 1,2-dichloroethane or chloroform; ethers, such as dioxane or 1,2-dimethoxyethane; amides, such as N,N-dimethylformamide or N,N-dimethylacetamide; and esters, such as ethyl acetate or butyl acetate.

Although there is no particular limitation on the nature of the trialkyltin or triaryl tin azide, and any such compound commonly used in reactions of this type may equally be employed here, we generally prefer to use: a trialkyltin azide in which each of the alkyl groups (which may be the same or different, although they are preferably the same) have from 1 to 4 carbon atoms, for example trimethyltin azide, triethyltin azide or tributyltin azide; or a triaryltin azide in which each of the aryl groups (which may be the same or different, although they are preferably the same) is as defined above in relation to the aralkyl groups which may be used to protect a tetrazolyl group in the group R^{7a}, preferably a phenyl or substituted phenyl group, for example triphenyltin azide or tritolyltin azide. The amount of the trialkyltin azide or triaryltin azide employed is not critical, although an amount of from 1 to 3 equivalents per equivalent of cyano compound is preferred, and from 1 to 2 equivalents is more preferred.

The reaction of the cyano compound with the trialkyltin azide or triaryltin azide can take place over a wide range of temperatures, and the precise reaction temperature is not critical to the invention. In general, we find it convenient to carry out the reaction at a temperature of from 60 to 150°C, more preferably from 80 to 120°C. The time required for the reaction may also vary widely, depending on many factors, notably the reaction temperature and the nature of the reagents and solvent employed. However, provided that the reaction is effected under the preferred conditions outlined above, a period of from 8 hours to 7 days, more preferably from 1 to 5 days, will usually suffice.

The tin-containing compound produced by this reaction is then treated with an acid, a base or an alkali metal fluoride, to convert it to the desired tetrazolyl compound. Any acid, base or alkali metal fluoride commonly used for this type of reaction may be used in this reaction, and examples of suitable compounds include: acids, especially mineral acids, such as hydrochloric acid or sulphuric acid; bases, especially inorganic bases, such as alkali metal carbonates and hydrogencarbonates (for example sodium carbonate, potassium carbonate, sodium hydrogencarbonate or potassium hydrogencarbonate) or alkali metal hydroxides (for example sodium hydroxide or potassium hydroxide); and alkali metal fluorides, such as lithium fluoride, sodium fluoride or potassium fluoride.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include those listed above for the reaction of the cyano compound with the trialkyltin azide or triaryltin azide and other solvents, such as alcohols (for example methanol or ethanol), water or aqueous alcohols. The reaction can take place over a wide range of temperatures, and the precise reaction temperature is not critical to the invention. In general, we find it convenient to carry out the reaction at a temperature of from 0°C to 100° C, preferably about room temperature. The time required for the reaction may also vary widely, depending on many factors, notably the reaction temperature and the nature of the reagents and solvent employed. However, provided that the reaction is effected under the preferred conditions outlined above, a period of from 30 minutes to 3 days, more preferably from 1 hour to 24 hours, will usually suffice.

A further alternative method of converting a cyano group to a tetrazolyl group is to react the cyano compound with a trialkyltin halide or triaryltin halide, in the presence of an alkali metal azide, after which the resulting tin compound is treated with an acid, a base or an alkali metal fluoride.

The reaction of the cyano compound with the trialkyltin halide or triaryltin halide in the presence of an alkali metal azide is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic hydrocarbons, such as benzene, toluene, xylene or heptane; halogenated hydrocarbons, especially halogenated aliphatic hydrocarbons, such as 1,2-dichloroethane or chloroform; ethers, such as dioxane or 1,2-dimethoxyethane; ketones, such as acetone or methyl ethyl ketone; amides, such as N,N-dimethylformamide or N,N-dimethylacetamide; and esters, such as ethyl acetate or butyl acetate.

Although there is no particular limitation on the nature of the trialkyltin or triaryl tin halide, and any such compound commonly used in reactions of this type may equally be employed here, we generally prefer to use: a trialkyltin halide in which each of the alkyl groups (which may be the same or different, although they are preferably the same) has from 1 to 4 carbon atoms, for example trimethyltin chloride, trimethyltin bromide, triethyltin chloride or tributyltin chloride; or a triaryltin halide in which each of the aryl groups (which may be the same or different, although they are preferably the same) is as defined above in relation to the aralkyl groups which may be used to protect a tetrazolyl group in the group R^{7a}, preferably a phenyl or substituted phenyl group, for example triphenyltin chloride or tritolyltin chloride. The amount of the trialkyltin halide or triaryltin halide employed is not critical, although an amount of from 1 to 3 equivalents per equivalent of cyano compound is preferred, and from 1 to 2 equivalents is more preferred.

There is no particular restriction on the alkali metal azide which is also employed in this reaction. Examples include lithium azide, sodium azide and potassium azide, of which sodium azide is preferred. The amount of the alkali metal azide employed is not critical, although an amount of from 1 to 3 equivalents per equivalent of cyano compound is preferred, and from 1 to 2 equivalents is more preferred.

The reaction of the cyano compound with the trialkyltin halide or triaryltin halide in the presence of an alkali metal azide can take place over a wide range of temperatures, and the precise reaction temperature is not critical to the invention. In general, we find it convenient to carry out the reaction at a temperature of from 60 to 150°C, more preferably from 80 to 120°C. The time required for the reaction may also vary widely, depending on many factors, notably the reaction temperature and the nature of the reagents and solvent employed. However, provided that the reaction is effected under the preferred conditions outlined above, a period of from 8 hours to 7 days, more preferably from 1 to 5 days, will usually suffice.

The tin-containing compound produced by this reaction is then treated with an acid, a base or an alkali metal fluoride, to convert it to the desired tetrazolyl compound. The reaction is essentially the same as the reaction of the tin-containing compound (produced by reacting the cyano compound with a trialkyltin azide or triaryltin azide) with an acid, a base or an alkali metal fluoride, and may be carried out using the same solvents and reaction conditions.

### (vi) Conversion of an alkylcarbamoyl group or a carbamoyl group to a cyano group

To convert an alkylcarbamoyl group to a cyano group, the alkylcarbamoyl compound is reacted with a halogen compound capable of acting as a halogenating agent, preferably chlorinating agent, for example oxalyl chloride, phosphorus oxychloride or sulphonyl chloride. There is no particular restriction on the amount of halogen compound employed, although we generally find it convenient to use from 1 to 3 equivalents, more preferably from 1 to 2 equivalents, per equivalent of the alkylcarbamoyl compound.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic hydrocarbons, such as benzene, toluene, xylene or heptane; halogenated hydrocarbons, especially halogenated aliphatic hydrocarbons, such as methylene chloride or chloroform; ethers, such as dioxane, tetrahydrofuran or diethyl ether; and esters, such as ethyl acetate or butyl acetate.

The reaction can take place over a wide range of temperatures, and the precise reaction temperature is not critical to the invention. In general, we find it convenient to carry out the reaction at a temperature of from -10 to 100°C, more preferably from 0 to 50°C. The time required for the reaction may also vary widely, depending on many factors, notably the reaction temperature and the nature of the reagents and solvent employed. However, provided that the reaction is effected under the preferred conditions outlined above, a period of from 10 minutes to 16 hours, more preferably from 30 minutes to 6 hours, will usually suffice.

To convert a carbamoyl group to a cyano group, the carbamoyl compound is reacted with a dehydrating agent, for example acetic anhydride, trifluoroacetic anhydride, methanesulphonic anhydride, trifluoromethanesulphonic anhydride, oxalyl chloride or sulphonyl chloride, in the presence of an organic amine, for example triethylamine, pyridine or N-methylmorpholine.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic hydrocarbons, such as benzene, toluene, xylene or heptane; halogenated hydrocarbons, especially halogenated aliphatic hydrocarbons, such as methylene chloride or chloroform; ethers, such as dioxane, tetrahydrofuran or diethyl ether; and esters, such as ethyl acetate or butyl acetate.

The reaction can take place over a wide range of temperatures, and the precise reaction temperature is not critical to the invention. In general, we find it convenient to carry out the reaction at a temperature of from -10 to 100°C, more preferably from 0 to 50°C. The time required for the reaction may also vary widely, depending on many factors, notably the reaction temperature and the nature of the reagents and solvent employed. However, provided that the reaction is effected under the preferred conditions outlined above, a period of from 10 minutes to 16 hours, more preferably from 30 minutes to 6 hours, will usually suffice.

The desired product of these reactions can be recovered from the reaction mixture by conventional means, for example by neutralizing the mixture with a weak base, such as sodium hydrogencarbonate and then working up the product in a similar manner to that described in Step A1 of Reaction Scheme A.

The cyano compound thus obtained may then be converted to the corresponding tetrazolyl compound, using any of the reactions described above.

### (vii) Removing hydroxy-protecting groups

Where R⁴ represents an acyl group, which can be regarded as a hydroxy-protecting group, the protecting group may be removed, to produce a compound in which R⁴ represents a hydrogen atom. The nature of the reaction employed to remove the protecting group, will, of course, depend on the nature of the protecting group, as is well known in the art, and any of the many well known reactions used for deprotecting compounds of this type may equally be used here.

Where the hydroxy-protecting group is an aliphatic acyl group, it can be removed by treating the protected compound with a base.

There is no particular limitation upon the nature of the base used, provided that it does not affect other parts of the compound. Preferred examples of such bases include: metal alkoxides, especially alkali metal alkoxides, such as sodium methoxide; alkali metal carbonates, such as sodium carbonate or potassium carbonate; alkali metal hydroxides, such as sodium hydroxide or potassium hydroxide; and ammonia, which is preferably in the form of aqueous ammonia or a concentrated solution of ammonia in methanol.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: water; organic solvents, such as alcohols (e.g. methanol, ethanol or propanol) or ethers (e.g. tetrahydrofuran or dioxane); or a mixture of water and one or more of these organic solvents.

The reaction can take place over a wide range of temperatures, and the precise reaction temperature is not critical to the invention. In general, we find it convenient to carry out the reaction at a temperature of from 0°C to 150°C, more preferably from 0°C to 60°C. The time required for the reaction may also vary widely, depending on many factors, notably the reaction temperature and the nature of the reagents and solvent employed. However, provided that the reaction is effected under the preferred conditions outlined above, a period of from 1 to 20 hours, more preferably from 1 to 16 hours, will usually suffice.

After completion of any of the above reactions, the desired compound of the invention can be recovered from the reaction mixture by conventional means depending on the nature of the reaction and the reaction medium. An example of one such technique comprises: neutralizing the reaction mixture appropriately; removing any insoluble material which may exist in the mixture, for example by filtration; adding a water-immiscible organic solvent; washing with water; and finally distilling off the solvent. The resulting product can, if necessary, be purified by conventional means, for example, by recrystallization, or by the various chromatography techniques, notably preparative thin layer chromatography or column chromatography.

Under the conditions used for removing the hydroxy-protecting group, simultaneous deprotection of a protected carboxy group may take place occasionally.

### (viii) Alkylation and acylation of hydroxy groups

Alkylation of a hydroxy group may be carried out by reacting the hydroxy compound with an alkyl halide in which the alkyl group is a methyl or ethyl group, preferably methyl iodide, ethyl iodide or ethyl bromide, or a dialkyl sulphate (in which each alkyl group is a methyl or ethyl group and may be the same or different, although they are preferably the same), such as dimethyl sulphate or diethyl sulphate.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: amides, such as N,N-dimethylformamide, N,N-dimethylacetamide or N-methylpyrrolidinone; ketones, such as acetone or methyl ethyl ketone; or sulphoxides, such as dimethyl sulphoxide.

The reaction is effected in the presence of a base, the nature of which is not critical, provided that it does not damage the reagents or products. Preferred examples of bases which may be used include alkali metal hydrides, such as sodium hydride, potassium hydride or lithium hydride. The reaction can take place over a wide range of temperatures, and the precise reaction temperature is not critical to the invention. In general, we find it convenient to carry out the reaction at a temperature of from 0°C to 120°C, more preferably from 20°C to 80°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

Acylation of a hydroxy group may also be carried out by well known methods commonly used in organic synthetic chemistry. For example, it can be carried out by reacting the hydroxy compound with: an alkanoyl halide, containing from 2 to 5 carbon atoms, such as acetyl chloride, propionyl chloride, butyryl bromide or valeryl chloride; or a carboxylic acid anhydride or mixed carboxylic acid anhydride, in which the group derived from the or each carboxylic acid contains from 1 to 5, preferably from 2 to 5, carbon atoms, such as a mixed anhydride of formic acid and acetic acid, acetic anhydride, propionic anhydride or valeric anhydride.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons, such as methylene chloride or chloroform; esters, such as ethyl acetate; and ethers, such as tetrahydrofuran or dioxane. The reaction is effected in the presence of a base, preferably an organic tertiary amine, such as triethylamine, pyridine, diethylisopropylamine or 4-dimethylaminopyridine. The reaction can take place over a wide range of temperatures, and the precise reaction temperature is not critical to the invention. In general, we find it convenient to carry out the reaction at a temperature of from -10°C to 120°C, more preferably from 0°C to 80°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of either of the above reactions, the desired product can be recovered from the reaction mixture by conventional means. For example, one suitable recovery method is as already described for recovering the product of Step Al.

### Reaction Scheme B:

Compounds of formula (Ia) in which R⁴ represents a hydrogen atom, that is to say compounds of formula (Ib), may also be prepared as shown in the following Reaction Scheme B:

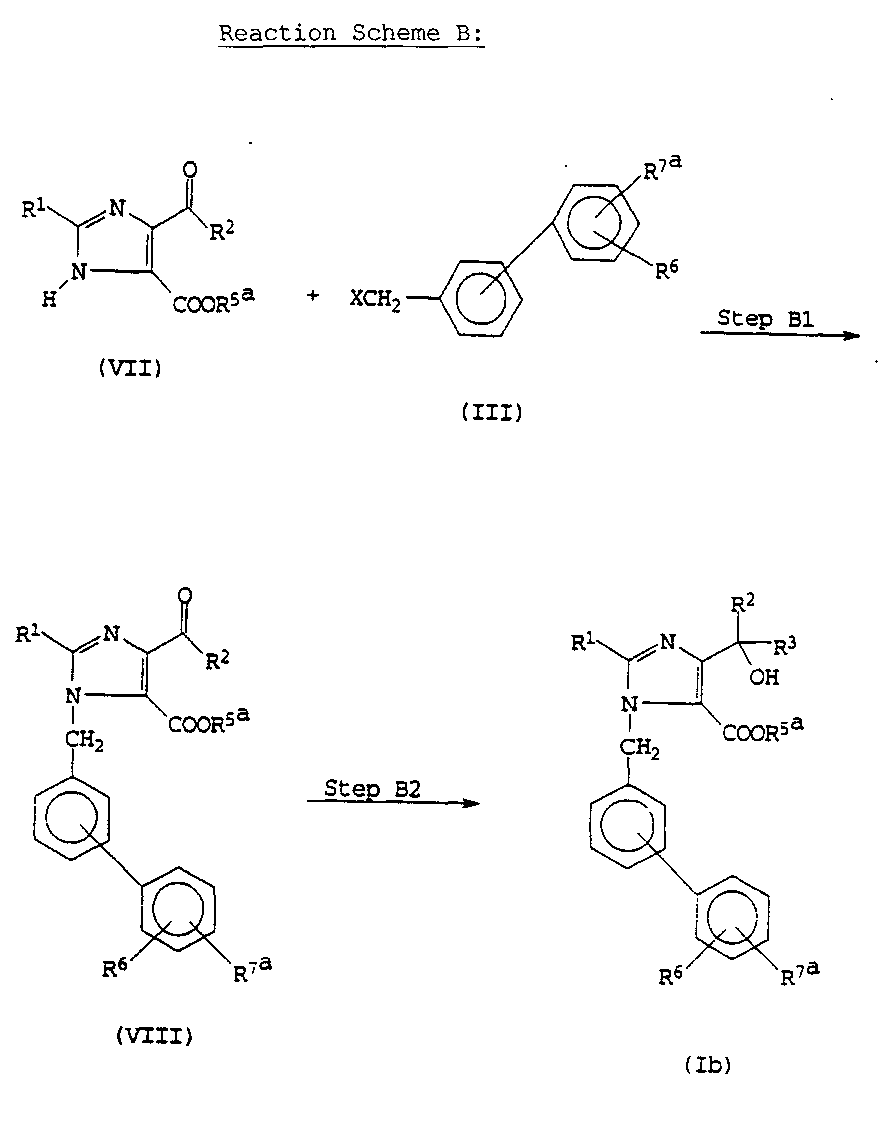

In the above formulae, R¹, R², R³, R^{5a}, R⁶, R^{7a} and X are as defined above, and R^{5a} preferably represents a group other than a hydrogen atom.

In Step B1, an imidazole-5-carboxylate compound of formula (VII) is reacted with a biphenylmethyl compound of formula (III), to give a compound of formula (VIII). This reaction is essentially the same as that of Step A1 in Reaction Scheme A, and may be carried out using the same reagents and reaction conditions.

In Step B2, a compound of formula (Ib) is prepared by reacting a compound of formula (VIII) with a reducing agent or with a Grignard reagent of formula, R^{3a}-Mg-X (in which R^{3a} represents any of the groups defined above for R³ other than a hydrogen atom, and X is as defined above).

Examples of the reducing agents which may be used in this reaction include: alkylaluminium hydrides, such as diisobutylaluminium hydride; and metal, especially alkali metal, borohydrides, such as sodium borohydride or sodium cyanoborohydride. Of these, we prefer diisobutylaluminium hydride and sodium borohydride.

The reaction of the compound of formula (VIII) with the reducing agent is normally and preferably conducted in an inert solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, especially aromatic hydrocarbons, such as toluene or hexane; ethers, such as tetrahydrofuran or dioxane; alcohols, such as methanol or ethanol; water; and mixtures of water with any one or more of the above organic solvents. Preferred solvents vary depending upon the nature of the reducing agent used. For example, where the reducing agent is an alkylaluminium hydride, hydrocarbons or ethers are preferred; alternatively, where it is an alkali metal borohydride, alcohols, water or mixtures of water with an alcohol are preferred.

The reaction can take place over a wide range of temperatures, and the precise reaction temperature is not critical to the invention. In general, we find it convenient to carry out the reaction at a temperature of from -30°C to 80°C, more preferably from -20°C to 20 °C, when the reducing agent is an alkylaluminium hydride, or at a temperature of from -30°C to 80°C, more preferably from 0°C to 50°C, when it is an alkali metal borohydride. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

The reaction of the compound of formula (VIII) with a Grignard reagent is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic, such as hexane or toluene; halogenated hydrocarbons, especially halogenated aliphatic hydrocarbons, such as methylene chloride or 1,2-dichloroethane; and ethers, such as tetrahydrofuran or diethyl ether, of which the ethers and halogenated hydrocarbons are preferred.

The reaction can take place over a wide range of temperatures, and the precise reaction temperature is not critical to the invention. In general, we find it convenient to carry out the reaction at a temperature of from -50°C to 100°C, more preferably from -10°C to 50°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of any of the above reactions, the desired compounds of each reaction can be recovered from the reaction mixture by conventional means. For example, one suitable recovery procedure comprises: mixing the reaction mixture with water or with an aqueous solution of ammonium chloride; and stirring it at room temperature, after which it is extracted with a water-immiscible solvent, such as ethyl acetate. The extract may then be washed with water and dried over a drying agent, such as anhydrous magnesium sulphate, after which the solvent is distilled off; if necessary, the product can be further purified by conventional means, for example, by recrystallization, or by the various chromatography techniques, notably preparative thin layer chromatography or column chromatography.

### Reaction Scheme C:

Compounds of formula (Ia) in which R², R³ and R⁴ all represent hydrogen atoms, that is to say compounds of formula (Ic), and compounds of formula (VIII), which are intermediates in reaction Scheme B, can be prepared as shown in Reaction Scheme C:

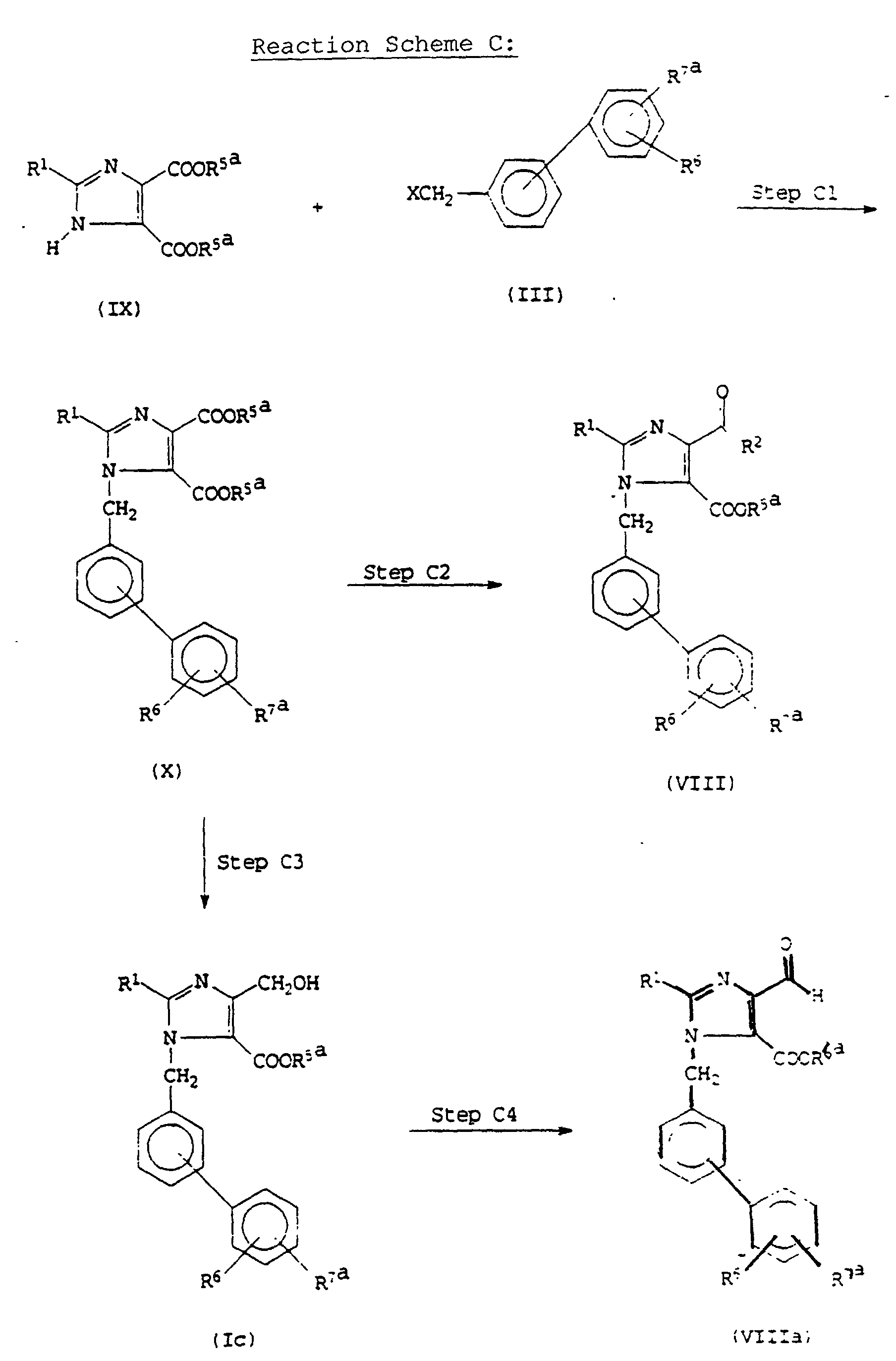

In the above formulae, R¹, R², R^{5a}, R⁶, R^{7a} and X are as defined above, and R^{5a} preferably represents a group other than a hydrogen atom.

In Step C1 of this reaction scheme, an imidazole-5- carboxylate compound of formula (IX) is reacted with a biphenylmethyl compound of formula (III), to give a dicarboxylate compound of formula (X). This reaction is essentially the same as that described above in Step A1 of Reaction Scheme A, and may be carried out using the same reagents and reaction conditions.

In Step C2 of this reaction scheme, the dicarboxylate compound of formula (X) obtained as shown in Step C1 is reacted with about one equivalent of a Grignard reagent of formula R^{2a}MgX (in which X is as defined above and R^{2a} represents any of the groups defined above for R² other than a hydrogen atom) and/or with about one equivalent of a reducing agent to give the compound of formula (VIII). These reactions are essentially the same as those described above in Step B2 of Reaction Scheme B, and may be carried out using the same reagents and reaction conditions.

In Step C3 of this reaction scheme, which is an alternative to Step C2, the dicarboxylate compound of formula (X) is reacted with two or more molar equivalents of a reducing agent to give the compound of formula (Ic). The reaction is essentially the same as that described above in Step B2 of Reaction Scheme B, and may be carried out using the same reagents and reaction conditions.

In Step C4, the hydroxymethyl compound of formula (Ic), obtained as shown in Step C3, is oxidized to convert the hydroxymethyl group to a formyl group and prepare a compound of formula (VIIIa).

The oxidization reaction in this Step may be carried out by reacting the hydroxymethyl compound of formula (Ic) with an oxidizing agent, such as magnesium oxide or silver oxide.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, which may be aliphatic or aromatic hydrocarbons, such as benzene, toluene, xylene or heptane; halogenated hydrocarbons, especially halogenated aliphatic hydrocarbons, such as methylene chloride or chloroform; ethers, such as diethyl ether, tetrahydrofuran or dioxane; esters, such as ethyl acetate or butyl acetate; and ketones, such as acetone or methyl ethyl ketone.

The reaction can take place over a wide range of temperatures, and the precise reaction temperature is not critical to the invention. In general, we find it convenient to carry out the reaction at a temperature of from 0 to 100°C, more preferably from 10 to 60°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more, preferably from 1 to 16 hours, will usually suffice.

Alternatively, the reaction of Step C4 may be carried out by reacting the hydroxymethyl compound of formula (Ic) with dimethyl sulphoxide and with a dehydrating agent in the presence of an organic amine. Suitable dehydrating agents include, for example, sulphur trioxide-dioxane complex, oxalyl chloride and trifluoroacetic anhydride. Suitable organic amines include, for example, triethylamine and pyridine.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons, such as methylene chloride or chloroform; ethers, such as diethyl ether, tetrahydrofuran or dioxane; esters, such as ethyl acetate or butyl acetate; and sulphoxides, such as dimethyl sulphoxide.

The reaction can take place over a wide range of temperatures, and the precise reaction temperature is not critical to the invention. In general, we find it convenient to carry out the reaction at a temperature of from -60°C to 60°C, more preferably from -50°C to 30 °C. The time required for the reaction may also vary widely, depending on many factors, notably the reaction temperature and the nature of the reagents and solvent employed. However, provided that the reaction is effected under the preferred conditions outlined above, a period of from 10 minutes to 8 hours, more preferably from 30 minutes to 5 hours, will usually suffice.

After completion of any of the above reactions, the desired product of the reaction can be recovered from the reaction mixture by conventional means. For example, the reaction mixture is mixed with water and with a water-immiscible solvent, such as ethyl acetate. The organic layer is separated, washed with water and dried over a drying agent, such as anhydrous magnesium sulphate; the solvent is then removed by distillation, normally under reduced pressure. If necessary, the product can be further purified by conventional means, for example, by recrystallization, or by the various chromatography techniques, notably preparative thin layer chromatography or column chromatography.

The resulting compound of formula (VIII) may then, if desired, be allowed to react with a Grignard reagent of formula R^{3a}MgX (in which R^{3a} and X are as defined above) according to the method described above in Step B2 of Reaction Scheme B, to give the corresponding compound having a group of formula - CR²(R^{3a})-OH (in which R² and R^{3a} are as described above) at the 4-position of the imidazolyl ring - not shown in the reaction scheme.

### Reaction Scheme D:

In this reaction scheme, a cyano compound of formula (XII) is first prepared, and then this is converted to a compound of formula (I):

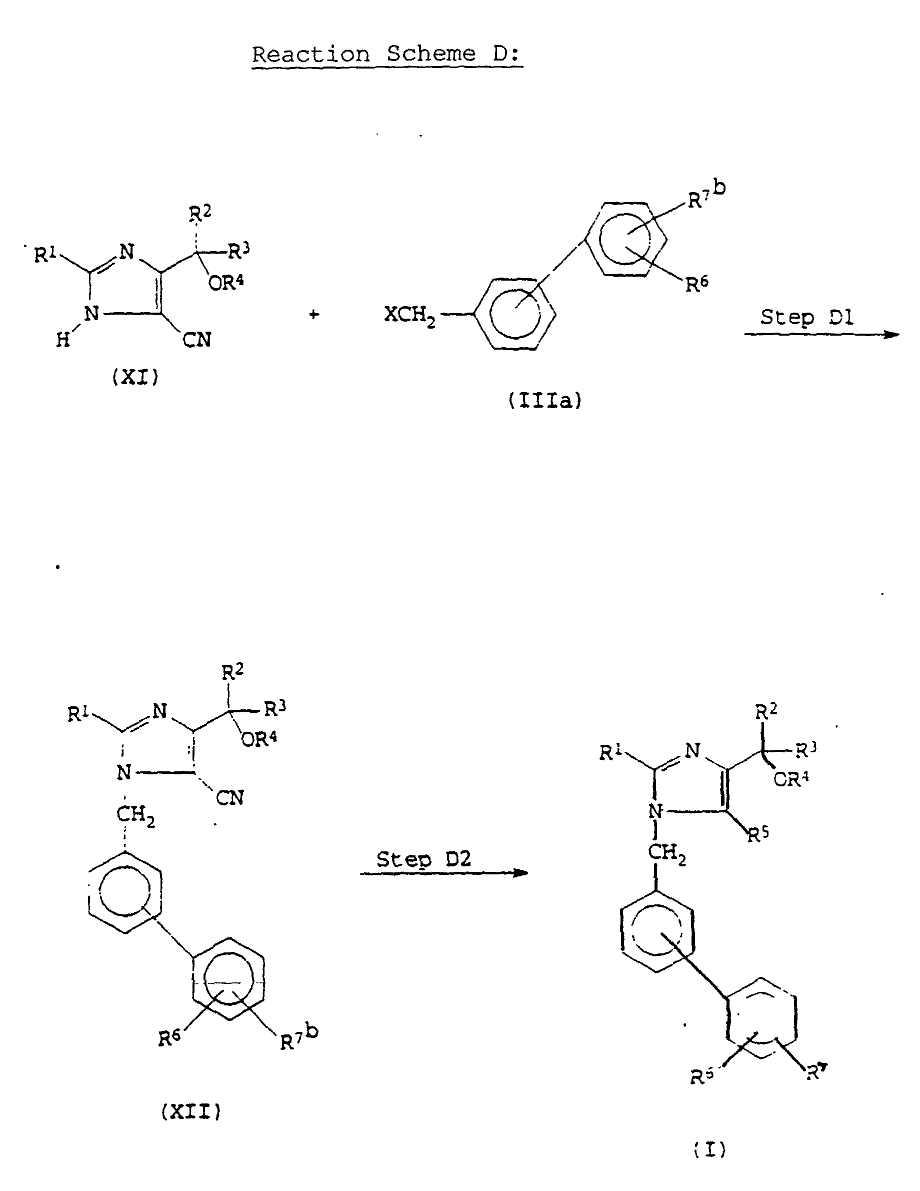

In the above formulae, R¹, R², R³, R⁴, R⁵, R⁶, R⁷ and X are as defined above, and R^{7b} represents a protected carboxy group or a protected tetrazolyl group, both of which may be as previously exemplified in relation to R^{7a}.

In Step D1 of this reaction scheme, an imidazole-5- carbonitrile compound of formula (XI) is reacted with a biphenylmethyl compound of formula (IIIa), to give a compound of formula (XII). This reaction is essentially the same as that described above in Step A1 of Reaction Scheme A, and may be carried out using the same reagents and reaction conditions.

In Step D2, the resulting compound of formula (XII) may be subjected to any one or (in appropriate cases) more of the following reactions:
(ix) converting the cyano group at the 5-position of the imidazole ring to a carboxy group;
(x) converting the cyano group at the 5-position of the imidazole ring to a carbamoyl group;
(xi) removing any carboxy-protecting groups;
(xii) esterifying the carboxy group at the 5-position of the imidazole ring or on the biphenyl group;
(xiii) converting the carboxy group at the 5-position of the imidazole ring to a group of formula -CONR⁸R⁹;
(xiv) removing the tetrazolyl-protecting group;
(xv) where R⁴ represents an aliphatic acyl group which can be regarded as a hydroxy-protecting group, removing the protecting group to produce a compound in which R⁴ represents a hydrogen atom; and
(xvi) where R⁴ represents a hydroxy group, alkylating or acylating this group.

The above reactions may be carried out as follows:

### (ix) Conversion of a cyano group to a carboxy group

The conversion is effected by hydrolysis of the cyano group in the compound of formula (XII) via a carbamoyl group. This reaction is well known in chemical synthesis generally, and may be carried out using any reagent known for this purpose. For example, an alkali metal hydroxide, such as sodium hydroxide, potassium hydroxide or lithium hydroxide.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: water; alcohols, such as methanol or ethanol; ethers, such as tetrahydrofuran or dioxane; or a mixture of any two or more of these solvents; an aqueous solvent is preferred.

The reaction can take place over a wide range of temperatures, and the precise reaction temperature is not critical to the invention. In general, we find it convenient to carry out the reaction at a temperature of from 0°C to 120°C, more preferably from 20°C to 100° C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the desired product can be recovered from the reaction mixture by conventional means. For example, one suitable recovery procedure comprises: neutralizing the reaction mixture by adding a mineral acid, such as hydrochloric acid; if the desired product of formula (I) precipitates, it can then be recovered by filtration; alternatively, after neutralizing the reaction mixture, the solvent is distilled off and the resulting residue is purified by column chromatography to give the desired product; alternatively, the residue is mixed with water and with a water-immiscible solvent, such as ethyl acetate, and the resulting mixture is extracted with an organic solvent, after which the extract is dried over a drying agent, such as anhydrous magnesium sulphate, and freed from the solvent to give the desired product. If necessary, the product can be further purified by conventional means, for example, by recrystallization, or by the various chromatography techniques, notably preparative thin layer chromatography or column chromatography.

In this reaction, where the starting material is a compound, in which R⁴ represents an acyl group and/or R^{7b} represents an ester group of a primary or secondary alcohol (such as methanol, ethanol or isopropanol), the acyl group of R⁴ and the ester residue of R^{7b} are simultaneously removed.

### (x) Conversion of a cyano group to a carbamoyl group

In this reaction, a cyano group in the compound of formula (XII) is converted to a carbamoyl group.

The product of this reaction is an intermediate of the previous reaction (ix). Therefore the reaction is carried out in a similar way but under milder conditions than those employed in reaction (ix).

The reaction is carried out by treating the compound of formula (XII) with an alkali, for example: an alkali metal hydroxide, such as lithium hydroxide, sodium hydroxide or potassium hydroxide; or an alkali metal carbonate, such as sodium carbonate or potassium carbonate. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: water; a mixture of water and an alcohol, such as methanol or ethanol; or a mixture of water and an ether, such as tetrahydrofuran or dioxane.

The reaction can take place over a wide range of temperatures, and the precise reaction temperature is not critical to the invention. In general, we find it convenient to carry out the reaction at a temperature of from 0°C to 100°C, more preferably from 10°C to 80° C. The time required for the reaction may also vary widely, depending on many factors, notably the reaction temperature and the nature of the reagents and solvent employed. However, provided that the reaction is effected under the preferred conditions outlined above, a period of from 0.5 to 24 hours, more preferably from 1 to 8 hours, will usually suffice. The reaction can be accelerated by adding a catalytic amount of hydrogen perioxide.

After completion of the reaction, the reaction product can be recovered from the reaction mixture by conventional means. For example, one suitable recovery procedure comprises: neutralizing the reaction mixture with a mineral acid, such as hydrochloric acid; distilling off the solvent under reduced pressure; adding water to the residue; extracting the mixture with a water-immiscible solvent, such as ethyl acetate; drying the organic extract solution over a drying agent, such as anhydrous magnesium sulphate; and distilling off the solvent. If necessary, the product can be further purified by conventional means, for example, by recrystallization, or by the various chromatography techniques, notably preparative thin layer chromatography or column chromatography.

### (xi) Removing carboxy-protecting groups

This is the same reaction as is involved in reaction (i) of Step A2 of Reaction Scheme A, and may be carried out using the same reagents and reaction conditions.

### (xii) Esterification

This is the same reaction as is involved in reaction (ii) of Step A2, and may be carried out using the same reagents and reaction conditions.

### (xiii) Conversion of a carboxy group to a group of formula -CONR⁸R⁹

This is the same reaction as is involved in reaction (iii) of

### (xiv) Removal of tetrazolyl-protecting groups

This is the same reaction as is involved in reaction (iv) of Step A2, and may be carried out using the same reagents and reaction conditions.

### (xv) Removing hydroxy-protecting groups

This is the same reaction as is involved in reaction (vii) of Step A2, and may be carried out using the same reagents and reaction conditions.

### (xvi) Alkylation and acylation of hydroxy groups

This is the same reaction as is involved in reaction (viii) of Step A2, and may be carried out using the same reagents and reaction conditions.

### Reaction Scheme E:

In this reaction scheme, a compound of formula (XII) in which R⁴ is hydrogen, that is to say a compound of formula (XV), is prepared from the corresponding compound of formula (XIII) having a ketonic [-C(O)R²] group at the 4-position of the imidazole ring.

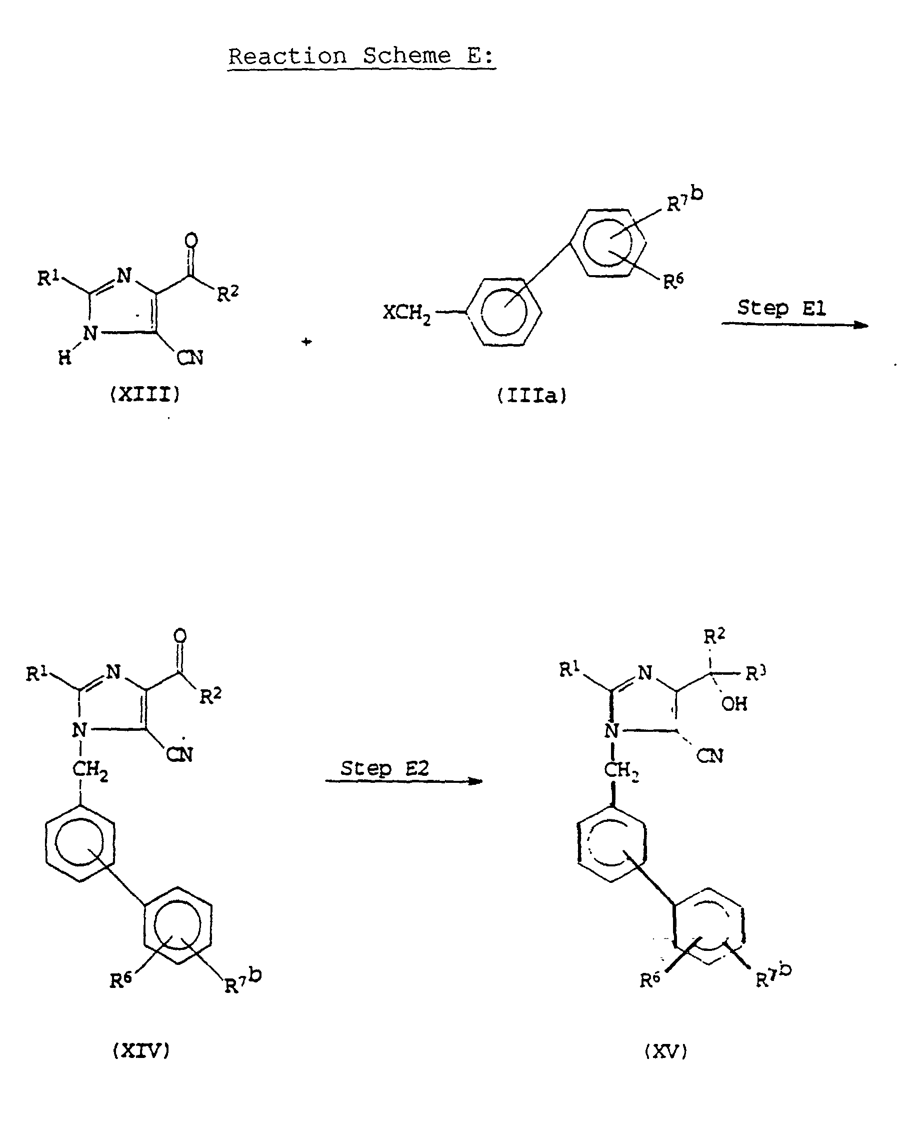

In the above formulae, R¹, R², R³, R⁶, R^{7b} and X are as defined above.

In Step E1 of this reaction scheme, an imidazole-5- carboxylate compound of formula (XIII) is reacted with a biphenylmethyl compound of formula (IIIa), to give a compound of formula (XIV). This reaction is essentially the same as that described above in Step A1 of Reaction Scheme A, and may be carried out using the same reagents and reaction conditions.

The resulting compound of formula (XIV) is then reacted in Step E2 with a reducing agent or with a Grignard reagent of formula, R^{3a}-Mg-X (in which R^{3a} and X are as defined above). This reaction is essentially the same as that described above in Step B2 of Reaction Scheme B, and may be carried out using the same reagents and reaction conditions. The resulting product may then be recovered and, if desired, further purified, as described in Step B2.

### Reaction Scheme F:

Certain 5-cyanoimidazole derivatives, for use as intermediates in the foregoing reaction schemes may be prepared as illustrated in the following Reaction Scheme F:

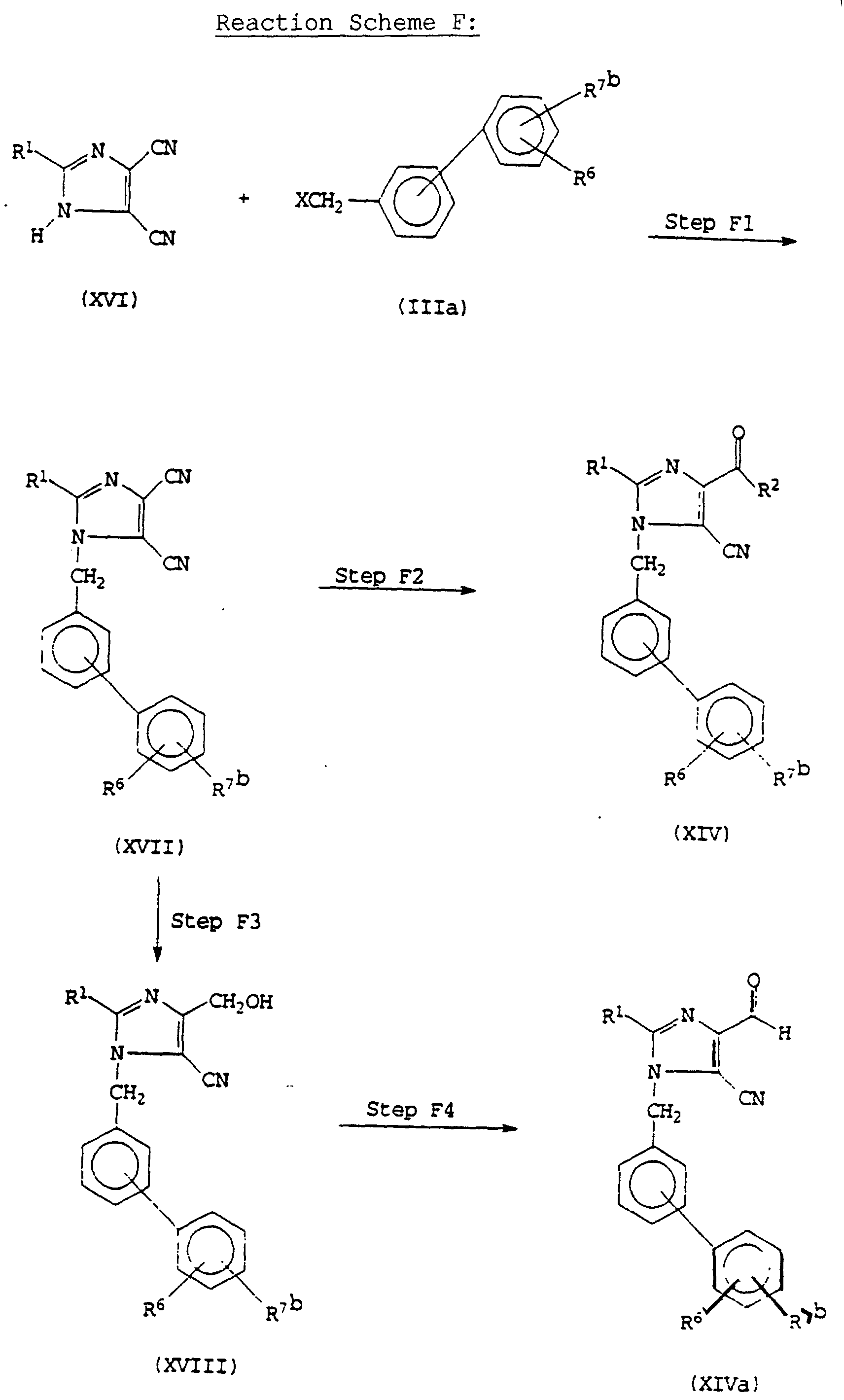

In the above formulae, R¹, R², R⁶, R^{7b} and X are as defined above.

In Step F1 of this reaction scheme, an imidazole-5- carboxylate compound of formula (XVI) is reacted with a biphenylmethyl compound of formula (IIIa), to give a compound of formula (XVII). This reaction is essentially the same as that described above in Step A1 of Reaction Scheme A, and may be carried out using the same reagents and reaction conditions.

Steps F2, F3 and F4 are essentially the same as Steps C2, C3 and C4, respectively, of Reaction Scheme C, and may be carried out using the same reagents and reaction conditions. The resulting product may then be recovered and, if desired, further purified, as described in Reaction Scheme C.

The preparation of certain of the starting materials used in the above reaction schemes is shown in Reaction Schemes G and H:

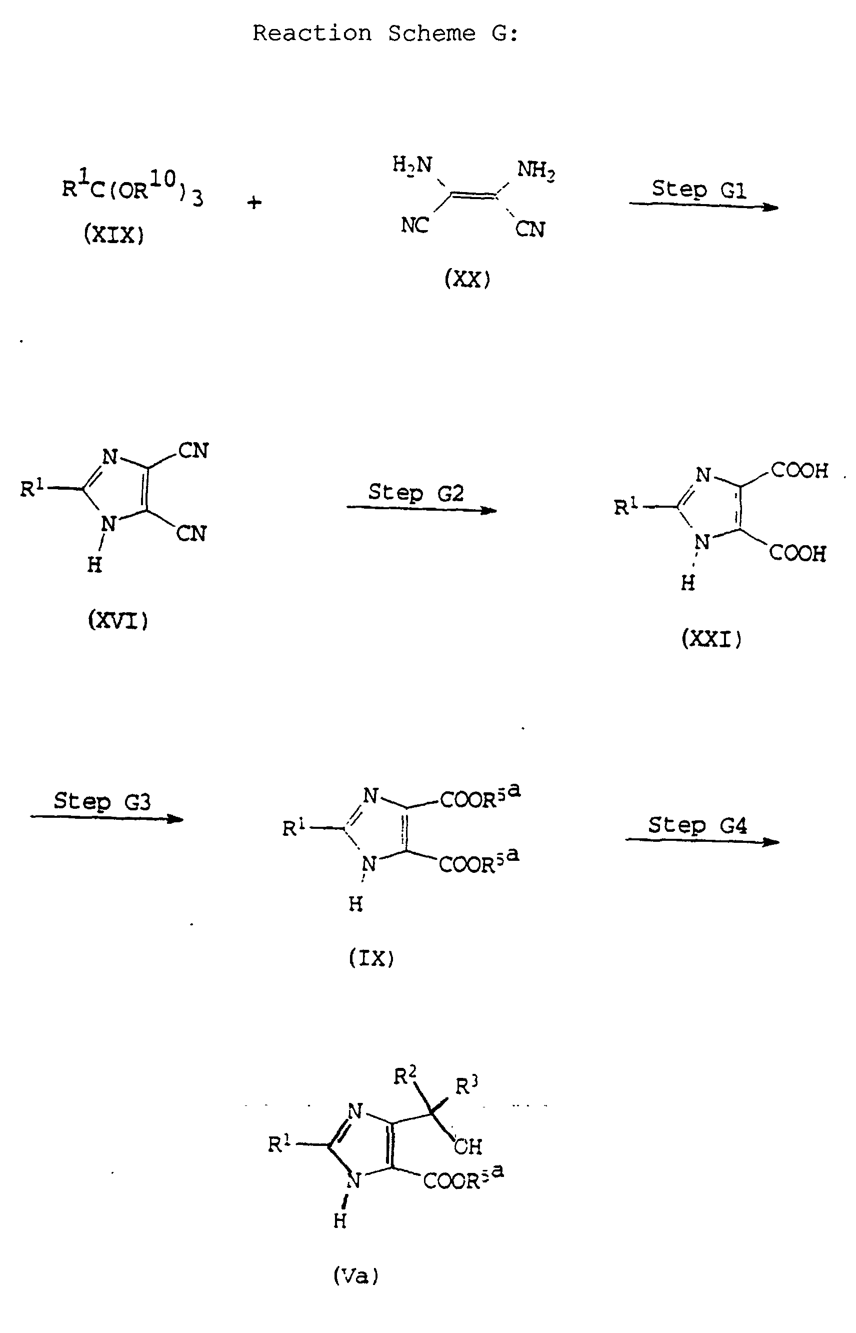

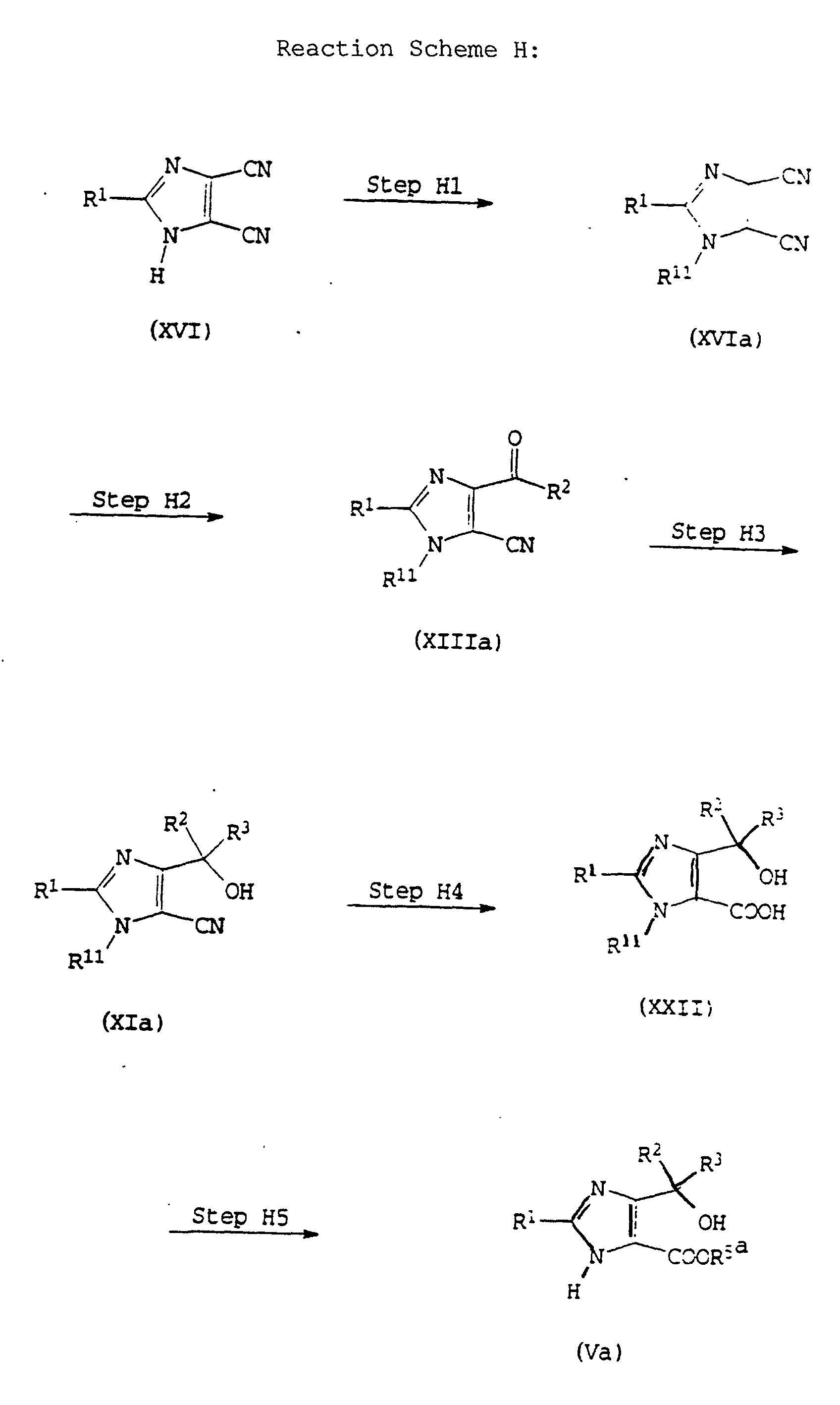

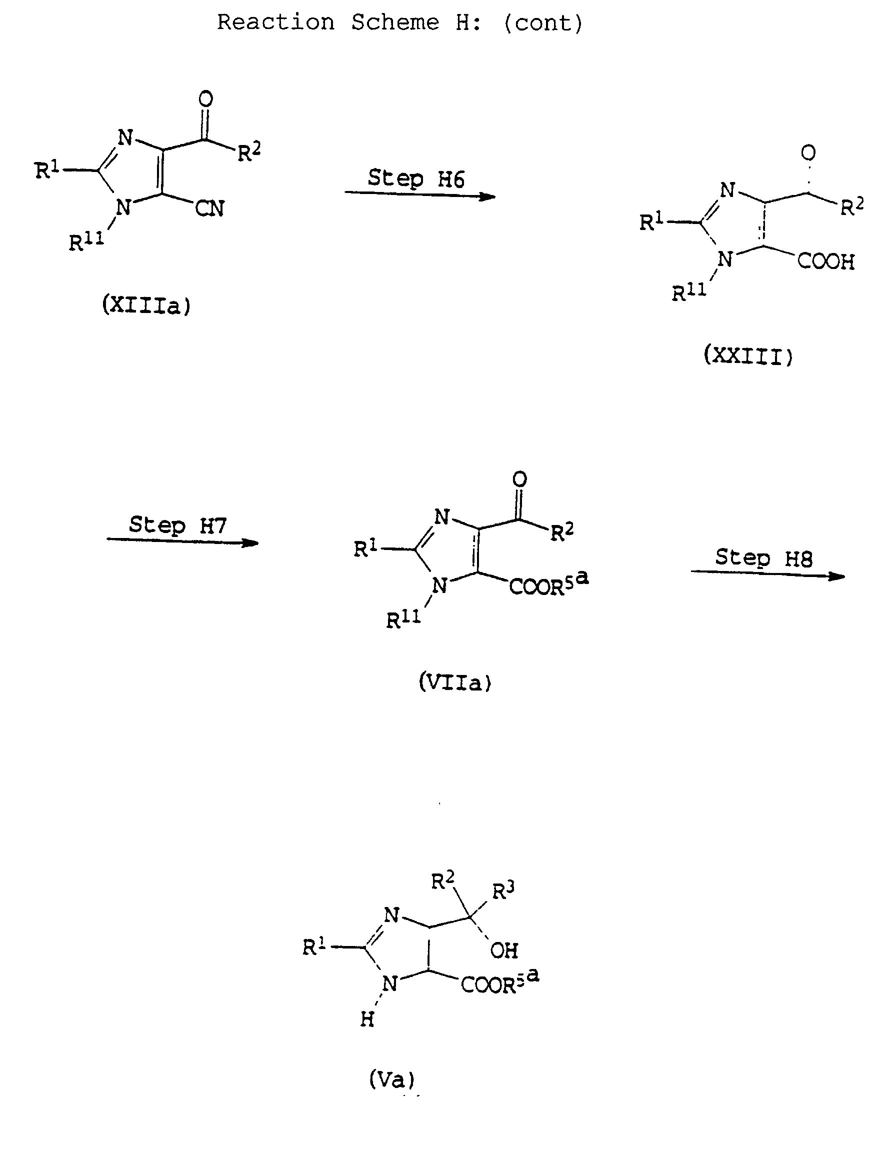

In the above formulae, R¹, R², R³ and R^{5a} are as defined above. R¹⁰ represents an alkyl group containing from 1 to 6 carbon atoms, such as those illustrated above in respect of R¹, and is preferably an alkyl group having from 1 to 4 carbon atoms, and more preferably a methyl or ethyl group. R¹¹ represents a hydrogen atom or a imidazolyl-protecting group, for example an aralkyl group, such as a trityl group, a diphenylmethyl group or a benzyl group, or an alkoxymethyl group in which the alkoxy part has from 1 to 4 carbon atoms, such as a methoxymethyl, ethoxymethyl, propoxymethyl or butoxymethyl group, preferably a trityl group, a benzyl group, a methoxymethyl group or an ethoxymethyl group, more preferably a trityl group.

### Reaction Scheme G:

In this Reaction Scheme G, a compound of formula (V) in which R⁴ represents a hydrogen atom, that is a compound of formula (Va), (IX) or (XVI) (which are starting materials in Reaction Schemes A, C or F, respectively) is prepared. The compound of formula (Va) may then, if desired, be protected, e.g. by alkylation, acylation, formation of a tetrahydropyranyloxy, tetrahydrothiopyranyloxy, tetrahydrothienyloxy or tetrahydrofuryloxy group, a substituted tetrahydropyranyloxy, tetrahydrothiopyranyloxy, tetrahydrothienyloxy or tetrahydrofuryloxy group or a group of formula - SiR^{a}R^{b}R^{c}, in which R^{a}, R^{b} and R^{c} are as defined above. These reactions, other than formation of an optionally substituted tetrahydropyranyloxy, tetrahydrothiopyranyloxy, tetrahydrothienyloxy or tetrahydrofuryloxy group, may be carried out as described in reaction (viii) of Step A2 of Reaction Scheme A, to give the corresponding compound in which R⁴ represents any of the groups represented by R⁴ other than a hydrogen atom.

Formation of a tetrahydropyranyloxy, tetrahydrothiopyranyloxy, tetrahydrothienyloxy or tetrahydrofuryloxy group or a substituted tetrahydropyranyloxy, tetrahydrothiopyranyloxy, tetrahydrothienyloxy or tetrahydrofuryloxy group may be carried out by reacting a compound of formula (V) in which R⁴ represents a hydrogen atom with dihydropyran, dihydrothiopyran, dihydrothiophene or dihydrofuran or a substituted dihydropyran, dihydrothiopyran, dihydrothiophene or dihydrofuran having at least one halogen or C₁ - C₆ alkoxy substituent in the presence of an acid (such as p-toluenesulphonic acid) in an inert solvent (for example a halogenated hydrocarbon, such as methylene chloride) at about room temperature for from 1 to 24 hours.

In Step G1, a compound of formula (XVI) is prepared by reacting an ortho ester compound of formula (XIX) with diaminomaleonitrile, which has the formula (XX). The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: aromatic hydrocarbons, such as benzene, toluene or xylene; halogenated hydrocarbons, especially halogenated aliphatic hydrocarbons, such as 1,2-dichloroethane or carbon tetrachloride; ethers, such as tetrahydrofuran or dioxane; and nitriles, such as acetonitrile.

The reaction can take place over a wide range of temperatures, and the precise reaction temperature is not critical to the invention. In general, we find it convenient to carry out the reaction at a temperature of from 50°C to 180°C, more preferably from 80°C to 150 °C. The time required for the reaction may also vary widely, depending on many factors, notably the reaction temperature and the nature of the reagents and solvent employed. However, provided that the reaction is effected under the preferred conditions outlined above, a period of from 1 to 24 hours, more preferably from 2 to 10 hours, will usually suffice.

The reaction product of formula (XVI) can be recovered by collecting the crystals deposited in the reaction system or by distilling off the solvent. The product can, if necessary, be further purified by conventional means, for example, by recrystallization, or by the various chromatography techniques, notably preparative thin layer chromatography or column chromatography.

Step G2 consists of preparing an imidazole-4,5-dicarboxylic acid compound of formula (XXI) by hydrolyzing the compound of formula (XVI) prepared in Step G1. This reaction may be carried out by heating the compound of formula (XVI) under reflux with an aqueous mineral acid, such as aqueous hydrochloric acid, sulphuric acid or nitric acid, for a period of from 1 to 24 hours (preferably from 3 to 16 hours). The product of formula (XXI) can be recovered by collecting the crystals deposited in the reaction mixture upon cooling, by filtration or by distilling off the solvent.

Step G3, an optional step, consists of preparing a diester compound of formula (IX) by protecting the carboxy group of the imidazole-4,5-dicarboxylic acid compound of formula (XXI) prepared in Step G2. This reaction may be carried out by reacting the compound (XXI) with a compound of formula R^{5b}-Y, in which R^{5b} and Y are as defined above.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, especially aromatic hydrocarbons, such as benzene or toluene; halogenated hydrocarbons, especially halogenated aliphatic hydrocarbons, such as methylene chloride or chloroform; ethers, such as tetrahydrofuran or dioxane; alcohols, such as methanol, ethanol or t-butanol; amides, such as N,N-dimethylacetamide, N,N-dimethylformamide or N-methyl-2-pyrrolidinone; ketones, such as acetone or methyl ethyl ketone; nitriles, such as acetonitrile; and sulphoxides, such as dimethyl sulphoxide. Of these, we prefer the nitriles, halogenated hydrocarbons or amides.

We also prefer that the reaction should be carried out in the presence of a base, the nature of which is not critical, provided that it does not affect any other parts of the reagents. Preferred examples of bases which may be used include: organic amines, such as triethylamine, N,N-diisopropylethylamine or N-methylmorpholine.

The reaction can take place over a wide range of temperatures, and the precise reaction temperature is not critical to the invention, although the preferred temperature may varies depending upon the nature of the starting material, the solvent and the base. In general, we find it convenient to carry out the reaction at a temperature of from -10°C to 100°C, more preferably from 0°C to 80°C. The time required for the reaction may also vary widely, depending on many factors, notably the reaction temperature and the nature of the reagents and solvent employed. However, provided that the reaction is effected under the preferred conditions outlined above, a period of from 0.5 to 24 hours, more preferably from 1 to 16 hours, will usually suffice.

After completion of the reaction, the desired compound can be recovered from the reaction mixture by conventional means. For example, after distilling off the solvent, the residue is mixed with water; the mixture is extracted with a water-immiscible organic solvent, such as ethyl acetate; the extract is dried over a drying agent, such as anhydrous magnesium sulphate; and the solvent is distilled off. The product can, if necessary, be further purified by conventional means, for example, by recrystallization, or by the various chromatography techniques, notably preparative thin layer chromatography or column chromatography.

Alternatively, the dicarboxylic acid compound of formula (XXI) may be esterified, to give the diester of formula (IX). The reaction employed for this will, as is well known in the art, depend on the nature of the ester residue R^{5b}.

For example, where the group represented by R^{5b} is an alkyl group or an aralkyl group, such as a benzyl group, the compound of formula (IX) can be prepared by reacting the corresponding dicarboxylic acid with methanol or ethanol, or benzyl alcohol, in the presence of an acid catalyst, such as hydrogen chloride or sulphuric acid in an inert solvent (for example: methanol or ethanol which may be used as the starting material described above; a halogenated hydrocarbon, such as methylene chloride; or an ether, such as tetrahydrofuran or dioxane) at a temperature of from 0°C to 100°C, preferably from 20°C to 80°C, for a period of from 1 hour to 3 days, preferably from 16 to 24 hours; or by treating the corresponding dicarboxylic acid with a halogenating agent (e.g. phosphorus pentachloride, thionyl chloride or oxalyl chloride) in an inert solvent (for example: a halogenated hydrocarbon, such as methylene chloride; an ether, such as tetrahydrofuran or dioxane; or an aromatic hydrocarbon, such as benzene or toluene) at about room temperature for a period of from 30 minutes to 5 hours, preferably from 1 to 3 hours, to give the corresponding acyl halide and then reacting this acyl halide with the corresponding alcohol (when the t-butyl ester is prepared, it is desirable to use potassium t-butoxide in place of the alcohol) in an inert solvent (e.g. benzene or methylene chloride) in the presence of a base (e.g. triethylamine) at about room temperature for a period of from 30 minutes to 10 hours.

The desired compound can be recovered from the reaction mixture by conventional means. For example, after distilling off the solvent, the residue is dissolved in water and a water-immiscible organic solvent, such as ethyl acetate, and the resulting solution is neutralized with sodium hydrogencarbonate; the organic layer is then separated and dried over a drying agent, such as anhydrous magnesium sulphate; the solvent is then distilled off to leave the desired product. The product can, if necessary, be further purified by conventional means, for example, by recrystallization, or by the various chromatography techniques, notably preparative thin layer chromatography or column chromatography.

In Step G4, a compound of formula (Va) is prepared by reacting a diester compound of formula (IX) with a Grignard reagent of formula R^{2a}MgX and/or R^{3a}MgX (in which R^{2a}, R^{3a} and X are as defined above).

The reaction is essentially the same as that described above in Step B2 of Reaction Scheme B, and may be carried out using the same reagents and reaction conditions.

### Reaction Scheme H:

These reactions prepare compounds of formulae (XIIIa), (XIa) and (VIIa), in each of which R¹¹ is a hydrogen atom, that is to say compounds of formulae (XIII), (XI) and (VII), and a compound of formula (Va), which are starting materials used in Reaction Schemes E, D, A and B, respectively.

In Step H1, which is an optional step, a compound of formula (XVIa) is prepared by reacting a dinitrile compound of formula (XVI) with a compound of formula R^{11a}-X (in which X is as defined above and R^{11a} represents any of the groups defined above for R¹¹ other than a hydrogen atom) in the presence of a base.

Examples of suitable bases include: alkali metal hydrides, such as lithium hydride or sodium hydride; alkali metal carbonates, such as sodium carbonate or potassium carbonate; and alkali metal alkoxides, such as sodium methoxide, sodium ethoxide or potassium t-butoxide.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, such as methylene chloride or chloroform; ethers, such as tetrahydrofuran or dioxane; amides, such as dimethylformamide or dimethylacetamide; and ketones, such as acetone or methyl ethyl ketone. The reaction can take place over a wide range of temperatures, and the precise reaction temperature is not critical to the invention. In general, we find it convenient to carry out the reaction at a temperature of from 0°C to 120°C, more preferably from 20°C to 80°C. The time required for the reaction may also vary widely, depending on many factors, notably the reaction temperature and the nature of the reagents and solvent employed. However, provided that the reaction is effected under the preferred conditions outlined above, a period of from 1 to 24 hours, more preferably from 3 to 8 hours, will usually suffice.

After completion of the reaction, the desired compound can be recovered from the reaction mixture by conventional means. For example, one suitable recovery procedure comprises: adding water to the reaction mixture; extracting the mixture with a water-miscible organic solvent, such as ethyl acetate; washing the extract with water and drying it over a drying agent, such as anhydrous magnesium sulphate; and finally distilling off the solvent. The product can, if necessary, be further purified by conventional means, for example, by recrystallization, or by the various chromatography techniques, notably preparative thin layer chromatography or column chromatography.

In Step H2, a compound of formula (XIIIa) is prepared by reacting a dinitrile compound of formula (XVIa) with a Grignard reagent of formula R^{2a}MgX, in which R^{2a} and X are as defined above, or with a reducing agent. This reaction is essentially the same as that described above in Step B2 of Reaction Scheme B, and may be carried out using the same reagents and reaction conditions.

An imidazolyl-protecting group of a compound of formula (XIIIa) may optionally be removed by treating the compound of formula (XIIIa) in a conventional manner, depending on the nature of the protecting group, to give the compound of formula (XIII).

For example, when the protecting group is a trityl group or an alkoxymethyl group, it may be removed by reacting the protected compound with an acid.

Examples of suitable acids include: inorganic acids, such as hydrochloric acid or sulphuric acid; and organic acids, such as acetic acid, formic acid, trifluoroacetic acid, methanesulphonic acid or p-toluenesulphonic acid.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: ethers, such as tetrahydrofuran or dioxane; alcohols, such as methanol or ethanol; acids, such as acetic acid; water; or a mixture of any two or more of the above solvents.

The reaction can take place over a wide range of temperatures, and the precise reaction temperature is not critical to the invention. In general, we find it convenient to carry out the reaction at a temperature of from 0°C to 120°C, more preferably from 10°C to 100° C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the desired compound can be recovered from the reaction mixture by conventional means. For example, one suitable recovery procedure comprises: evaporating the solvent and purifying the product by recrystallization or chromatography; or neutralizing the reaction mixture with a weak base (such as sodium hydrogencarbonate), extracting with a water-immiscible organic solvent, such as ethyl acetate, and evaporating off the solvent. The product can, if necessary, be further purified by conventional means, for example, by recrystallization, or by the various chromatography techniques, notably preparative thin layer chromatography or column chromatography.

When the imidazolyl-protecting group is an aralkyl group, such as a benzyl or diphenylmethyl group, it can be removed by catalytic hydrogenation. The reaction is essentially the same as that described above in reaction (i) of Step A2 of Reaction Scheme A, in which the carboxy-protecting group is an an aralkyl group, and may be carried out using the same reagents and reaction conditions.

In Step H3, the resulting carbonyl compound of formula (XIIIa) is then reacted with a Grignard reagent of formula R^{3a}MgX, in which R^{3a} and X are as defined above, or with a reducing agent, to give the compound of formula (XIa). This reaction is essentially the same as that described above in Step B2 of Reaction Scheme B, and may be carried out using the same reagents and reaction conditions.

If desired, the imidazolyl-protecting group of the compound of formula (XIa) can be removed by essentially the same reaction as that optional reaction described above as Step H2 of Reaction Scheme H, which may be carried out using the same reagents and reaction conditions.

In Step H4, a carboxylic acid compound of formula (XXII) is prepared by hydrolyzing the remaining cyano group at the 5-position of the imidazole ring. The reaction may be carried out using an alkali metal hydroxide, such as sodium hydroxide, potassium hydroxide or lithium hydroxide, in an inert solvent (preferably water; an alcohol, such as methanol or ethanol; an ether, such as tetrahydrofuran or dioxane; or a mixture of any two or more of the above solvents). The reaction can take place over a wide range of temperatures, and the precise reaction temperature is not critical to the invention. In general, we find it convenient to carry out the reaction at a temperature of from 0°C to 120°C, more preferably from 20°C to 100°C. The time required for the reaction may also vary widely, depending on many factors, notably the reaction temperature and the nature of the reagents and solvent employed. However, provided that the reaction is effected under the preferred conditions outlined above, a period of from 0.5 to 24 hours, more preferably from 1 to 16 hours, will usually suffice. After completion of the reaction, the reaction product can be recovered by conventional means. For example, the reaction mixture is neutralized by adding a mineral acid, such as hydrochloric acid; if the desired compound of formula (XXII) appears as a precipitate in the reaction medium, it can be collected by filtration. Alternatively, the desired compound can be recovered as follows: after neutralizing the reaction mixture, the solvent is distilled off and the residue is subjected to column chromatography; alternatively, the residue may be mixed with water and a water-immiscible organic solvent and extracted with the organic solvent, after which the extract is dried over a drying agent, such as anhydrous magnesium sulphate, and the solvent is distilled off to leave the desired product. The product can, if necessary, be further purified by conventional means, for example, by recrystallization, or by the various chromatography techniques, notably preparative thin layer chromatography or column chromatography.

In Step H5, an optional step, a compound of formula (Va) is prepared by esterification of the carboxylic acid compound of formula (XXII), optionally followed by deprotection of the imidazolyl group. This esterification reaction is essentially the same as that described above in reaction (ii) of Step A2 of Reaction Scheme A, and the optional deprotection is essentially the same as Step H2 of Reaction Scheme H, and each may be carried out using the same reagents and reaction conditions.

In Step H6, a compound of formula (XXIII) is prepared by hydrolysing a compound of formula (XIIIa). This reaction is essentially the same as that described above in Step H4 of Reaction Scheme H, and may be carried out using the same reagents and reaction conditions.

In Step H7, a compound of formula (VIIa) is prepared by esterification of the compound of formula (XXIII). This reaction is essentially the same as that described above in Step H5 of Reaction Scheme H, and may be carried out using the same reagents and reaction conditions.

If desired, the imidazolyl-protecting group of the compound of formula (VIIa) can be removed by essentially the same reaction as that optional reaction described above as Step H2 of Reaction Scheme H, which may be carried out using the same reagents and reaction conditions.

In Step H8, a compound of formula (Va) is prepared by reacting a compound of formula (VIIa) with a Grignard reagent and/or a reducing agent, and then optionally deprotecting the imidazolyl group. This reaction is essentially the same as that described above in Step B2 of Reaction Scheme B, and the optional deprotection is essentially the same as Step H2 of Reaction Scheme H, and each may be carried out using the same reagents and reaction conditions.

The compounds of the present invention can form salts. There is no particular restriction on the nature of these salts, provided that, where they are intended for therapeutic use, they are pharmaceutically acceptable. Where they are intended for non-therapeutic uses, e.g. as intermediates in the preparation of other, and possibly more active, compounds, even this restriction does not apply. The compounds of the present invention can form salts with bases. Examples of such salts include: salts with an alkali metal, such as sodium, potassium or lithium; salts with an alkaline earth metal, such as barium or calcium; salts with another metal, such as magnesium or aluminium; organic base salts, such as a salt with dicyclohexylamine, guanidine or triethylamine; and salts with a basic amino acid, such as lysine or arginine. Also, the compound of the present invention contains a basic group in its molecule and can therefore form acid addition salts. Examples of such acid addition salts include: salts with mineral acids, especially hydrohalic acids (such as hydrofluoric acid, hydrobromic acid, hydroiodic acid or hydrochloric acid), nitric acid, carbonic acid, sulphuric acid or phosphoric acid; salts with lower alkylsulphonic acids, such as methanesulphonic acid, trifluoromethanesulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid or citric acid; and salts with amino acids, such as glutamic acid or aspartic acid. The compounds of the present invention can be converted to a pharmaceutically acceptable salt by treatment with an acid or a base by conventional means, as is well known in the art.

The compounds of the present invention exhibit an excellent inhibitory effect against the elevation of blood pressure induced by angiotensin II and are therefore extremely useful for prevention or treatment of circulatory diseases as a hypotensive drug or a therapeutic drug for heart diseases.

Their biological activity was determined by the following experiment.

### Evaluation of All receptor blocking activity by Inhibition of pressor response to angiotensin II

The biological activity of each compound was assessed by determining the dose required to inhibit the pressor response to intravenous angiotensin II by fifty percent (ID₅₀) in rats. Male Wister-Imamichi rats, each weighing 300 to 400 g, were anesthesized by intraperitoneal injection of 100 mg/Kg of sodium thiobutabarbital [Inactin (trade name)] and two cannulae were inserted: one into the femoral artery for measuring blood pressure and the other into the femoral vein for injecting drugs. Fifty ng/kg of angiotension II were intravenously administered at intervals of about 10 minutes, and the elevation of blood pressure (normally about 50 mmHg) was observed. After constant pressor responses to angiotensin II were obtained, a test compound was intravenously administered. Two minutes later, angiotension II was again injected, and the inhibitory effect of the test compound was estimated. The percent inhibitions of the pressor response to angiotensin II by progressive increase of the test compound was used to calculate the value of ID₅₀. Angiotensin II was used in this test dissolved in 0.5 % bovine serum albumin (BSA) and the test compounds were dissolved in 100% dimethyl sulphoxide (DMSO). Table 7 shows the ID₅₀ values thus determined.

In addition to the compounds of the invention (which are identified hereafter by the number of the one of the following Examples which illustrates their preparation), we also carried out the same experiment using a prior art compound (identified in the Table as "compound A"), which is 2-[4-(2-butyl-5-chloro-4-chloromethylimidazoll-ylmethyl)phenyl]benzoic acid, which is disclosed in Example 118 of European Patent Publication No. 253 310.

**Table 7**

| Test compound (Compound of Example No.) | ID50 (mg/kg, i.v.) |
|---|---|
| 5 | 0.22 |
| 11 | 0.25 |
| 36 | 0.0062 |
| 39 | 0.010 |
| 41 | 0.0063 |
| 44 | 0.0082 |
| 45 | 0.19 |
| 46 | 0.18 |
| 48 | 0.064 |
| 55 | 0.23 |
| 59 | 0.066 |
| 60 | 0.134 |
| 74 | 0.036 |
| 75 | 0.11 |
| 76 | 0.022 |
| A | 3.3 |

The compounds of the present invention can be administered, for example, orally in the form of tablets, capsules, granules, powders, syrups or the like, or parenterally by injection, suppository or the like. These pharmaceutical preparations can be produced in the conventional manner using the adjuvants generally known in the art, such as excipients, binders, disintegrating agents, lubricants, stabilizers, corrigents and the like. Although the dosage may vary depending upon the symptoms and age of the patient, the nature and severity of the disease or disorder and the route and manner of administration, in the case of oral administration to an adult human patient, the compounds of the present invention may normally be administered at a total daily dose of from 1 to 1000 mg, preferably from 5 to 300 mg, either in a single dose, or in divided doses, for example two or three times a day; in the case of intravenous injection, a dose of from 0.1 to 100 mg, preferably from 0.5 to 30 mg, may be administered between one and three times a day.

The invention is further illustrated by the following Examples, which demonstrate the preparation of various of the compounds of the invention. The preparation of certain starting materials used in these Examples is shown in the subsequent Preparations.

### EXAMPLE 1

### Methyl 1-[(2'-t-butoxycarbonylbiphenyl-4-yl)methyl]-2-butyl-4-hydroxymethylimidazole-5-carboxylate (Compound No. 1-94)

### 1 (a) Dimethyl 1-[(2'-t-butoxycarbonylbiphenyl-4-yl)-methyl]-2-butylimidazole-4,5-dicarboxylate

A sodium methoxide solution prepared from 0.69 g of sodium and 40 ml of methanol was added to a solution of 7.2 g of dimethyl 2-butylimidazole-4,5-dicarboxylate (prepared as described in Preparation 4) in 40 ml of methanol, and the resulting mixture was concentrated by evaporation under reduced pressure. The resulting residue was mixed with benzene, and the mixture was concentrated by distillation under reduced pressure. After this operation had been repeated three times, the solid thus obtained was dissolved in 72 ml of N,N-dimethylacetamide. A solution of 10.41 g of t-butyl 4'-bromomethylbiphenyl-2-carboxylate in 100 ml of N,N-dimethylacetamide was then added dropwise to the resulting solution. The reaction mixture was then stirred at room temperature for 1 hour and at 50 - 55°C for 2 hours. At the end of this time, it was mixed with ethyl acetate and water, and the ethyl acetate layer was separated, and dried over anhydrous magnesium sulphate; the solvent was then removed by distillation under reduced pressure. The residue was purified by column chromatography through silica gel, using a 1 : 1 by volume mixture of ethyl acetate and hexane as the eluent, to give 15.1 g of the title compound as a gum.

Nuclear Magnetic Resonance Spectrum (CDCl₃) δ ppm:
0.90 (3H, triplet, J = 7 Hz);
1.26 (9H, triplet);
1.1 - 2.0 (4H, multiplet);
2.70 (2H, triplet, J = 7 Hz) ;
3.81 (3H, singlet);
3.90 (3H, singlet);
5.47 (2H, singlet);
6.95 - 7.85 (8H, multiplet).

### 1 (b) Methyl 1-[(2'-t-butoxycarbonylbiphenyl-4-yl)-methyl]-2-butyl-4-hydroxymethylimidazole-5-carboxylate

42 ml of diisobutylaluminium hydride (as a 1.5 M solution in in toluene) were added dropwise at a temperature between -20°C and -15°C to a solution of 16.0 g of dimethyl 1-[(2'-t-butoxycarbonylbiphenyl-4-yl)methyl]-2-butylimidazole-4,5-dicarboxylate [prepared as described in step (a) above] in 200 ml of tetrahydrofuran, and the resulting mixture was allowed to stand at 0 - 5°C for 16 hours. At the end of this time, the reaction mixture was mixed with an aqueous solution of ammonium chloride and ethyl acetate and was then stirred for 1 hour. After this, precipitates were removed by filtration. The ethyl acetate layer was then separated and dried over anhydrous magnesium sulphate, and the solvent was removed by distillation under reduced pressure. The residue was then purified by column chromatography through silica gel, using ethyl acetate as the eluent, to give 12.0 g of the title compound as crystals, melting at 99°C.

Nuclear Magnetic Resonance Spectrum (CDCl₃) δ ppm:
0.90 (3H, triplet, J = 7 Hz);
1.20 (9H, singlet);
1.1 - 2.0 (4H, multiplet);
2.69 (2H, triplet, J = 7 Hz);
3.55 (1H, broad singlet);
3.78 (3H, singlet);
4.84 (2H, doublet, J = 5 Hz);
5.60 (2H, singlet);
6.95 - 7.9 (8H, multiplet).

### EXAMPLE 2

### Ethyl 1-[(2'-t-butoxycarbonylbiphenyl-4-yl)methyl]-2-butyl-4-hydroxymethylimidazole-5-carboxylate (Compound No. 1-95)

### 2(a) Diethyl 1-[(2'-t-butoxycarbonylbiphenyl-4-yl)-methyl]-2-butylimidazole-4,5-dicarboxylate

Following a procedure similar to that described in Example 1(a), but using 8.0 g of diethyl 2-butylimidazole-4,5-dicarboxylate (prepared as described in Preparation 3) and 10.41 g of t-butyl 4'-bromomethylbiphenyl-2-carboxylate, 15.4 g of the title compound were obtained as a gum.

Nuclear Magnetic Resonance Spectrum (CDCl₃) δ ppm:
0.90 (3H, triplet, J = 7 Hz);
1.1 - 2.0 (4H, multiplet);
1.24 (9H, singlet);
1.26 (3H, triplet, J = 7 Hz);
1.39 (3H, triplet, J = 7 Hz);
2.72 (2H, triplet, J = 7 Hz);
4.28 (2H, quartet, J = 7 Hz);
4.40 (2H, quartet, J = 7 Hz);
5.50 (2H, singlet);
7.0 - 7.9 (8H, multiplet).

### 2(b) Ethyl 1-[(2'-t-butoxycarbonylbiphenyl-4-yl)-methyl]-2-butyl-4-hydroxymethylimidazole-5-carboxylate

Following a procedure similar to that described in Example 1(b), but using 1.50 g of diethyl 1-[(2'-t-butoxycarbonylbiphenyl-4-yl) methyl] -2-butylimidazole-4,5- dicarboxylate [prepared as described in step (a) above] and 3.9 ml of diisobutylaluminium hydride (as a 1.5 M solution in toluene), 1.1 g of the title compound was obtained as a gum.

Nuclear Magnetic Resonance Spectrum (CDCl₃) δ ppm:
0.90 (3H, triplet, J = 7 Hz);
1.24 (9H, singlet);
1.30 (3H, triplet, J = 7 Hz);
1.1 - 2.0 (4H, multiplet);
2.68 (2H, triplet, J = 7 Hz);
3.60 (1H, broad singlet);
4.24 (2H, quartet, J = 7 Hz) ;
4.84 (2H, singlet);
5.57 (2H, singlet);
6.9 - 7.85 (8H, multiplet).

### EXAMPLE 3

### Methyl 2-butyl-1-[(2'-carboxybiphenyl-4-yl)methyl]-4-hydroxymethylimidazole-5-carboxylate (Compound No. 1-5)

A solution of 0.36 g of methyl 1-[(2'-t-butoxy-carbonylbiphenyl-4-yl)methyl]-2-butyl-4-hydroxymethyl-imidazole-5-carboxylate (prepared as described in Example 1) in 4 ml of a 4 N solution of hydrogen chloride in dioxane was allowed to stand at room temperature for 4 hours. At the end of this time, the reaction mixture was concentrated by evaporation under reduced pressure, and the residue was triturated with ethyl acetate, to give crystals, which were collected by filtration to give 0.35 g of the title compound in the form of its hydrochloride, melting at 192-195°C (with decomposition).

Nuclear Magnetic Resonance Spectrum (hexadeuterated dimethyl sulphoxide) δ ppm:
0.81 (3H, triplet, J = 7 Hz);
1.22 - 1.35 (2H, multiplet);
1.43 - 1.56 (2H, multiplet);
3.00 (2H, triplet, J = 7 Hz);
3.82 (3H, singlet);
4.81 (2H, singlet);
5.77 (2H, singlet);
7.18 - 7.75 (8H, multiplet).

### EXAMPLE 4

### 1-[(2'-t-Butoxycarbonylbiphenyl-4-yl)methyl]-2-butyl-4-hydroxymethylimidazole-5-carboxylic acid (Compound No. 1-96)

A solution of 2.01 g of lithium hydroxide monohydrate in 97 ml of water was added to a solution of 4.78 g of methyl 1-[(2'-t-butoxycarbonylbiphenyl-4-yl)- methyl]-2-butyl-4-hydroxymethylimidazole-5-carboxylate (prepared as described in Example 1) in 48 ml of dioxane, and the resulting mixture was stirred at room temperature for 18 hours. At the end of this time, the reaction mixture was freed from dioxane by distillation under reduced pressure, and 47.6 ml of 1 N aqueous hydrochloric acid were added to the aqueous residue. The crystals which precipitated were collected by filtration and then washed with water and with diethyl ether, in that order, to give 4.26 g of the title compound, melting at 187°C (with decomposition).

Nuclear Magnetic Resonance Spectrum (CDCl₃) δ ppm:
0.85 (3H, triplet, J = 7 Hz);
1.24 (9H, singlet);
1.1 - 1.9 (4H, multiplet);
2.80 (2H, triplet, J = 7 Hz);
5.05 (2H, singlet);
5.93 (2H, singlet);
7.0 - 7.85 (8H, multiplet).

### EXAMPLE 5

### 2-Butyl-1-[(2'-carboxybiphenyl-4-yl)methyl]-4-hydroxymethylimidazole-5-carboxylic acid (Compound No. 1-2)

A solution of 0.12 g of 1-[(2'-t-butoxycarbonyl-biphenyl-4-yl)methyl]-2-butyl-4-hydroxymethylimidazole5-carboxylic acid (prepared as described in Example 4) in 2 ml of a 4 N solution of hydrogen chloride in dioxane was allowed to stand at room temperature for 5 hours and then the solvent was removed by distillation under reduced pressure. The resulting residue was triturated in ethyl acetate, to give 0.11 g of the title compound in the form of its hydrochloride, melting at 130 - 140°C (with softening).

Nuclear Magnetic Resonance Spectrum (hexadeuterated dimethyl sulphoxide) δ ppm:
0.80 (3H, triplet, J = 7 Hz);
1.2 - 1.33 (2H, multiplet);
1.4 - 1.53 (2H, multiplet);
2.98 (2H, triplet, J = 7 Hz);
4.84 (2H, singlet);
5.81 (2H, singlet);
7.17 - 7.74 (8H, multiplet).

### EXAMPLE 6

### Pivaloyloxymethyl 1-[(2'-t-butoxycarbonylbiphenyl-4-yl)methyl]-2-butyl-4-hydroxymethylimidazole-5-carboxylate (Compound No. 1-97)

350 mg of potassium carbonate were added to a solution of 552 mg of 1-[(2'-t-butoxycarbonylbiphenyl-4-yl)methyl]-2-butyl-4-hydroxymethylimidazole-5-carboxylic acid (prepared as described in Example 4) and 220 mg of pivaloyloxymethyl chloride in 7 ml of N,N-dimethylacetamide, and the resulting mixture was stirred at room temperature for 5 hours. At the end of this time, the reaction mixture was mixed with ethyl acetate and water, and the ethyl acetate layer was separated and dried over anhydrous magnesium sulphate; the solvent was then removed by distillation under reduced pressure. The resulting residue was purified by column chromatography through silica gel, using ethyl acetate as the eluent, to give 0.62 g of the title compound as a syrup.

Nuclear Magnetic Resonance Spectrum (CDCl₃) δ ppm:
0.91 (3H, triplet, J = 7 Hz);
1.18 (9H, singlet);
1.21 (9H, singlet);
1.1 - 2.0 (4H, multiplet);
2.72 (2H, triplet, J = 7 Hz);
3.35 (1H, broad);
4.85 (2H, doublet, J = 5 Hz);
5.61 (2H, singlet);
5.90 (2H, singlet);
6.95 - 7.9 (8H, multiplet).

### EXAMPLE 7

### Pivaloyloxymethyl 2-butyl-1-[(2'-carboxybiphenyl-4-yl)methyl]-4-hydroxymethylimidazole-5-carboxylate (Compound No. 1-98)

A solution of 0.62 g of pivaloyloxymethyl 1-[(2'-tbutoxycarbonylbiphenyl-4-yl) methyl]-2-butyl-4-hydroxymethylimidazole-5-carboxylate (prepared as described in Example 6) in 10 ml of a 4 N solution of hydrogen chloride in dioxane was allowed to stand at room temperature for 4 hours, after which it was concentrated by evaporation under reduced pressure. The syrupy residue was stirred in diethyl ether, and then the solvent was removed by decantation and the residue was dried in vacuo, to give 0.46 g of the hydrochloride of the title compound as a powder.

Nuclear Magnetic Resonance Spectrum (CDCl₃) δ ppm:
0.85 (3H, triplet, J = 7 Hz);
1.19 (9H, singlet);
1.25 - 1.45 (2H, multiplet);
1.65 - 1.80 (2H, multiplet);
2.99 (2H, triplet, J = 7 Hz);
5.01 (2H, singlet);
5.70 (2H, singlet);
5.89 (2H, singlet);
7.05 - 7.97 (8H, multiplet).

### EXAMPLE 8

### Methyl 1-[(2'-t-butoxycarbonylbiphenyl-4-yl)methyl]-2-butyl-4-(methoxymethyl)imidazole-5-carboxylate (Compound No. 1-99)

0.057 g of sodium hydride (as a 55% w/w dispersion in mineral oil) was added to a solution of 0.478 g of methyl 1-[(2'-t-butoxycarbonylbiphenyl-4-yl)methyl]-2-butyl-4-hydroxymethylimidazole-5-carboxylate (prepared as described in Example 1) in 5 ml of N,N-dimethyl-acetamide, and the resulting mixture was stirred at room temperature for 30 minutes. At the end of this time, 0.125 ml of iodomethane were added, and the reaction mixture was stirred at 50°C for 3 hours. The reaction mixture was then mixed with ethyl acetate and water. The ethyl acetate layer was separated and dried over anhydrous magnesium sulphate; the solvent was then removed by distillation under reduced pressure. The resulting residue was purified by column chromatography through silica gel, using a 1 : 1 by volume mixture of ethyl acetate and methylene chloride as the eluent, to give 0.30 g of the title compound as a gum.

Nuclear Magnetic Resonance Spectrum (CDCl₃) δ ppm:
0.90 (3H, triplet, J = 7 Hz);
1.24 (9H, singlet);
1.1 - 2.0 (4H, multiplet);
2.71 (2H, triplet, J = 7 Hz);
3.46 (3H, singlet);
3.80 (3H, singlet);
4.68 (2H, singlet);
5.60 (2H, singlet);
6.9 - 7.9 (8H, multiplet).

### EXAMPLE 9

### Methyl 2-butyl-1-[(2'-carboxybiphenyl-4-yl)methyl]-4-(methoxymethyl)imidazole-5-carboxylate (Compound No. 1-121)

A solution of 0.30 g of methyl 1-[(2'-t-butoxycarbonylbiphenyl-4-yl)methyl]-2-butyl-4-(methoxymethyl)- imidazole-5-carboxylate (prepared as described in Example 8) in 3 ml of a 4 N solution of hydrogen chloride in dioxane was allowed to stand at room temperature for 5 hours, after which the solvent was removed by distillation under reduced pressure. The syrupy residue was triturated in diethyl ether and collected by filtration, to give 0.26 g of the title compound in the form of its hydrochloride, melting at 106 - 110°C (with softening).

Nuclear Magnetic Resonance Spectrum (hexadeuterated dimethyl sulphoxide) δ ppm:
0.81 (3H, triplet, J = 7 Hz);
1.2 - 1.35 (2H, multiplet);
1.45 - 1.6 (2H, multiplet);
2.97 (2H, triplet, J = 7 Hz);
3.39 (3H, singlet);
3.82 (3H, singlet);
4.72 (2H, singlet);
5.75 (2H, singlet);
7.16-7.74 (8H, multiplet).

### EXAMPLE 11

### 2-Butyl-1-[(2'-carboxybiphenyl-4-yl)methyl]-4-(1-hydroxyethyl) imidazole-5-carboxylic acid (Compound No. 1-25)

### 11(a) 4-Acetyl-1-[(2'-t-butoxycarbonylbiphenyl-4-yl)-methyl]-2-butyl-5-cyanoimidazole

0.87 g of potassium carbonate and 2.4 g of t-butyl 4'-bromomethylbiphenyl-2-carboxylate were added to a solution of 1.2 g of 4-acetyl-2-butyl-5-cyanoimidazole (prepared as described in Preparation 5) in 12 ml of N,N-dimethylacetamide, and the resulting mixture was stirred at room temperature for 3 hours. At the end of this time, the reaction mixture was diluted with 100 ml of ethyl acetate and washed with a saturated aqueous solution of sodium chloride. The aqueous layer was once again extracted with 50 ml of ethyl acetate, and the combined extracts were washed with a saturated aqueous solution of sodium chloride. The solvent was removed by distillation under reduced pressure, and the resulting residue was purified by column chromatography through silica gel, using a 3 : 1 by volume mixture of hexane and ethyl acetate as the eluent, to give 1.31 g of the title compound.

Nuclear Magnetic Resonance Spectrum (CDCl₃) δ ppm:
0.93 (3H, triplet, J = 7 Hz);
1.1 - 2.1 (4H, multiplet);
1.23 (9H, singlet);
2.58 (3H, singlet);
2.75 (2H, triplet, J = 7 Hz);
5.32 (2H, singlet);
7.0 - 8.0 (8H, multiplet).

### 11 (b) 4-Acetyl-2-butyl-1-[(2'-carboxybiphenyl-4-yl)-methyl]-5-cyanoimidazole

A solution of 1.3 g of 4-acetyl-1-[(2'-t-butoxy-carbonylbiphenyl-4-yl)methyl]-2-butyl-5-cyanoimidazole [prepared as described in step (a) above] in 30 ml of a 4 N solution of hydrogen chloride in dioxane was allowed to stand overnight at room temperature, after which it was concentrated by evaporation under reduced pressure. The concentrate was purified by column chromatography through silica gel, using a 10 : 1 by volume mixture of methylene chloride and methanol as the eluent, to give a colourless amorphous solid. The solid was triturated in hexane, collected by filtration and dried, to give 1.1 g of the title compound, melting at above 55°C (with softening).

Nuclear Magnetic Resonance Spectrum (CDCl₃) δ ppm:
0.84 (3H, triplet, J = 7 Hz);
1.0 - 2.0 (4H, multiplet);
2.54 (3H, singlet);
2.66 (2H, triplet, J = 7 Hz);
5.17 (2H, singlet);
6.8 - 7.0 (8H, multiplet).

### 11 (c) 2-Butyl-1-[(2'-carboxybiphenyl-4-yl)methyl]-5-cyano-4-(1-hydroxyethyl)imidazole

68 mg of sodium borohydride were added to a solution of 718 mg of 4-acetyl-2-butyl-1-[(2'-carboxybiphenyl4-yl)methyl]-5-cyanoimidazole [prepared as described in step (b) above] in a mixture of 20 ml of isopropanol and 10 ml of ethanol, and the resulting mixture was stirred at room temperature for 3 hours. At the end of this time, the pH of the reaction mixture was adjusted to a value of 3 by the addition of 1 N aqueous hydrochloric acid, after which the solvent was distilled off under reduced pressure. The resulting residue was mixed with methylene chloride and water, and the methylene chloride layer was separated. The aqueous layer was extracted three times with methylene chloride, and the combined extracts were dried and concentrated by evaporation under reduced pressure. The resulting residue was dissolved in 10 ml of ethyl acetate and allowed to stand at room temperature. The solid which then deposited was collected by filtration and dried, to give 398 mg of the title compound as a colourless powder, melting at 200 - 201°C.

Nuclear Magnetic Resonance Spectrum (hexadeuterated dimethyl sulphoxide) δ ppm:
0.88 (3H, triplet, J = 7 Hz);
1.0 - 2.0 (4H, multiplet);
1.54 (3H, doublet, J = 7 Hz);
2.68 (2H, triplet, J = 7 Hz);
4.91 (1H, quartet, J = 7 Hz) ;
5.21 (2H, singlet);
7.0 - 8.0 (8H, multiplet).

### 11(d) 2-Butyl-1-[(2'-carboxybiphenyl-4-yl)methyl]-4-(1-hydroxyethyl)imidazole-5-carboxylic acid

A mixture of 300 mg of 2-butyl-1-[(2'-carboxybiphenyl-4-yl) methyl]-5-cyano-4-(1-hydroxyethyl)imidazole [prepared as described in step (c) above] and 3 ml of a 1 N aqueous solution of sodium hydroxide was stirred in an oil bath kept at 80 °C for 3 hours. At the end of this time, the reaction mixture was cooled and then weakly acidified with hydrochloric acid; it was then extracted four times, each time with 30 ml of methylene chloride. The combined extracts were dried and concentrated to dryness by evaporation under reduced pressure, to give an amorphous solid. This solid was purified by column chromatography through silica gel, using mixtures of methylene chloride and methanol ranging from 10 : 1 to 3 : 1 by volume as the eluent. A solid obtained from the eluate was triturated in diethyl ether. The resulting powder was collected by filtration and dried, to give 72.3 mg of the title compound as a colourless powder, melting at 168 - 170°C (with softening above 140°C).

Nuclear Magnetic Resonance Spectrum (hexadeuterated dimethyl sulphoxide) δ ppm:
0.84 (3H, triplet, J = 7 Hz);
1.0 - 2.0 (4H, multiplet);
1.52 (3H, doublet, J = 7 Hz);
2.3 - 2.8 (2H, overlapped with a peak of dimethyl sulphoxide);
4.93 (1H, quartet, J = 7 Hz);
5.60 (2H, broad singlet);
6.8 - 7.8 (8H, multiplet).

### EXAMPLE 35

### Ethyl 4-hydroxymethyl-2-propyl-1-{4-[2-(tetrazol-5-yl)phenyl]phenyl}methylimidazole-5-carboxylate (Compound No. 4-3)

### 35(a) Diethyl 2-propyl-1-{4-[2-(trityltetrazol-5-yl)-phenyl]phenyl}methylimidazole-4,5-dicarboxylate

0.441 g of potassium t-butoxide was added to a solution of 1.00 g of diethyl 2-propylimidazole-4,5-dicarboxylate (prepared as described in Preparation 12) in 15 ml of N,N-dimethylacetamide, and the resulting mixture was stirred at room temperature for 30 minutes. A solution of 2.19 g of 4-[2-(trityltetrazol-5-yl)- phenyl]benzyl bromide in 15 ml of N,N-dimethylacetamide was then added dropwise to the reaction mixture at room temperature, and the reaction mixture was stirred at room temperature for 3 hours. At the end of this time, it was diluted with water and then extracting with ethyl acetate. The extract was dried over anhydrous magnesium sulphate and then freed from the solvent by distillation. The residue was purified by column chromatography through silica gel, using a 1 : 1 by volume mixture of hexane and ethyl acetate as the eluent, to give 2.24 g of the title compound as an amorphous solid.

Nuclear Magnetic Resonance Spectrum (CDCl₃) δ ppm:
0.88 (3H, triplet, J = 7.5 Hz);
1.20 (3H, triplet, J = 7.5 Hz);
1.39 (3H, triplet, J = 7.5 Hz);
1.59 (6H, singlet);
1.61 - 1.72 (2H, multiplet);
2.55 (2H, triplet, J = 7.5 Hz);
4.20 (2H, quartet, J = 7.5 Hz);
4.39 (2H, quartet, J = 7.5 Hz);
5.30 (2H, singlet);
6.78 (2H, doublet, J = 8 Hz);
6.92 - 7.52 (20H, multiplet);
7.90 (1H, doublet, J = 7.5 Hz).

### 35(b) Ethyl 4-hydroxymethyl-2-propyl-1-{4-[2-(trityl-tetrazol-5-yl)phenyl]phenyl}methylimidazole-5-carboxylate

10 ml of a 1.5 M solution of diisobutylaluminium hydride in toluene were added dropwise at -20°C under an atmosphere of nitrogen to a solution of 4.27 g of diethyl 2-propyl-1-{4-[2-(trityltetrazol-5-yl)phenyl]-phenyl}methylimidazole-4,5-dicarboxylate [prepared as described in step (a) above] in 50 ml of tetrahydrofuran. The resulting mixture was allowed to stand at 0°C for 16 hours and then mixed with ethyl acetate and with a saturated aqueous solution of ammonium chloride; it was then stirred at room temperature for 1 hour. The resulting precipitate was filtered off, and the ethyl acetate layer was separated and dried over anhydrous magnesium sulphate; the solvent was then removed by distillation under reduced pressure. The crystalline residue was washed with diisopropyl ether, to give 4.03 g of the title compound, melting at 135 - 138°C.

Nuclear Magnetic Resonance Spectrum (CDCl₃) δ ppm:
0.94 (6H, triplet, J = 7.5 Hz);
1.29 (3H, triplet, J = 7 Hz);
1.67 - 1.77 (2H, multiplet);
2.56 (2H, triplet, J = 7.5 Hz);
3.43 (1H, broad triplet, J = 4 Hz);
4.25 (2H, quartet, J = 7 Hz);
4.91 (2H, doublet, J = 4 Hz);
5.49 (2H, singlet);
6.82 (2H, doublet, J = 7.5 Hz);
6.98 - 7.57 (20H, multiplet);
7.94 (1H, doublet, J = 7 Hz).

### 35(c) Ethyl 4-hydroxymethyl-2-propyl-1-{4-[2-(tetrazol5-yl)phenyl]phenyl}methylimidazole-5-carboxylate

A solution of 0.28 g of ethyl 4-hydroxymethyl-2-propyl-{4-[2-(trityltetrazol-5-yl)phenyl]phenyl}-methylimidazole-5-carboxylate [prepared as described in step (b) above] in 4 ml of 75% v/v aqueous acetic acid was stirred at 60°C for 2 hours. The reaction mixture was then concentrated by evaporation under reduced pressure, and the residue was dissolved in toluene. The resulting solution was again concentrated by evaporation under reduced pressure, to remove as much water and acetic acid as possible. The residue was then purified by column chromatography through silica gel, using 9 : 1 and 4 : 1 by volume mixtures of methylene chloride and methanol as the eluent, to give 0.20 g of the title compound as an amorphous solid.

Nuclear Magnetic Resonance Spectrum (CDCl₃) δ ppm:
0.80 (3H, triplet, J = 7.5 Hz);
1.20 (3H, triplet, J = 7.5 Hz);
1.45 - 1.65 (2H, multiplet);
2.44 (2H, triplet, J = 7.5 Hz);
4.20 (2H, quartet, J = 7.5 Hz);
4.58 (2H, singlet);
5.43 (2H, singlet);
6.78 (2H, doublet, J = 7.5 Hz);
6.98 (2H, doublet, J = 7.5 Hz);
7.38 - 7.60 (3H, multiplet);
7.79 (1H, doublet, J = 7.5 Hz).

### EXAMPLE 36

### 4-Hydroxymethyl-2-propyl-1-{4-[2-(tetrazol-5-yl)phenyl]phenyl}methylimidazole-5-carboxylic acid (Compound No. 4-1)

A mixture of 0.20 g of ethyl 4-hydroxymethyl-2-propyl-1-(4-[2-(tetrazol-5-yl) phenyl]phenyl}methylimidazole-5-carboxylate [prepared as described in Example 35(c)] and 0.10 g of lithium hydroxide monohydrate in 3 ml of water was stirred at room temperature for 3 hours, after which it was allowed to stand for 16 hours at the same temperature. The reaction mixture was then mixed with 2.38 ml of 1 N aqueous hydrochloric acid and the resulting precipitate was collected by filtration, to give 150 mg of the title compound, melting at 233°C (with decomposition).

Nuclear Magnetic Resonance Spectrum (hexadeuterated dimethyl sulphoxide) δ ppm:
0.88 (3H, triplet, J = 7.5 Hz);
1.59 (2H, sextet, J = 7.5 Hz);
2.58 (2H, triplet, J = 7.5 Hz);
4.64 (2H, singlet);
5.62 (2H, singlet);
6.98 (2H, doublet, J = 8 Hz);
7.08 (2H, doublet, J = 8 Hz);
7.39 - 7.69 (4H, multiplet).

### EXAMPLE 37

### Pivaloyloxymethyl 4-hydroxymethyl-2-propyl-1-{4-[2-(tetrazol-5-yl)phenyl]phenyl}methylimidazole-5-carboxylate (Compound No. 4-4)

### 37 (a) 4-Hydroxymethyl-2-propyl-1-{4-[2-(trityltetrazol-5-yl)phenyl]phenyl}-methylimidazole-5-carboxylic acid

A solution of 0.66 g of lithium hydroxide monohydrate in 20 ml of water was added to a solution of 1.22 g of ethyl 4-hydroxymethyl-2-propyl-1-{4-[2-(trityltetrazol-5-yl)phenyl]phenyl}methylimidazole-5-carboxylate [prepared as described in Example 35(b)] in 5 ml of dioxane, and the resulting mixture was stirred at 80°C for 5 hours. At the end of this time, the reaction mixture was freed from dioxane by distillation under reduced pressure, and the aqueous residue was mixed with ice and with ethyl acetate; 15.7 ml of 1 N aqueous hydrochloric acid were then added. The title compound precipitated, and was collected by filtration and washed with water. The ethyl acetate layer was then separated from the filtrate and dried over anhydrous magnesium sulphate, and the solvent was removed by distillation under reduced pressure. The resulting residue was washed with diethyl ether, to give more of the title compound as a powder. The two portions of the title compound were combined and together weighed 0.98 g, and this was immediately used in the subsequent esterification reaction without further purification or characterisation.

### 37 (b) Pivaloyloxymethyl 4-hydroxymethyl-2-propyl-1-{4-[2-(trityltetrazol-5-yl)phenyl]phenyl}methyl-imidazole-5-carboxylate

0.30 g of potassium carbonate and 0.24 g of pivaloyloxymethyl chloride were added to a solution of 0.98 g of 4-hydroxymethyl-2-propyl-1-{4-[2-(trityl-tetrazol-5-yl)phenyl]phenyl}methylimidazole-5-carboxylic acid [prepared as described in step (a) above] in 10 ml of N,N-dimethylacetamide, and the resulting mixture was stirred at room temperature for 6 hours. At the end of this time, the reaction mixture was mixed with ethyl acetate and water. The ethyl acetate layer was separated and dried over anhydrous magnesium sulphate, and then the solvent was removed by distillation under reduced pressure. The resulting residue was purified by column chromatography through silica gel, using a 2 : 1 by volume mixture of ethyl acetate and hexane as the eluent, to give 0.91 g of the title compound as a gum.

Nuclear Magnetic Resonance Spectrum (CDCl₃) δ ppm:
0.89 (3H, triplet, J = 7.5 Hz);
1.18 (9H, singlet);
1.70 (1H, sextet, J = 7.5 Hz);
2.52 (2H, triplet, J = 8 Hz);
3.35 (1H, broad singlet);
4.83 (2H, singlet);
5.42 (2H, singlet);
5.80 (2H, singlet);
6.76 (2H, doublet, J = 8 Hz);
6.92 - 7.51 (20H, multiplet);
7.90 (1H, doublet, J = 7.5 Hz).

### 37 (c) Pivaloyloxymethyl 4-hydroxymethyl-2-propyl-1-{4-[2-(tetrazol-5-yl)phenyl]phenyl}methylimidazole-5-carboxylate

Following a procedure similar to that described in Example 35 (c), 0.91 g of pivaloyloxymethyl 4-hydroxymethyl-2-propyl-1-{4-[2-(trityltetrazol-5-yl)phenyl]-phenyl}methylimidazole [prepared as described in step (b) above] was detritylated by treatment with 75% v/v aqueous acetic acid, to give 0.42 g of the title compound as a powder, melting at above 60°C (with softening).

Nuclear Magnetic Resonance Spectrum (CDCl₃) δ ppm:
0.94 (3H, triplet, J = 7.5 Hz);
1.14 (9H, singlet);
1.72 (2H, sextet, J = 7.5 Hz);
2.61 (2H, triplet, J = 7.5 Hz);
2.90 (2H, broad singlet);
4.77 (2H, singlet);
5.49 (2H, singlet);
5.84 (2H, singlet);
6.94 (2H, doublet, J = 8 Hz);
7.15 (2H, doublet, J = 8 Hz);
7.26 - 7.61 (3H, multiplet);
8.07 (1H, doublet, J = 7.5 Hz).

### EXAMPLE 38

### Methyl 2-butyl-4-hydroxymethyl-1-{4- [2- (tetrazol-5-yl)phenyl]phenyl}methylimidazole-5-carboxylate (Compound No. 4-47)

### 38(a) Dimethyl 2-butyl-1-{4-[2-(trityltetrazol-5-yl)-phenyl]phenyl}methylimidazole-4,5-dicarboxylate

Following a procedure similar to that described in Example 35(a), but using 0.50 g of dimethyl 2-butylimidazole-4,5-dicarboxylate (prepared as described in Preparation 4) and 1.17 g of 4-[2-(trityltetrazol-5-yl)phenyl]benzyl bromide, 0.51 g of the title compound was obtained as an amorphous solid.

Nuclear Magnetic Resonance Spectrum (CDCl₃) δ ppm:
0.85 (3H, triplet, J = 7.5 Hz);
1.20 - 1.80 (4H, multiplet);
2.59 (2H, triplet, J = 8.0 Hz);
3.73 (3H, singlet);
3.92 (3H, singlet) ;
5.30 (2H, singlet);
6.6 - 7.6 (22H, multiplet);
7.8 - 8.0 (1H, multiplet).

### 38(b) Methyl 2-butyl-4-hydroxymethyl-1-{4-[2-(trityltetrazol-5-yl)phenyl]phenyl}methylimidazole-5-carboxylate

Following a procedure similar to that described in Example 35(b), 0.51 g of dimethyl 2-butyl-1-{4-[2-(trityltetrazol-5-yl)phenyl]phenyl}methylimidazole-4,5- dicarboxylate [prepared as described in step (a) above] was reduced using 0.99 ml of a 1.5 M solution of diisobutylaluminium hydride in toluene, to give 0.44 g of the title compound as an oil.

Nuclear Magnetic Resonance Spectrum (CDCl₃) δ ppm:
0.86 (3H, triplet, J = 7.5 Hz);
1.23 - 1.36 (2H, multiplet);
1.58 - 1.70 (2H, multiplet);
1.80 - 1.95 (1H, multiplet);
2.54 (2H, triplet, J = 8.0 Hz);
3.72 (3H, singlet);
4.85 (2H, doublet, J = 6.0 Hz);
5.43 (2H, singlet);
6.77 (2H, doublet, J = 8.5 Hz);
6.92 - 6.95 (4H, multiplet);
7.08 (2H, doublet, J = 8.5 Hz);
7.22 - 7.51 (14H, multiplet);
7.87 - 7.90 (1H, multiplet).

### 38 (c) Methyl 2-butyl-4-hydroxymethyl-1-{4-[2-tetrazol-5-yl)phenyl]phenyl}methylimidazole-5-carboxylate

A solution of 0.44 g of methyl 2-butyl-4-hydroxymethyl-1-{4-[2-(trityltetrazol-5-yl)phenyl]phenyl}-methylimidazole-5-carboxylate [prepared as described in step (b) above] in 10 ml of methanol and 0.70 ml of 1 N aqueous hydrochloric acid was allowed to stand overnight at room temperature. At the end of this time, the reaction mixture was concentrated to dryness by distillation under reduced pressure, and the residue was triturated with diethyl ether to give 0.30 g of the hydrochloride of the title compound as a solid.

Nuclear Magnetic Resonance Spectrum (hexadeuterated dimethyl sulphoxide) δ ppm:
0.81 (3H, triplet, J = 7.5 Hz);
1.19 - 1.32 (2H, multiplet);
1.38 - 1.51 (2H, multiplet);
2.95 (2H, triplet, J = 7.5 Hz);
4.80 (2H, singlet);
5.71 (2H, singlet);
7.20 - 7.75 (8H, multiplet).

### EXAMPLE 39

### 2-Butyl-4-hydroxymethyl-1-{4-[2-(tetrazol-5-yl)-phenyl]phenyl}methylimidazole-5-carboxylic acid (Compound No. 4-46)

Following a procedure similar to that described in Example 36, but using 0.30 g of methyl 2-butyl-4-hydroxymethyl-1-{4-[2-(tetrazol-5-yl)phenyl]phenyl}-methylimidazole-5-carboxylate [prepared as described in Example 38(c)] and 2.50 ml of a 1 N aqueous solution of sodium hydroxide, 95 mg of the title compound were obtained as crystals, melting at 215 - 217°C.

Nuclear Magnetic Resonance Spectrum (hexadeuterated dimethyl sulphoxide) δ ppm:
0.82 (3H, triplet, J = 7.5 Hz);
1.27 (2H, multiplet);
1.52 (2H, multiplet);
2.56 (2H, triplet, J = 7.5 Hz);
4.60 (2H, singlet);
5.58 (2H, singlet);
6.94 (2H, doublet, J = 8.5 Hz);
7.06 (2H, doublet, J = 8.5 Hz);
7.50 - 7.70 (4H, multiplet).

### EXAMPLE 40

### Ethyl 4-(1-hydroxyethyl)-2-propyl-1-{4-[2-(tetrazol-5-yl)phenyl]phenyl}methylimidazole-5-carboxylate (Compound No. 4-30)

### 40(a) Ethyl 4-formyl-1-propyl-1-{4-[2-(trityltetrazol-5-yl)phenyl]phenyl}methylimidazole-5-carboxylate

6 g of activated manganese dioxide were added to a solution of 2 g of ethyl 4-hydroxymethyl-2-propyl-1-{4-[2-(trityltetrazol-5-yl)phenyl]phenyl}methyl-imidazole-5-carboxylate [prepared as described in Example 35(b)] in 40 ml of methylene chloride, and the resulting mixture was stirred at room temperature for 2.5 hours. At the end of this time, the manganese dioxide was filtered off and the filtrate was concentrated by evaporation under reduced pressure. The resulting residue was purified by column chromatography through silica gel, using a 1 : 1 by volume mixture of ethyl acetate and hexane as the eluent, to give 1.45 g of the title compound as crystals, melting at 177 - 179°C (with decomposition).

Nuclear Magnetic Resonance Spectrum (CDCl₃) δ ppm:
0.88 (3H, triplet, J = 7.5 Hz);
1.29 (3H, triplet, J = 7 Hz);
1.74 (2H, sextet, J = 7.5 Hz);
2.57 (2H, triplet, J = 7.5 Hz);
4.29 (2H, quartet, J = 7 Hz);
5.49 (2H, singlet);
6.76 (2H, doublet, J = 8.5 Hz);
6.92 - 7.88 (20H, multiplet);
7.90 (1H, doublet, J = 7.5 Hz);
10.42 (1H, singlet).

### 40(b) Ethyl 4-(1-hydroxyethyl)-2-propyl-1-{4-[2-(trityltetrazol-5-yl)phenyl]phenyl}methyl-imidazole-5-carboxylate

4.0 ml of a 1 M solution of methylmagnesium bromide in tetrahydrofuran were added dropwise at -10° C to a solution of 1.2 g of ethyl 4-formyl-2-propyl-1-{4-[2- (trityltetrazol-5-yl)phenyl] phenyl}methylimidazole-5- carboxylate [prepared as described in step (a) above] in 5 ml of tetrahydrofuran, and the resulting mixture was stirred at a temperature between -10°C and 0°C for 3 hours. At the end of this time, the reaction mixture was mixed with ethyl acetate and with an aqueous solution of ammonium chloride, and the mixture was stirred at room temperature for 20 minutes. The ethyl acetate layer was then separated and dried over anhydrous magnesium sulphate. The solvent was removed by distillation under reduced pressure, and the residue was purified by column chromatography through silica gel, using 1 : 4 and 1 : 2 by volume mixtures of ethyl acetate and methylene chloride as the eluent, to give 1.23 g of the title compound as a viscous oil.

Nuclear Magnetic Resonance Spectrum (CDCl₃) δ ppm:
0.87 (3H, triplet, J = 7.5 Hz);
1.22 (3H, triplet, J = 7 Hz);
1.54 (3H, doublet, J = 7 Hz);
1.68 (2H, sextet, J = 7.5 Hz);
2.50 (2H, triplet, J = 7.5 Hz);
3.82 (1H, doublet, J = 8 Hz);
4.18 (2H, quartet, J = 7 Hz);
5.23 (1H, quintet, J = 7 Hz);
5.42 (2H, singlet);
6.76 (2H, doublet, J = 8 Hz);
6.93 - 7.52 (20H, multiplet);
7.88 (1H, doublet, J = 7.5 Hz).

### 40(c) Ethyl 4-(1-hydroxyethyl)-2-propyl-1-{4-[2(tetrazol-5-yl)phenyl]phenyl}methylimidazole5-carboxylate

Following a procedure similar to that described in Example 35(c), 1.23 g of ethyl 4-(1-hydroxyethyl)-2-propyl-1-{4-[2-(trityltetrazol-5-yl)phenyl]phenyl}-methylimidazole-5-carboxylate [prepared as described in step (b) above] were treated with 75% v/v aqueous acetic acid, to give 0.82 g of the title compound as an amorphous solid.

Nuclear Magnetic Resonance Spectrum (CDCl₃) δ ppm:
0.85 (3H, triplet, J = 7.5 Hz);
1.24 (3H, triplet, J = 7 Hz);
1.42 (3H, doublet, J = 6 Hz);
1.59 (2H, sextet, J = 7.5 Hz);
2.50 (2H, triplet, J = 7 Hz);
4.22 (2H, quartet, J = 7 Hz);
5.13 - 5.20 (1H, multiplet);
5.44 (2H, AB-quartet, Δδ = 0.12 ppm, J = 16.5 Hz);
6.78 (2H, doublet, J = 8 Hz);
6.99 (2H, doublet, J = 8 Hz);
7.38 - 7.59 (3H, multiplet);
7.76 (1H, doublet, J = 7.5 Hz).

### EXAMPLE 41

### 4-(1-Hydroxyethyl)-2-propyl-1-{4-[2-(tetrazol-5-yl)phenyl]phenyl}methylimidazole-5-carboxylic acid (Compound No. 4-29)

Following a procedure similar to that described in Example 36, 0.82 g of ethyl 4-(1-hydroxyethyl)-2-propyll-{4-[2-(tetrazol-5-yl)phenyl] phenyl}methylimidazole5-carboxylate [prepared as described in Example 40(c)] was hydrolyzed using 0.43 g of lithium hydroxide monohydrate, to give 0.50 g of the title compound as a powder, melting at 198 - 201°C.

Nuclear Magnetic Resonance Spectrum (hexadeuterated dimethyl sulphoxide) δ ppm:
0.86 (3H, triplet, J = 7.5 Hz);
1.38 (3H, doublet, J = 6.5 Hz);
1.55 (2H, sextet, J = 7.5 Hz);
2.58 (2H, triplet, J = 8 Hz);
5.21 (1H, quartet, J = 6.5 Hz);
5.61 (2H, singlet);
6.95 - 7.08 (4H, multiplet);
7.51 - 7.70 (4H, multiplet).

### EXAMPLE 42

### Ethyl 4-(1-hydroxyethyl)-2-propyl-1-{4-[2-tetrazol-5-yl)phenyl]phenyl}methylimidazole-5-carboxylate (Compound No. 4-30)

### 42(a) Ethyl 4-(1-hydroxyethyl)-2-propyl-1-{4-[2-(trityltetrazol-5-yl)phenyl]phenyl}methylimidazole-5-carboxylate

Following a procedure similar to that described in Example 35(a), but using 113 mg of ethyl 4-(1-hydroxyethyl)-2-propylimidazole-5-carboxylate [prepared as described in Preparation 23(iii)], 280 mg of 4-[2-(trityltetrazol-5-yl)phenyl]benzyl bromide and 60 mg of potassium t-butoxide, 255 mg of the title compound were obtained as a viscous oil. The Nuclear Magnetic Resonance Spectrum of this compound was identical with that of the compound obtained as described in Example 40(b).

### 42(b) Ethyl 4-(1-hydroxyethyl)-2-propyl-1-{4-[2-(tetrazol-5-yl)phenyl]phenyl}methylimidazole5-carboxylate

Following a procedure similar to that described in Example 35(c), 255 mg of ethyl 4-(1-hydroxyethyl)-2-propyl-1-{4-[2-(trityltetrazol-5-yl)phenyl]phenyl}-methylimidazole-5-carboxylate [prepared as described in step (a) above] was detritylated by treatment with 75% v/v aqueous acetic acid, to give 170 mg of the title compound as an amorphous solid. The Nuclear Magnetic Resonance Spectrum of this compound was identical with that of the compound obtained as described in Example 40(c).

### EXAMPLE 43

### Ethyl 2-butyl-4-(1-hydroxyethyl)-1-{4-[2-(tetrazol-5-yl)phenyl]phenyl}methylimidazole-5-carboxylate (Compound No. 4-75)

### 43(a) Ethyl 2-butyl-4-(1-hydroxyethyl)-1-{4-[2-(trityltetrazol-5-yl)phenyl]phenyl-methylimidazole-5-carboxylate

Following a procedure similar to that described in Example 35(a), but using 400 mg of ethyl 2-butyl-4-(1-hydroxyethyl)imidazole-5-carboxylate [prepared as described in Preparation 24(iii)], 1.00 g of 4-[2-(trityltetrazol-5-yl)phenyl]benzyl bromide and 197 mg of potassium t-butoxide, 0.94 g of the title compound was obtained as a viscous oil.

Nuclear Magnetic Resonance Spectrum (CDCl₃) δ ppm:
0.87 (3H, triplet, J = 7.5 Hz);
1.24 (3H, triplet, J = 7 Hz) ;
1.25 - 1.38 (2H, multiplet);
1.55 (3H, doublet, J = 6.5 Hz) ;
1.60 - 1.72 (2H, multiplet);
2.54 (2H, triplet, J = 8 Hz);
3.84 (1H, doublet, J = 6.5 Hz);
4.20 (4H, quartet, J = 7 Hz);
5.25 (1H, quintet, J = 6.5 Hz);
5.44 (2H, singlet);
6.78 (2H, doublet, J = 8 Hz);
6.94 - 7.54 (20H, multiplet);
7.90 (1H, doublet, J = 7.5 Hz).

### 43(b) Ethyl 2-butyl-4-(1-hydroxyethyl)-1-{4-[2-(tetrazol-5-yl)phenyl]phenyl}methylimidazole5-carboxylate

Following a procedure similar to that described in Example 40(c), 0.84 g of ethyl 2-butyl-4-(1-hydroxyethyl)-1-{4-[2-(trityltetrazol-5-yl)phenyl]phenyl}-methylimidazole-5-carboxylate [prepared as described in step (a) above] was treated with 75% v/v aqueous acetic acid, to give 0.54 g of the title compound as an amorphous solid.

Nuclear Magnetic Resonance Spectrum (CDCl₃) δ ppm:
0.78 (3H, triplet, J = 7.5 Hz);
1.15 - 1.30 (2H, multiplet);
1.19 (3H, triplet, J = 7 Hz);
1.35 (3H, doublet, J = 6.5 Hz);
1.44 - 1.60 (2H, multiplet);
2.49 (2H, triplet, J = 8 Hz);
4.17 (2H, quartet, J = 7 Hz);
5.09 (1H, quartet, J = 6.5 Hz);
5.35 & 5.45 (each 1H, AB-quartet, J = 16.5 Hz);
6.89 (2H, doublet, J = 8 Hz);
6.96 (2H, doublet, J = 8 Hz);
7.30 - 7.50 (3H, multiplet);
7.65 (1H, doublet, J = 7.5 Hz).

### EXAMPLE 44

### 2-Butyl-4-(1-hydroxyethyl)-1-{4-[2-(tetrazol-5-yl)phenyl]phenyl}methylimidazole-5-carboxylic acid (Compound No. 4-74)

Following a procedure similar to that described in Example 36, 0.54 g of ethyl 2-butyl-4-(1-hydroxyethyl)- 1-{4-[2- (tetrazol-5-yl)phenyl]phenyl}methylimidazole5-carboxylate [prepared as described in Example 43(b)] was hydrolyzed, using 245 mg of lithium hydroxide monohydrate, to give 0.43 g of the title compound as a powder, melting at 214 - 217°C.

Nuclear Magnetic Resonance Spectrum (hexadeuterated dimethyl sulphoxide) δ ppm:
0.82 (3H, triplet, J = 7.5 Hz);
1.27 (2H, sextet, J = 7.5 Hz);
1.37 (3H, doublet, J = 6.5 Hz);
1.50 (2H, quintet, J = 7.5 Hz);
2.58 (2H, triplet, J = 8 Hz);
5.20 (1H, quartet, J = 6.5 Hz);
5.61 (2H, singlet);
6.96 (2H, doublet, J = 8 Hz);
7.06 (2H, doublet, J = 8 Hz);
7.50 - 7.66 (4H, multiplet).

### EXAMPLE 45

### 2-Butyl-1-[(2'-carboxybiphenyl-4-yl)methyl]-4-(1-hydroxyethyl) imidazol-5-carboxamide (Compound No. 5-64)

### 45(a) 4-Acetyl-1-[(2'-t-butoxycarbonylbiphenyl-4-yl)-methyl]-2-butylimidazole-5-carbonitrile

0.192 g of sodium hydride (as a 55% w/w dispersion in mineral oil) was added to a solution of 0.843 g of 4-acetyl-2-butylimidazole-5-carbonitrile [prepared as described in Preparation 24(i)] in 17 ml of N,N-dimethylacetamide, and the resulting mixture was stirred at room temperature for 20 minutes. 1.68 g of t-butyl 4'-(bromomethyl)biphenyl-2-carboxylate was then added, and the resulting mixture was stirred at 55°C for 2.5 hours. At the end of this time, an aqueous solution of sodium chloride was added to the mixture, which was then extracted with ethyl acetate. The extract was washed with water and dried over anhydrous magnesium sulphate, and then the solvent was removed by distillation under reduced pressure. The resulting oily residue was purified by column chromatography through silica gel, using 4 : 1 and 2 : 1 by volume mixtures of hexane and ethyl acetate as the eluent, to afford 1.14 g of the title compound as a viscous oil.

Nuclear Magnetic Resonance Spectrum (CDCl₃) δ ppm:
0.93 (3H, triplet, J = 7 Hz);
1.23 (9H, singlet);
1.3 - 2.1 (4H, multiplet);
2.58 (3H, singlet);
2.75 (2H, triplet, J = 7 Hz);
5.32 (2H, singlet);
7.0 - 8.0 (8H, multiplet).

### 45(b) 1-[(2'-t-Butoxycarbonylbiphenyl-4-yl)methyl] -2-butyl-4-(1-hydroxyethyl)imidazole-5-carbonitrile

0.098 g of sodium borohydride was added to a solution of 1.18 g of 4-acetyl-1-[(2'-t-butoxycarbonylbiphenyl-4-yl)methyl]-2-butylimidazole-5-carbonitrile [prepared as described in step (a) above] in 30 ml of ethanol, and the resulting mixture was stirred at room temperature for 1 hour. The excess sodium borohydride was decomposed by adding acetone, and then the reaction mixture was concentrated by evaporation under reduced pressure. The residue was diluted with an aqueous solution of sodium chloride and extracted with ethyl acetate. The extract was dried and concentrated by evaporation under reduced pressure. The oily residue was purified by column chromatography through silica gel, using a 3 : 2 by volume mixture of ethyl acetate and hexane as the eluent, to afford 1.18 g of the title compound as a viscous oil.

Nuclear Magnetic Resonance Spectrum (CDCl₃) δ ppm:
0.92 (3H, triplet, J = 7.5 Hz);
1.25 (9H, singlet);
1.3 - 1.5 (2H, multiplet);
1.60 (3H, doublet, J = 6.5 Hz);
1.6 - 1.8 (2H, multiplet);
2.6 - 2.8 (2H, multiplet);
5.00 (1H, quartet, J = 6.5 Hz);
5.22 (2H, singlet);
7.1 - 7.9 (8H, multiplet).

### 45(c) 1-[(2'-t-Butoxycarbonylbiphenyl-4-yl)methyl]-2-butyl-4-(1-hydroxyethyl)imidazole-5-carboxamide

12 ml of a 1 N aqueous solution of sodium hydroxide were added to a solution of 0.52 g of 1-[(2'-t-butoxycarbonylbiphenyl-4-yl)methyl]-2-butyl-4-(1-hydroxyethyl)- imidazole-5-carbonitrile [prepared as described in step (b) above] in 3 ml of ethanol, and the resulting mixture was heated under reflux for 3 hours. At the end of this time, the reaction mixture was neutralized by the addition of dilute aqueous hydrochloric acid and extracted with ethyl acetate. The extract was washed with water and dried over anhydrous magnesium sulphate. The solvent was then removed by distillation under reduced pressure. The resulting residue was purified by column chromatography through silica gel, using a 4 : 1 by volume mixture of ethyl acetate and hexane, followed by ethyl acetate alone, as the eluent, to afford 0.14 g of the title compound as an amorphous solid.

Nuclear Magnetic Resonance Spectrum (CDCl₃) δ ppm:
0.90 (3H, triplet, J = 7.5 Hz);
1.23 (9H, singlet) ;
1.2 - 1.5 (2H, multiplet);
1.6 - 1.8 (2H, multiplet);
1.66 (3H, doublet, J = 6.5 Hz);
2.63 (2H, triplet, J = 8 Hz);
5.11 (1H, quartet, J = 6.5 Hz);
5.59 & 5.74 (each 1H, AB-quartet, J = 16 Hz);
7.0 - 7.9 (8H, multiplet).

### 45(d) 2 -Butyl-1-[(2'-carboxybiphenyl-4-yl)methyl]-4-(1-hydroxyethyl)imidazole-5-carboxamide

A solution of 0.15 g of 1-[(2'-t-butoxycarbonyl-biphenyl-4-yl)methyl]-2-butyl-4-(1-hydroxyethyl)-imidazole-5-carboxamide [prepared as described in step (c) above] dissolved in 3 ml of a 4 N solution of hydrogen chloride in dioxane was allowed to stand overnight at room temperature. The solution was then concentrated by evaporation under reduced pressure. The resulting residue was triturated in hexane and the powder thus obtained was collected by filtration, to afford 0.105 g of the hydrochloride of the title compound as an amorphous solid, melting at 212 - 214°C (with decomposition).

Nuclear Magnetic Resonance Spectrum (CDCl₃) δ ppm:
0.94 (3H, triplet, J = 7.5 Hz);
1.3 - 1.6 (2H, multiplet);
1.59 (3H, doublet, J = 6.5 Hz);
1.6 - 2.0 (2H, multiplet);
3.0 - 3.4 (2H, multiplet);
5.16 (1H, quartet, J = 6.5 Hz);
5.41 & 5.58 (each 1H, AB-quartet, J = 15 Hz);
7.1 - 7.9 (8H, multiplet).

### EXAMPLE 46

### 2 -Butyl-1-[(2'-carboxybiphenyl-4-yl)methyl]-4-(1-hydroxypropyl)imidazole-5-carboxamide (Compound No. 5-65)

### 46(a) 1-[(2'-t-Butoxycarbonylbiphenyl-4-yl)methyl]-2-butyl-4-propionylimidazole-5-carbonitrile

Following a procedure similar to that described in Example 45(a) but using 0.923 g of 2-butyl-4-propionylimidazole-5-carbonitrile (prepared as described in Preparation 25), 1.56 g of t-butyl 4'-(bromomethyl)- biphenyl-2-carboxylate and 196 mg of sodium hydride (as a 55% w/w dispersion in mineral oil) in 20 ml of N,N-dimethylacetamide, 1.84 g of the title compound were obtained as a viscous oil.

Nuclear Magnetic Resonance Spectrum (CDCl₃) δ ppm:
0.91 (3H, triplet, J = 7 Hz);
1.0 - 2.1 (4H, multiplet);
1.25 (9H, singlet);
2.72 (2H, triplet, J = 7 Hz);
3.02 (2H, quartet, J = 7 Hz);
5.30 (2H, singlet);
7.0 - 8.0 (8H, multiplet).

### 46(b) 1-[(2'-t-Butoxycarbonylbiphenyl-4-yl)methyl]-2-butyl-4-(1-hydroxypropyl)imidazole-5-carbonitrile

Following a procedure similar to that described in Example 45(b), but using 451 mg of 1-[(2'-t-butoxycarbonylbiphenyl-4-yl)methyl]-2-butyl-4-propionyl-imidazole-5-carbonitrile [prepared as described in step (a) above] and 36 mg of sodium borohydride in 10 ml of ethanol, 369 mg of the title compound were obtained as a viscous oil.

Nuclear Magnetic Resonance Spectrum (CDCl₃) δ ppm:
0.91 (3H, triplet, J = 7 Hz);
0.99 (3H, triplet, J = 7 Hz);
1.0 - 2.3 (6H, multiplet);
1.25 (9H, singlet);
2.70 (2H, triplet, J = 7 Hz);
3.16 (1H, doublet, J = 6.5 Hz);
4.74 (1H, quartet, J = 7 Hz);
5.21 (2H, singlet);
7.0 - 8.0 (8H, multiplet).

### 46(c) 1-[(2'-t-Butoxycarbonylbiphenyl-4-yl)methyl]-2-butyl-4-(1-hydroxypropyl)imidazole-5-carboxamide

20 ml of a 1 N aqueous solution of sodium hydroxide were added to a solution of 368 mg of 1-[(2'-t-butoxycarbonylbiphenyl-4-yl)methyl] -2-butyl-4-(1-hydroxypropyl)imidazole-5-carbonitrile [prepared as described in step (b) above] dissolved in 20 ml of ethanol, and the resulting mixture was heated under reflux for 6 hours. At the end of this time, the reaction mixture was worked up in a similar manner to that described in Example 45(c), to afford 316 mg of the title compound as an amorphous solid.

Nuclear Magnetic Resonance Spectrum (CDCl₃) δ ppm:
0.89 (6H, triplet, J = 7 Hz);
1.0 - 2.3 (6H, multiplet);
1.24 (9H, singlet);
2.61 (2H, triplet, J = 7 Hz);
4.76 (1H, triplet, J = 7 Hz);
5.52 & 5.83 (each 1H, AB-quartet, J = 17 Hz);
6.9 - 7.9 (8H, multiplet).

### 46(d) 2-Butyl-1-[(2'-carboxybiphenyl-4-yl)methyl]-4-(1-hydroxypropyl)imidazole-5-carboxamide

Following a procedure similar to that described in Example 45 (d), but using 316 mg of 1-[(2'-t-butoxycarbonylbiphenyl-4-yl)methyl]-2-butyl-4-(1-hydroxy-propyl)imidazole-5-carboxamide [prepared as described in step (c) above] and 10 ml of a 4 N solution of hydrogen chloride in dioxane, 148 mg of the hydrochloride of the title compound were obtained as an amorphous powder, melting at above 120°C (with softening).

Nuclear Magnetic Resonance Spectrum (hexadeuterated dimethyl sulphoxide) δ ppm:
0.80 (3H, triplet, J = 7.5 Hz);
0.87 (3H, triplet, J = 7.5 Hz);
1.1 - 2.0 (6H, multiplet);
2.94 (2H, triplet, J = 7.5 Hz);
4.85 (1H, triplet, J = 7 Hz);
5.68 (2H, singlet);
7.0 - 7.8 (8H, multiplet).

### EXAMPLE 47

### 2 -Butyl-1-[(2'-carboxybiphenyl-4-yl)methyl]-4-(1-hydroxybutyl)imidazole-5-carboxamide (Compound No. 5-66)

### 47(a) 1-[(2'-t-Butoxycarbonylbiphenyl-4-yl)methyl]-2-butyl-4-butyrylimidazole-5-carbonitrile

Following a procedure similar to that described in Example 45(a), but using 0.877 g of 2-butyl-4-butyrylimidazole-5-carbonitrile (prepared as described in Preparation 26), 1.53 g of t-butyl 4'-(bromomethyl)-biphenyl-2-carboxylate and 0.175 g of sodium hydride (as a 55% w/w dispersion in mineral oil) in 18 ml of N,N-dimethylacetamide, 0.99 g of the title compound was obtained as a viscous oil.

Nuclear Magnetic Resonance Spectrum (CDCl₃) δ ppm:
0.93 (3H, triplet, J = 7 Hz);
1.01 (3H, triplet, J = 7 Hz);
1.28 (9H, singlet);
1.4 - 2.1 (6H, multiplet);
2.74 (2H, triplet, J = 7 Hz);
3.00 (2H, triplet, J = 7 Hz);
5.30 (2H, singlet);
7.0 - 8.0 (8H, multiplet).

### 47(b) 1-[(2'-t-Butoxycarbonylbiphenyl-4-yl)methyl]-2-butyl-4-(1-hydroxybutyl)imidazole-5-carbonitrile

Following a procedure similar to that described in Example 45(b), but using 0.99 g of 1-[(2'-t-butoxycarbonylbiphenyl-4-yl)methyl-2-butyl-4-butyrylimidazole5-carbonitrile [prepared as described in step (a) above] and 0.077 g of sodium borohydride in 20 ml of ethanol, 0.88 g of the title compound was obtained as a viscous oil.

Nuclear Magnetic Resonance Spectrum (CDCl₃) δ ppm:
0.7 - 1.2 (6H, multiplet);
1.2 - 2.1 (8H, multiplet);
1.23 (9H, singlet);
2.71 (2H, triplet, J = 7 Hz);
4.28 (1H, doublet, J = 6 Hz);
4.82 (1H, quartet, J = 6 Hz);
5.28 (2H, singlet);
7.0 - 8.0 (8H, multiplet).

### 47(c) 1-[(2'-t-Butoxycarbonylbiphenyl-4-yl)methyl]-2-butyl-4-(1-hydroxybutyl)imidazole-5-carboxamide

14 ml of a 1 N aqueous solution of sodium hydroxide were added to a solution of 0.86 g of 1-[(2'-t-butoxycarbonylbiphenyl-4-yl)methyl]-2-butyl-4-(1-hydroxybutyl)-imidazole-5-carbonitrile [prepared as described in step (b) above] in 14 ml of ethanol, and the resulting mixture was heated under reflux for 10 hours. At the end of this time, the reaction mixture was worked up in a similar manner to that described in Example 45(c) to afford 0.58 g of the title compound as an amorphous solid.

Nuclear Magnetic Resonance Spectrum (CDCl₃) δ ppm:
0.90 (3H, triplet, J = 7.5 Hz);
0.94 (3H, triplet, J = 7.5 Hz);
1.23 (9H, singlet);
1.3 - 2.1 (8H, multiplet);
2.63 (2H, triplet, J = 8 Hz);
4.91 (1H, triplet, J = 7 Hz);
5.56 & 5.77 (each 1H, AB-quartet, J = 16 Hz);
7.0 - 7.8 (8H, multiplet).

### 47(d) 2-Butyl-1-[(2'-carboxybiphenyl-4-yl)methyl]-4-(1-hydroxybutyl)imidazole-5-carboxamide

Following a procedure similar to that described in Example 45(d), but using 0.58 g of 1-[(2'-t-butoxycarbonylbiphenyl-4-yl)methyl]-2-butyl-4-(1-hydroxybutyl)- imidazole-5-carboxamide [prepared as described in step (c) above] and 13 ml of a 4 N solution of hydrogen chloride in dioxane, 0.55 g of the hydrochloride of the title compound was obtained as an amorphous powder, melting at above 110°C (with softening).

Nuclear Magnetic Resonance Spectrum (hexadeuterated dimethyl sulphoxide) δ ppm:
0.80 (3H, triplet, J = 7.5 Hz);
0.89 (3H, triplet, J = 7.5 Hz);
1.1 - 1.9 (8H, multiplet);
2.96 (2H, triplet, J = 7.5 Hz);
4.96 (1H, triplet, J = 7.5 Hz);
5.68 (2H, singlet);
7.2 - 7.8 (8H, multiplet).

### EXAMPLE 48

### 2-Butyl-1-[(2'-carboxybiphenyl-4-yl)methyl]-4-(1-hydroxy-2 -methylpropyl)imidazole-5-carboxamide (Compound No. 5-67)

### 48(a) 1-[(2'-t-butoxycarbonylbiphenyl-4-yl)methyl]-2-butyl-4-isobutyrylimidazole-5-carbonitrile

Following a procedure similar to that described in Example 45(a), but using 0.85 g of 2-butyl-4-isobutyrylimidazole-5-carbonitrile (prepared as described in Preparation 27), 1.34 g of t-butyl 4'-(bromomethyl)- biphenyl-2-carboxylate and 170 mg of sodium hydride (as a 55% w/w dispersion in mineral oil) in 15 ml of N,N-dimethylacetamide, 1.62 g of the title compound were obtained as a viscous oil.

Nuclear Magnetic Resonance Spectrum (CDCl₃) δ ppm:
0.93 (3H, triplet, J = 7 Hz);
1.0 - 2.1 (4H, multiplet);
1.21 (6H, doublet, J = 7 Hz);
1.22 (9H, singlet);
2.73 (2H, triplet, J = 7 Hz);
3.66 (1H, septet, J = 7 Hz);
5.30 (2H, singlet);
7.0 - 8.0 (8H, multiplet).

### 48 (b) 1-[(2'-t-Butoxycarbonylbiphenyl-4-yl)methyl]-2-butyl-4-(1-hydroxy-2-methylpropyl)imidazole-5-carbonitrile

Following a procedure similar to that described in Example 45(b), but using 500 mg of 1-[(2'-t-butoxycarbonylbiphenyl-4-yl)methyl]-2-butyl-4-isobutyrylimidazole-5-carbonitrile [prepared as described in step (a) above] and 25 mg of sodium borohydride in 10 ml of ethanol, 297 mg of the title compound were obtained as a viscous oil.

Nuclear Magnetic Resonance Spectrum (CDCl₃) δ ppm:
0.7 - 1.2 (9H, multiplet).
1.0 - 2.5 (5H, multiplet);
1.27 (9H, singlet);
2.70 (2H, doublet, J = 7 Hz);
3.01 (1H, doublet, J = 7 Hz);
4.54 (1H, triplet, J = 7 Hz);
5.23 (2H, singlet);
7.0 - 8.0 (8H, multiplet).

### 48(c) 1-[(2'-t-Butoxycarbonylbiphenyl-4-yl)methyl]-2-butyl-4-(1-hydoxy-2-methylpropyl)imidazole-5-carboxamide

20 ml of a 1 N aqueous solution of sodium hydroxide were added to a solution of 297 mg of 1-[(2'-t-butoxycarbonylbiphenyl-4-yl)methyl]-2-butyl-4-(1-hydroxy-2-methylpropyl)imidazole-5-carbonitrile [prepared as described in step (b) above] in 20 ml of ethanol, and the resulting mixture was heated under reflux for 8 hours. At the end of this time, the reaction mixture was worked up in a similar manner to that described in Example 45(c), to afford 151 mg of the title compound as an amorphous solid.

Nuclear Magnetic Resonance Spectrum (CDCl₃) δ ppm:
0.66 (3H, doublet, J = 7 Hz);
0.85 (3H, triplet, J = 7 Hz);
1.01 (3H, doublet, J = 7 Hz);
1.0 - 2.4 (5H, multiplet);
1.22 (9H, singlet);
2.59 (2H, triplet, J = 7 Hz);
4.40 (1H, doublet, J = 7 Hz);
5.53 & 5.83 (each 1H, AB-quartet, J = 17 Hz);
6.9 - 7.9 (8H, multiplet).

### 48(d) 2-Butyl-1-[(2'-carboxybiphenyl-4-yl)methyl]-4-(1-hydroxy-2-methylpropyl)imidazole-5-carboxamide

Following a procedure similar to that described in Example 45(d), but using 151 mg of 1-[(2'-t-butoxycarbonylbiphenyl-4-yl)methyl]-2-butyl-4-(1-hydroxy-2-methylpropyl)-5-carboxamide [prepared as described in step (c) above] and 5 ml of a 4 N solution of hydrogen chloride in dioxane, 119 mg of the hydrochloride of the title compound were obtained as an amorphous powder, melting at above 131°C (with softening).

Nuclear Magnetic Resonance Spectrum (hexadeuterated dimethyl sulphoxide) δ ppm:
0.73 (3H, doublet, J = 6.5 Hz);
0.79 (3H, triplet, J = 7.5 Hz);
0.98 (3H, doublet, J = 6.5 Hz);
1.1 - 1.6 (4H, multiplet);
1.9 - 2.1 (1H, multiplet);
2.98 (2H, triplet, J = 7.5 Hz);
4.65 (1H, doublet, J = 8 Hz);
5.69 (2H, singlet);
7.1 - 7.8 (8H, multiplet).

### EXAMPLE 49

### 1-[2'-Carboxybiphenyl-4-yl)methyl]-4-(1-hydroxybutyl)-2-propylimidazole-5-carboxamide (Compound No. 5-4)

### 49(a) 1-[(2'-t-Butoxycarbonylbiphenyl-4-yl)methyl]-4-butyryl-2-propylimidazole-5-carbonitrile

Following a procedure similar to that described in Example 45(a), but using 1.026 g of 4-butyryl-2-propylimidazole-5-carbonitrile (prepared as described in Preparation 28), 1.91 g of t-butyl 4'-(bromomethyl)- biphenyl-2-carboxylate and 0.209 g of sodium hydride (as a 55% w/w dispersion in mineral oil) in 20 ml of N,N-dimethylacetamide, 1.70 g of the title compound were obtained as a viscous oil.

Nuclear Magnetic Resonance Spectrum (CDCl₃) δ ppm:
1.00 (6H, triplet, J = 7.5 Hz);
1.25 (9H, singlet);
1.7 - 1.9 (4H, multiplet);
2.70 (2H, triplet, J = 7.5 Hz);
2.99 (2H, triplet, J = 7.5 Hz);
5.31 (2H, singlet);
7.1 - 7.9 (8H, multiplet).

### 49(b) 1-[(2'-t-Butoxycarbonylbiphenyl-4-yl)methyl]-4-(1-hydroxybutyl)-2-propylimidazole-5-carbonitrile

Following a procedure similar to that described in Example 45 (b), but using 1.13 g of 1-[(2'-t-butoxycarbonylbiphenyl-4-yl)methyl]-4-butyryl-2-propyl-imidazole-5-carbonitrile [prepared as described in step (a) above] and 0.091 g of sodium borophydride in 23 ml of ethanol, 1.07 g of the title compound were obtained as a viscous oil.

Nuclear Magnetic Resonance Spectrum (hexadeuterated dimethyl sulphoxide) δ ppm:
0.87 (3H, triplet, J = 7.5 Hz);
0.90 (3H, triplet, J = 7.5 Hz);
1.17 (9H, singlet);
1.2 - 1.4 (2H, multiplet);
1.5 - 1.7 (4H, multiplet);
2.67 (2H, triplet, J = 7.5 Hz);
4.58 (1H, multiplet);
5.34 (2H, singlet);
5.41 (1H, doublet, J = 4.5 Hz);
7.1 - 7.7 (8H, multiplet).

### 49(c) 1-[(2'-t-Butoxycarbonylbiphenyl-4-yl)methyl]-4-(1-hydroxybutyl)-2-propylimidazole-5-carboxamide

16 ml of a 1 N aqueous solution of sodium hydroxide were added to a solution of 1.07 g of 1-[(2'-t-butoxycarbonylbiphenyl-4-yl)methyl]-4-(1-hydroxybutyl)-2- propylimidazole-5-carbonitrile [prepared as described in step (b) above] in 16 ml of ethanol, and the resulting mixture was worked up in a similar manner to that described in Example 45(c), to afford 0.82 g of the title compound as an amorphous solid.

Nuclear Magnetic Resonance Spectrum (CDCl₃) δ ppm:
0.93 (3H, triplet, J = 7.5 Hz);
0.95 (3H, triplet, J = 7.5 Hz);
1.23 (9H, singlet);
1.2 - 2.1 (6H, multiplet);
2.60 (2H, triplet, J = 8 Hz);
4.89 (1H, triplet, J = 7.5 Hz);
5.56 & 5.77 (each 1H, AB-quartet, J = 16 Hz);
7.0 - 7.8 (8H, multiplet).

### 49(d) 1-[(2'-Carboxybiphenyl-4-yl)methyl]-4-(1-hydroxybutyl)-2-propylimidazole-5-carboxamide

Following a procedure similar to that described in Example 45(d), but using a solution of 0.82 g of 1-[(2'-t-butoxycarbonylbiphenyl-4-yl)methyl]-4- (1-hydroxybutyl)-2-propylimidazole-5-carboxamide [prepared as described in step (c) above] in 17 ml of a 4 N solution of hydrogen chloride in dioxane, 0.78 g of the hydrochloride of the title compound was obtained as an amorphous powder, melting at 118 - 121 °C (with softening).

Nuclear Magnetic Resonance Spectrum (CDCl₃) δ ppm:
0.90 (3H, triplet, J = 7.5 Hz);
0.93 (3H, triplet, J = 7.5 Hz);
1.1 - 1.5 (2H, multiplet);
1.7 - 2.1 (4H, multiplet);
2.9 - 3.1 (2H, multiplet);
5.00 (1H, triplet, J = 7.5 Hz);
5.46 & 5.56 (each 1H, AB-quartet, J = 15.5 Hz);
7.1 - 7.9 (8H, multiplet).

### EXAMPLE 52

### 2-Butyl-1-[(2'-carboxybiphenyl-4-yl)methyl]-4-hydroxymethylimidazole-5-carboxamide (Compound No. 5-63)

### 52 (a) Succinimido 1-[(2'-t-butoxycarbonylbiphenyl-4-yl)methyl]-2-butyl-4-hydroxymethylimidazole-5-carboxylate

206 mg of N,N-dicyclohexylcarbodiimide were added to a suspension of 464 mg of 1-[(2'-t-butoxycarbonyl-biphenyl-4-yl)methyl]-2-butyl-4-hydroxymethylimidazole5-carboxylic acid (prepared as described in Example 4) and 140 mg of N-hydroxysuccinimide in 10 ml of tetrahydrofuran, and the resulting mixture was stirred at room temperature for 16 hours. At the end of this time, the material which had precipitated was filtered off and the filtrate was concentrated by evaporation under reduced pressure. The concentrate was purified by column chromatography through silica gel, using a 1 : 15 by volume mixture of methanol and methylene chloride as the eluent, to afford 0.52 g of the title compound as crystals, melting at 107 - 109°C.

Nuclear Magnetic Resonance Spectrum (CDCl₃) δ ppm:
0.89 (3H, triplet, J = 7 Hz);
1.0 - 2.0 (4H, multiplet);
1.23 (9H, singlet);
2.70 (2H, triplet, J = 7.5 Hz);
2.69 (4H, singlet);
4.10 (1H, broad singlet);
4.96 (2H, singlet);
5.56 (2H, singlet) ;
7.00 - 7.90 (8H, multiplet).

### 52(b) 1-[(2'-t-Butoxycarbonylbiphenyl-4-yl)methyl]-2-butyl-4-hydroxymethylimidazole-5-carboxamide

0.5 ml of concentrated aqueous ammonia was added to a solution of 0.60 g of succinimido 1-[(2'-t-butoxycarbonylbiphenyl-4-yl)methyl]-2-butyl-4-hydroxymethylimidazole-5-carboxylate [prepared as described in step (a) above] in 6 ml of tetrahydrofuran, and the title compound started to separate immediately. The solvent was removed by distillation under reduced pressure, and the resulting residue was washed with diethyl ether and with water, to afford 0.38 g of the title compound as a powder, melting at 222 - 224°C.

Nuclear Magnetic Resonance Spectrum (hexadeuterated dimethyl sulphoxide) δ ppm:
0.85 (3H, triplet, J = 7 Hz);
1.19 (9H, singlet);
1.0 - 1.9 (4H, multiplet);
2.57 (2H, triplet, J = 7.5 Hz);
4.52 (2H, doublet, J = 4.5 Hz);
5.63 (2H, singlet);
5.83 (1H, triplet, J = 4.5 Hz);
6.95 - 7.8 (8H, multiplet).

### 52(c) 2-Butyl-1-[(2'-carboxybiphenyl-4-yl)methyl]-4-hydroxymethylimidazole-5-carboxamide

A solution of 0.28 g of 1-[(2'-t-butoxycarbonyl-biphenyl-4-yl)methyl]-2-butyl-4-hydroxymethylimidazole5-carboxamide [prepared as described in step (b) above] in 3 ml of a 4 N solution of hydrogen chloride in dioxane was stirred at room temperature for 5 hours and then concentrated by evaporation under reduced pressure. The concentrate was triturated with a mixture of ethyl acetate and diethyl ether, and the solidified material was collected by filtration, to afford 0.26 g of the hydrochloride of the title compound, which softened at above 150°C and completely decomposed at 235°C.

Nuclear Magnetic Resonance Spectrum (hexadeuterated dimethyl sulphoxide) δ ppm:
0.80 (3H, triplet, J = 7.5 Hz);
1.20 - 1.31 (2H, multiplet);
1.43 - 1.54 (2H, multiplet);
2.96 (3H, triplet, J = 7.5 Hz);
4.68 (2H, singlet);
5.71 (2H, singlet);
7.21 - 7.75 (8H, multiplet).

### EXAMPLE 53

### N-Methyl-2-butyl-1-[(2'-carboxybiphenyl-4-yl)methyl]-4-hydroxymethylimidazole-5-carboxamide (Compound No. 5-71)

### 53(a) N-methyl-1-[(2'-t-butoxycarbonylbiphenyl-4-yl)-methyl]-2-butyl-4-hydroxymethylimidazole-5-carboxamide

0.4 ml of a 40% by volume solution of methylamine in water was added at room temperature to a solution of 0.278 g of succinimido 1-[(2'-t-butoxycarbonylbiphenyl4-yl)methyl]-2-butyl-4-hydroxymethylimidazole-5- carboxylate [prepared as described in Example 52(a)] in a mixture of 3 ml of methylene chloride and 2 ml of methanol, and the resulting mixture was allowed to stand for 16 hours at room temperature. At the end of this time, the solution was concentrated by evaporation under reduced pressure, and the concentrate was dissolved in ethyl acetate. The resulting solution was washed with an aqueous solution of potassium bisulphate and with an aqueous solution of sodium hydrogencarbonate, in that order, after which it was dried over anhydrous magnesium sulphate. The solvent was then removed by distillation under reduced pressure, and the resulting residue was purified by column chromatography through silica gel, using ethyl acetate as the eluent, to give 176 mg of the title compound as a glass.

Nuclear Magnetic Resonance Spectrum (CDCl₃) δ ppm:
0.85 (3H, triplet, J = 7 Hz);
1.23 (9H, singlet);
1.0 - 2.0 (4H, multiplet);
2.54 (2H, triplet, J = 7.5 Hz);
2.91 (3H, doublet, J = 5 Hz);
4.70 (2H, singlet);
5.62 (2H, singlet);
6.9 - 7.85 (8H, multiplet);
8.38 (1H, quartet, J = 5 Hz).

### 53(b) N-Methyl-2-butyl-1-[(2'-carboxybiphenyl-4-yl)-methyl]-4-hydroxymethylimidazole-5-carboxamide

A solution of N-methyl-1-[(2'-t-butoxycarbonyl-biphenyl-4-yl)methyl]-2-butyl-4-hydroxymethylimidazole5-carboxamide [prepared as described in step (a) above] in 2 ml of a 4 N solution of hydrogen chloride in dioxane was allowed to stand at room temperature for 16 hours and then concentrated by evaporation under reduced pressure. The resulting crystalline residue was washed with a mixture of ethyl acetate and diethyl ether, to afford 0.15 g of the hydrochloride of the title compound, melting at 205 - 208°C (with decomposition).

Nuclear Magnetic Resonance Spectrum (hexadeuterated dimethyl sulphoxide) δ ppm:
0.81 (3H, triplet, J = 7.5 Hz);
1.25 (2H, sextet, J = 7.5 Hz);
1.49 (2H, quintet, J = 7.5 Hz);
2.75 (3H, doublet, J = 4.5 Hz) ;
2.96 (2H, triplet, J = 8 Hz);
5.64 (2H, singlet);
7.21 - 7.75 (8H, multiplet);
8.91 (1H, quartet, J = 4.5 Hz).

### EXAMPLE 54

### N-Ethoxycarbonylmethyl-2-butyl-1-[(2'-carboxybiphenyl-4-yl)methyl]-4-hydroxymethylimidazole-5-carboxamide (Compound No. 5-126)

Following a procedure similar to that described in Example 53, but using 0.307 g of succinimido 1-[(2'-t-butoxycarbonylbiphenyl-4-yl)methyl]-2-butyl-4-hydroxy-methylimidazole-5-carboxylate [prepared as described in Example 52(a)], 89 mg of ethyl glycinate hydrochloride and 0.089 ml of triethylamine, 0.202 g of the hydrochloride of the title compound was obtained as an amorphous powder, melting at above 80°C (with softening).

Nuclear Magnetic Resonance Spectrum (hexadeuterated dimethyl sulphoxide) δ ppm:
0.80 (3H, triplet, J = 7.5 Hz);
1.18 (3H, triplet, J = 7 Hz);
1.20 - 1.33 (2H, multiplet);
1.47 (2H, quintet, J = 7.5 Hz);
2.94 (2H, triplet, J = 8 Hz);
4.05 (2H, doublet, J = 6 Hz);
4.12 (2H, quartet, J = 7 Hz);
4.72 (2H, singlet);
5.63 (2H, singlet);
7.24 - 7.75 (8H, multiplet);
9.37 (1H, triplet, J = 6 Hz).

### EXAMPLE 55

### N-Carboxymethyl-2-butyl-1-[(2'-carboxybiphenyl-4-yl)-methyl]-4-hydroxymethylimidazole-5-carboxamide (Compound No. 5-125)

Following a procedure similar to that described in Example 53, but using 0.32 g of succinimido 1-[(2'-t-butoxycarbonylbiphenyl-4-yl)methyl]-2-butyl-4-hydroxymethylimidazole-5-carboxylate [prepared as described in Example 52(a)], 0.11 g of t-butyl glycinate hydrochloride and 80 mg of 4-dimethylaminopyridine, 0.21 g of the hydrochloride of the title compound was obtained as an amorphous powder, melting at above 110° C (with softening).

Nuclear Magnetic Resonance Spectrum (hexadeuterated dimethyl sulphoxide) δ ppm:
0.81 (3H, triplet, J = 7.5 Hz);
1.25 (2H, sextet, J = 7.5 Hz);
1.48 (2H, quintet, J = 7.5 Hz);
2.95 (2H, triplet, J = 8 Hz);
3.98 (2H, doublet, J = 6 Hz);
4.71 (2H, singlet);
5.64 (2H, singlet);
7.26 - 7.75 (8H, multiplet);
9.22 (1H, triplet, J = 6 Hz).

### EXAMPLE 58

### Methyl (S)-N-{2-butyl-1-[(2'-carboxybiphenyl-4-yl)methyl]-4-hydroxymethylimidazole-5-carbonyl}-prolinate (Compound No. 5-335)

Following a procedure similar to that described in Example 53, but using 529 mg of succinimido 1-[(2'-t-butoxycarbonylbiphenyl-4-yl)methyl]-2-butyl-4-hydroxy-methylimidazole-5-carboxylate [prepared as described in Example 52(a)], 180 mg of methyl (S)-prolinate hydrochloride and 0.2 ml of triethylamine, 0.39 g of the hydrochloride of the title compound was obtained as an amorphous powder, melting at above 120°C (with softening).

Nuclear Magnetic Resonance Spectrum (hexadeuterated dimethyl sulphoxide) δ ppm:
0.88 (3H, triplet, J = 7.5 Hz);
1.34 (2H, sextet, J = 7.5 Hz);
1.4 - 2.25 (6H, multiplet);
2.9 - 3.7 (2H, multiplet);
3.64 (3H, singlet);
4.34 (1H, triplet, J = 7.5 Hz);
4.55 (2H, singlet);
5.25 & 5.56 (each 1H, AB-quartet, J = 15.5 Hz);
7.26 - 7.77 (8H, multiplet).

### EXAMPLE 59

### 2-Butyl-1-[(2'-carboxybiphenyl-4-yl)methyl]-4-(1-hydroxy-2,2-dimethylpropyl)imidazole-5-carboxamide (Compound No. 5-68)

### 59 (a) Methyl 1-[(2'-t-butoxycarbonylbiphenyl-4-yl)-methyl]-2-butyl-4-formylimidazole-5-carboxylate

5.07 ml of triethylamine and 6.0 g of sulphur trioxide}pyridine complex were added, in turn, at a temperature of 10°C to 15°C to a solution of 3.0 g of methyl 1-[(2'-t-butoxycarbonylbiphenyl-4-yl)methyl]-2-butyl-4-hydroxymethylimidazole-4-carboxylate [prepared as described in Example 1(b)] in 18 ml of dimethyl sulphoxide, and the resulting mixture was stirred at the same temperature for 45 minutes. At the end of this time, the reaction mixture was mixed with water and extracted with ethyl acetate. The extract was washed with water and with an aqueous solution of sodium hydrogencarbonate, in that order, after which it was dried over anhydrous magnesium sulphate, and the solvent was removed by distillation under reduced pressure. The resulting residue was purified by column chromatography through silica gel, using a 1 : 1 by volume mixture of hexane and ethyl acetate as the eluent, to afford 2.88 g of the title compound as an amorphous solid.

Nuclear Magnetic Resonance Spectrum (CDCl₃) δ ppm:
0.90 (3H, triplet, J = 7 Hz);
1.25 (9H, singlet);
1.1 - 2.1 (4H, multiplet);
2.77 (2H, triplet, J = 8 Hz);
3.91 (3H, singlet);
5.65 (2H, singlet);
6.9 - 7.9 (8H, multiplet);
10.48 (1H, singlet).

### 59(b) Methyl 1-[(2'-t-butoxycarbonylbiphenyl-4-yl)-methyl]-2-butyl-4-(1-hydroxy-2,2-dimethylpropyl)-imidazole-5-carboxylate

2.77 ml of a 2 M solution of t-butylmagnesium bromide in tetrahydrofuran were added at -55°C and under an atmosphere of nitrogen to a solution of 1.32 g of methyl 1-[(2'-t-butoxycarbonylbiphenyl-4-yl)methyl]-2- butyl-4-formylimidazole-5-carboxylate [prepared as described in step (a) above] in 26 ml of tetrahydrofuran, and the resulting mixture was stirred at a temperature of -55°C to -50°C for 30 minutes. At the end of this time, the reaction mixture was diluted with 50 ml of ethyl acetate and with a saturated aqueous solution of ammonium chloride. The organic layer was separated and dried over anhydrous magnesium sulphate and the solvent was removed by distillation under reduced pressure. The residue was purified by column chromatography through silica gel, using a 2 : 1 by volume mixture of hexane and ethyl acetate as the eluent, to afford 0.87 g of the title compound as an amorphous solid.

Nuclear Magnetic Resonance Spectrum (CDCl₃) δ ppm:
0.90 (3H, triplet, J = 7.5 Hz);
0.93 (9H, singlet);
1.0 - 2.0 (4H, multiplet);
1.19 (9H, singlet);
2.68 (2H, triplet, J = 7.5 Hz);
3.41 (1H, doublet, J = 10 Hz);
3.74 (3H, singlet);
4.92 (1H, doublet, J = 10 Hz);
5.59 (2H, singlet);
6.9 - 7.9 (8H, multiplet).

### 59(c) 1-[(2'-t-Butoxycarbonylbiphenyl-4-yl)methyl]-2-butyl-4-(1-hydroxy-2,2-dimethylpropyl)imidazole-5-carboxylic acid

Following a procedure similar to that described in Example 4, 0.87 g of methyl 1-[(2'-t-butoxycarbonylbiphenyl-4-yl)methyl]-2-butyl-4-(1-hydroxy-2,2-dimethylpropyl) imidazole-5-carboxylate [prepared as described in step (b) above] was hydrolyzed, using 342 mg of lithium hydroxide monohydrate, to afford 0.73 g of the title compound as crystals, melting at 199 - 201°C (with decomposition).

Nuclear Magnetic Resonance Spectrum (hexadeuterated dimethyl sulphoxide) δ ppm:
0.84 (3H, triplet, J = 7.5 Hz);
0.89 (9H, singlet);
1.16 (9H, singlet);
1.22 - 1.4 (2H, multiplet);
1.58 (2H, quintet, J = 7.5 Hz);
2.64 (2H, triplet, J = 7.5 Hz);
4.78 (1H, singlet);
5.68 (2H, AB-quartet, Δδ = 0.14 ppm, J = 17 Hz);
7.02 (2H, doublet, J = 8 Hz);
7.22 - 7.58 (5H, multiplet);
7.65 (1H, doublet, J = 7.5 Hz).

### 59(d) Succinimido 1-[(2'-t-butoxycarbonylbiphenyl-4-yl)- methyl]-2-butyl-4-(1-hydroxy-2,2-dimethylpropyl)-imidazole-5-carboxylate

Following a procedure similar to that described in Example 52(a), but using 600 mg of 1-[(2'-t-butoxy-carbonylbiphenyl-4-yl)methyl]-2-butyl-4-(1-hydroxy-2,2- dimethylpropyl)imidazole-5-carboxylic acid [prepared as described in step (c) above], 172 mg of N-hydroxysuccinimide and 250 mg of N,N-dicyclohexylcarbodiimide, 663 mg of the title compound were obtained as an amorphous solid.

Nuclear Magnetic Resonance Spectrum (CDCl₃) δ ppm:
0.92 (3H, triplet, J = 7.5 Hz);
1.01 (9H, singlet);
1.21 (9H, singlet);
1.38 (2H, sextet, J = 7.5 Hz);
1.73 (2H, quintet, J = 7.5 Hz);
2.71 (2H, triplet, J = 7.5 Hz);
2.84 (4H, singlet);
4.99 (1H, doublet, J = 7.5 Hz);
5.53 (2H, singlet);
7.03 (2H, doublet, J = 8.5 Hz);
7.26 - 7.50 (5H, multiplet);
7.77 (1H, doublet, J = 8 Hz).

### 59(e) 1-[(2'-t-Butoxycarbonylbiphenyl-4-yl)methyl]-2-butyl-4-(1-hydroxy-2,2-dimethylpropyl)imidazole-5-carboxamide

Following a procedure similar to that described in Example 52(b), but using 0.66 g of succinimido 1-[(2'-tbutoxycarbonylbiphenyl-4-yl)methyl]-2-butyl-4-(1-hydroxy2,2-dimethylpropyl) imidazole-5-carboxylate [prepared as described in step (d) above], 0.33 g of the title compound was obtained as an amorphous solid.

Nuclear Magnetic Resonance Spectrum (CDCl₃) δ ppm:
0.89 (3H, triplet, J = 7.5 Hz);
0.96 (9H, singlet);
1.22 (9H, singlet);
1.34 (2H, sextet, J = 7.5 Hz);
1.64 (2H, quintet, J = 7.5 Hz);
2.62 (2H, triplet, J = 7.5 Hz);
4.67 (1H, doublet, J = 5.5 Hz);
5.48 & 5.82 (each 1H, AB-quartet, J = 16 Hz);
7.02 (2H, doublet, J = 8.5 Hz);
7.23 - 7.50 (5H, multiplet);
7.76 (1H, doublet, J = 6.5 Hz).

### 59(f) 2-Butyl-1-[(2'-carboxybiphenyl-4-yl)methyl]-4-(1-hydroxy-2,2-dimethylpropyl)imidazole-5-carboxamide

Following a procedure similar to that described in Example 52(c), but using 326 mg of 1-[(2'-t-butoxycarbonylbiphenyl-4-yl)methyl]-2-butyl-4-(1-hydroxy-2,2- dimethylpropyl)imidazole-5-carboxamide [prepared as described in step (e) above], 228 mg of the hydrochloride of the title compound were obtained as a powdery solid, melting at 150 - 154°C (with softening).

Nuclear Magnetic Resonance Spectrum (hexadeuterated dimethyl sulphoxide) δ ppm:
0.80 (3H, triplet, J = 7.5 Hz);
0.91 (9H, singlet);
1.24 (2H, sextet, J = 7.5 Hz);
1.45 (2H, quintet, J = 7.5 Hz);
2.99 (2H, triplet, J = 7.5 Hz);
4.78 (1H, singlet);
5.69 (2H, singlet);
7.21 (2H, doublet, J = 8 Hz);
7.33 - 7.61 (5H, multiplet);
7.75 (1H, doublet, J = 8 Hz).

### EXAMPLE 60

### 1-[(2'-Carboxybiphenyl-4-yl)methyl]-4-(1-hydroxy-2,2-dimethylpropyl)-2-propylimidazole-5-carboxamide (Compound No. 5-6)

### 60(a) Diethyl 1-[(2'-t-butoxycarbonylbiphenyl-4-yl)-methyl]-2-propylimidazole-4,5-dicarboxylate

Following a procedure similar to that described in Example 1(a), but using 9.0 g of diethyl 2-propylimidazole-4,5-dicarboxylate (prepared as described in Preparation 12), 12.3 g of t-butyl 4'-bromomethylbiphenyl-2-carboxylate and 4.1 g of potassium t-butoxide as a base, 16.47 g of the title compound were obtained as a viscous oil.

Nuclear Magnetic Resonance Spectrum (CDCl₃) δ ppm:
0.95 (3H, triplet, J = 7.5 Hz);
1.5 - 2.0 (2H, multiplet);
1.23 (9H, singlet);
1.25 (3H, triplet, J = 7 Hz);
1.37 (3H, triplet, J = 7 Hz);
2.69 (2H, triplet, J = 7 Hz);
4.26 (2H, quartet, J = 7 Hz);
4.38 (2H, quartet, J = 7 Hz);
5.48 (2H, singlet);
7.0 - 7.9 (8H, multiplet).

### 60(b) Ethyl 1-[(2'-t-butoxycarbonylbiphenyl-4-yl)methyl]-4-hydroxymethyl-2-propylimidazole-5-carboxylate

Following a procedure similar to that described in Example 1(b), 16.47 g of diethyl 1-[(2'-t-butoxy-carbonylbiphenyl-4-yl)methyl]-2-propylimidazole-4,5-dicarboxylate [prepared as described in step (a) above] were reduced, using 44.4 ml of a 1.5 M solution of diisobutylaluminium hydride in tetrahydrofuran, to afford 10.83 g of the title compound as crystals, melting at 108 - 110°C.

Nuclear Magnetic Resonance Spectrum (CDCl₃) δ ppm:
0.98 (3H, triplet, J = 7.5 Hz);
1.23 (9H, singlet);
1.31 (3H, triplet, J = 7 Hz);
1.79 (2H, sextet, J = 7.5 Hz);
2.67 (2H, triplet, J = 7.5 Hz);
4.27 (2H, quartet, J = 7 Hz);
4.87 (2H, singlet);
5.59 (2H, singlet);
7.00 (2H, doublet, J = 8.5 Hz);
7.24 - 7.75 (5H, multiplet);
7.78 (1H, doublet, J = 7 Hz).

### 60(c) Ethyl 1-[(2'-t-butoxycarbonylbiphenyl-4-yl)methyl]-4-formyl-2-propylimidazole-5-carboxylate

Following a procedure similar to that described in Example 59(a), 2.71 g of ethyl 1-[(2'-t-butoxycarbonylbiphenyl-4-yl)methyl]-4-hydroxymethyl-1-propylimidazole5-carboxylate [prepared as described in step (b) above] were oxidized with 4.6 ml of triethylamine and 5.5 g of sulphur trioxide/pyridine complex in 17 ml of dimethyl sulphoxide, to afford 2.57 g of the title compound as crystals, melting at 117 - 119°C.

Nuclear Magnetic Resonance Spectrum (CDCl₃) δ ppm:
0.99 (3H, triplet, J = 7.5 Hz);
1.26 (9H, singlet);
1.38 (3H, triplet, J = 7 Hz);
1.84 (2H, sextet, J = 7.5 Hz);
2.73 (2H, triplet, J = 7.5 Hz);
4.40 (2H, quartet, J = 7 Hz);
5.67 (2H, singlet);
7.02 (2H, doublet, J = 8.5 Hz);
7.29 - 7.54 (5H, multiplet);
7.80 (1H, doublet, J = 8 Hz);
10.48 (1H, singlet).

### 60 (d) Ethyl 1-[(2'-t-butoxycarbonylbiphenyl-4-yl)methyl]-4-(1-hydroxy-2,2-dimethylpropyl)-2-propylimidazole5-carboxylate

Following a procedure similar to that described in Example 59(b), 1.14 g of ethyl 1-[(2'-t-butoxycarbonylbiphenyl-4-yl)methyl]-4-formyl-2-propylimidazole-5- carboxylate [prepared as described in step (c) above] was reacted with 2.4 ml of a 2 M solution of t-butylmagnesium bromide in tetrahydrofuran, to afford 0.78 g of the title compound as a viscous oil.

Nuclear Magnetic Resonance Spectrum (CDCl₃) δ ppm:
0.97 (3H, triplet, J = 7.5 Hz);
1.00 (9H, singlet);
1.25 (9H, singlet);
1.35 (3H, triplet, J = 7 Hz);
1.77 (2H, sextet, J = 7.5 Hz);
2.68 (2H, triplet, J = 7.5 Hz);
3.46 (1H, doublet, J = 9 Hz);
4.29 (2H, quartet, J = 7 Hz);
4.99 (1H, doublet, J = 9 Hz);
5.62 (2H, singlet);
7.00 (2H, doublet, J = 8 Hz);
7.29 - 7.54 (5H, multiplet);
7.80 (1H, doublet, J = 7.5 Hz).

### 60(e) 1-[(2'-t-Butoxycarbonylbiphenyl-4-yl)methyl]-4-(1-hydroxy-2,2-dimethylpropyl)-2-propylimidazole5-carboxylic acid

Following a procedure similar to that described in Example 4, 0.78 g of ethyl 1-[(2'-t-butoxycarbonyl-biphenyl-4-yl)methyl]-4-(1-hydroxy-2,2-dimethylpropyl)- 2-propylimidazole-5-carboxylate [prepared as described in step (d) above] was hydrolyzed, using 209 mg of lithium hydroxide monohydrate, to afford 0.62 g of the title compound as crystals, melting at 207°C.

Nuclear Magnetic Resonance Spectrum (hexadeuterated dimethyl sulphoxide) δ ppm:
0.88 (3H, triplet, J = 7.5 Hz);
0.89 (9H, singlet);
1.15 (9H, singlet);
1.63 (2H, sextet, J = 7.5 Hz);
2.63 (2H, triplet, J = 7.5 Hz);
4.79 (1H, singlet);
5.63 & 5.76 (each 1H, AB-quartet, J = 18.5 Hz);
7.02 (2H, doublet, J = 8 Hz);
7.22 - 7.67 (6H, multiplet).

### 60(f) Succinimido 1-[(2'-t-Butoxycarbonylbiphenyl-4-yl)methyl)-4-(1-hydroxy-2,2-dimethylpropyl)-2-propylimidazole-5-carboxylate

Following a procedure similar to that described in Example 52(a), but using 300 mg of 1-[(2'-t-butoxycarbonylbiphenyl-4-yl)methyl]-4-(1-hydroxy-2,2-dimethylpropyl)-2-propylimidazole-5-carboxylic acid [prepared as described in step (e) above], 110 mg of N-hydroxysuccinimide and 130 mg of N,N-dicyclohexylcarbodiimide, 321 mg of the title compound were obtained as an amorphous solid.

Nuclear Magnetic Resonance Spectrum (CDCl₃) δ ppm:
0.94 (3H, triplet, J = 7.5 Hz);
0.98 (9H, singlet);
1.18 (9H, singlet);
1.75 (2H, sextet, J = 7.5 Hz);
2.64 (2H, triplet, J = 7.5 Hz);
3.12 (1H, doublet, J = 9.5 Hz);
4.98 (1H, doublet, J = 9.5 Hz);
5.52 (2H, singlet);
7.0 - 7.9 (8H, multiplet).

### 60(g) 1-[(2'-t-Butoxycarbonylbiphenyl-4-yl)methyl]-4-(1-hydroxy-2,2-dimethylpropyl)-2-propylimidazole-5-carboxamide

Following a procedure similar to that described in Example 52(b), but using 0.13 g of succinimido 1-[(2'-tbutoxycarbonylbiphenyl-4-yl)methyl]-4-(1-hydroxy-2,2-dimethylpropyl)-2-propylimidazole-5-carboxylate [prepared as described in step (f) above], 0.12 g of the title compound was obtained as a glass.

Nuclear Magnetic Resonance Spectrum (CDCl₃) δ ppm:
0.88 (3H, triplet, J = 7.5 Hz);
0.90 (9H, singlet);
1.24 (9H, singlet);
1.60 (2H, sextet, J = 7.5 Hz);
2.58 (2H, triplet, J = 7.5 Hz);
4.65 (1H, doublet, J = 6 Hz);
5.53 & 5.87 (each 1H, AB-quartet, J = 16 Hz);
7.02 (2H, doublet, J = 8 Hz);
7.23 - 7.48 (5H, multiplet);
7.78 (1H, doublet, J = 6.5 Hz).

### 60(h) 1-[(2'-Carboxybiphenyl-4-yl)methyl]-4-(1-hydroxy2,2-dimethylpropyl)-2-propylimidazole-5-carboxamide

Following a procedure similar to that described in Example 52(c), but using 139 mg of 1-[(2'-t-butoxy-carbonylbiphenyl-4-yl)methyl]-4-(1-hydroxy-2, 2-dimethylpropyl)-2-propylimidazole-5-carboxamide [prepared as described in step (g) above], 96 mg of the hydrochloride of the title compound were obtained as a powder, melting at above 160°C (with softening).

Nuclear Magnetic Resonance Spectrum (hexadeuterated dimethyl sulphoxide) δ ppm:
0.82 (3H, triplet, J = 7.5 Hz);
0.90 (9H, singlet);
1.53 (2H, sextet, J = 7.5 Hz);
2.97 (2H, triplet, J = 7.5 Hz);
4.79 (1H, singlet);
5.69 (2H, singlet);
7.19 - 7.75 (8H, multiplet).

### EXAMPLE 73

### Pivaloyloxymethyl 4-(1-hydroxyethyl)-2-propyl-1-{4-[2-tetrazol-5-yl)phenyl ]phenyl}methylimidazole-5-carboxylate (Compound No.4-31)

### 73(a) Pivaloyloxymethyl 4-(1-hydroxyethyl)-2-propyl-1-{4-[2-(trityltetrazol-5-yl)phenyl]phenyl}-methylimidazole-5-carboxylate

A solution of 196 mg of lithium hydroxide monohydrate in 15 ml of water was added to a solution of 2.87 g of ethyl 4-(1-hydroxyethyl)-2-propyl-1-{4-[2- (trityltetrazol-5-yl)phenyl]phenyl}methylimidazole-5- carboxylate [prepared as described in Example 42(a)] in 30 ml of dioxane, and the resulting mixture was stirred at room temperature for 16 hours. At the end of this time, small pieces of dry ice were added to the mixture, which was then concentrated by evaporation under reduced pressure to dryness. The residue was dissolved in 40 ml of N,N-dimethylacetamide, and 0.45 g of potassium carbonate and then 1.1 ml of pivaloyloxymethyl chloride were added to the solution. The resulting mixture was stirred at 50°C for 3 hours. At the end of this time, water and ethyl acetate were added to the reaction mixture, and the ethyl acetate layer was separated, dried over anhydrous magnesium sulphate and concentrated by evaporation under reduced pressure. The residue was purified by column chromatography through silica gel, using a 1 : 1 by volume mixture of ethyl acetate and hexane as the eluent, to give 2.41 g of the title compound as an amorphous powder.

Nuclear Magnetic Resonance Spectrum (CDCl₃) δ ppm:
0.88 (3H, triplet, J = 7.5 Hz);
1.17 (9H, singlet);
1.50 (3H, doublet, J = 6 Hz);
1.69 (2H, sextet, J = 7.5 Hz);
2.51 (2H, triplet, J = 7.5 Hz);
3.62 (1H, doublet, J = 8 Hz);
5.17 - 5.29 (1H, multiplet);
5.37 (1H, doublet, J = 16.5 Hz);
5.46 (1H, doublet, J = 16.5 Hz);
5.77 (1H, doublet, J = 5.5 Hz);
5.82 (1H, doublet, J = 5.5 Hz);
6.75 (2H, doublet, J = 8.5 Hz);
6.92 - 7.89 (20H, multiplet);
7.90 (1H, doublet, J = 7.5 Hz).

### 73(b) Pivaloyloxymethyl 4-(1-hydroxyethyl)-2-propyl-1-{4-[2-(tetrazol-5-yl)phenyl]phenyl}methylimidazole-5-carboxylate

Following a procedure similar to that described in Example 35(c), but using 2.87 g of pivaloyloxymethyl 4-(1-hydroxyethyl)-2-propyl-1-{4-[2-(trityltetrazol-5-yl)phenyl]phenyl}methylimidazole-5-carboxylate [prepared as described in step (a) above] and 75% v/v aqueous acetic acid, 1.21 g of the title compound was obtained as a powder.

Nuclear Magnetic Resonance Spectrum (CDCl₃) δ ppm:
0.90 (3H, triplet, J = 7.5 Hz);
1.13 (9H, singlet);
1.43 (3H, doublet, J = 6.5 Hz);
1.67 (2H, sextet, J = 7.5 Hz);
2.55 (3H, triplet, J = 7.5 Hz);
5.16 (1H, quartet, J = 6.5 Hz);
5.40 (1H, doublet, J = 16.5 Hz);
5.51 (1H, doublet, J = 16.5 Hz);
5.80 (1H, doublet, J = 6 Hz);
5.85 (1H, doublet, J = 6 Hz);
6.86 (2H, doublet, J = 8 Hz);
7.08 (2H, doublet, J = 8 Hz);
7.40 - 7.61 (3H, multiplet);
7.92 (1H, doublet, J = 7.5 Hz).

### EXAMPLE 74

### 4-(1-Hydroxy-2,2-dimethylpropyl)-2-propyl-1-{4-[2-(tetrazol-5-yl)phenyl]phenyl}methylimidazole-5-carboxamide (Compound No. 5-37)

### 74(a) 2-Propyl-4-pivaloyl-1-{4-[2-(trityltetrazol-5-yl)phenyl]phenyl}methylimidazole-5-carbonitrile

1.08 g of potassium t-butoxide was added, whilst ice-cooling, to a solution of 2.00 g of 2-propyl-4-pivaloylimidazole-5-carbonitrile (prepared as described in Preparation 41) in 20 ml of N,N-dimethylacetamide, and the resulting mixture was stirred at same temperature for 10 minutes. 6.10 g of 4-[2-(trityltetrazol-5-yl)phenyl]benzyl bromide were then added to the solution, and the resulting mixture was stirred at 50°C for 4 hours. At the end of this time, ethyl acetate and water were added to the mixture, and the ethyl acetate layer was separated, dried over anhydrous magnesium sulphate and concentrated by evaporation under reduced pressure. The syrupy residue was purified by column chromatography through silica gel, using 1 : 3 and 1 : 2 by volume mixtures of ethyl acetate and hexane as the eluent, to give 5.44 g of the title compound as crystals, melting at 107 - 110°C.

Nuclear Magnetic Resonance Spectrum (CDCl₃) δ ppm:
0.92 (3H, triplet, J = 7.5 Hz);
1.42 (9H, singlet);
1.72 (2H, sextet, J = 7.5 Hz);
2.50 (2H, triplet, J = 7.5 Hz);
5.09 (2H, singlet);
6.92 (2H, doublet, J = 8 Hz);
7.13 - 7.53 (20H, multiplet);
7.95 (1H, doublet, J = 7 Hz).

### 74(b) 4-(1-Hydroxy-2,2-dimethylpropyl)-2-propyl-1-{4-[2-(trityltetrazol-5-yl)phenyl]phenyl}methylimidazole-5-carbonitrile

A solution of 108 mg of sodium borohydride in 20 ml of ethanol was added to a solution of 2.00 g of 2-propyl-4-pivaloyl-1-{4-[2-(trityltetrazol-5-yl)-phenyl]phenyl}methylimidazole-5-carbonitrile [prepared as described in step (a) above] in 40 ml of tetrahydrofuran, and the mixture was stirred at room temperature for 2.5 hours. At the end of this time, the reaction mixture was concentrated by evaporation under reduced pressure, and the residue was dissolved in a mixture of ethyl acetate and water. The ethyl acetate layer was separated, washed with water, dried over anhydrous magnesium sulphate and concentrated by evaporation under reduced pressure. The syrupy residue was crystallized in a 1 : 4 by volume mixture of ethyl acetate and hexane, to give 1.93 g of the title compound as crystals, melting at 115 - 117°C.

Nuclear Magnetic Resonance Spectrum (CDCl₃) δ ppm:
0.87 (3H, triplet, J = 7.5 Hz);
0.99 (9H, singlet);
1.64 (2H, sextet, J = 7.5 Hz);
2.49 (2H, triplet, J = 7.5 Hz);
2.76 (1H, doublet, J = 7.5 Hz);
4.46 (1H, doublet, J = 7.5 Hz);
5.04 (2H, singlet);
6.85 - 7.53 (22H, multiplet);
7.95 (1H, doublet, J = 7.5 Hz).

### 74(c) 4-(1-Hydroxy-2,2-dimethylpropyl)-2-propyl-1-{4-[2-(tetrazol-5-yl)phenyl]phenyl}methylimidazole5-carbonitrile

A suspension of 1.65 g of 4-(1-hydroxy-2,2-dimethylpropyl)-2-propyl-1-{4-[2-(trityltetrazol-5-yl)phenyl]- phenyl}methylimidazole-5-carbonitrile [prepared as described in step (b) above] in 24 ml of 75% v/v aqueous acetic acid was stirred at 60°C for 2 hours. At the end of this time, 6 ml of water was added to the reaction mixture, which was then cooled with ice. The trityl alcohol which precipitated was removed by filtration, and the filtrate was concentrated by evaporation under reduced pressure, to give 1.07 g of the title compound as a glass.

Nuclear Magnetic Resonance Spectrum (CDCl₃) δ ppm:
0.87 (3H, triplet, J = 7.5 Hz);
0.92 (9H, singlet);
1.63 (2H, sextet, J = 7.5 Hz);
2.58 (2H, triplet, J = 7.5 Hz);
4.36 (1H, singlet);
5.15 (2H, singlet);
7.00 (2H, doublet, J = 8 Hz);
7.07 (2H, doublet, J = 8 Hz);
7.30 - 7.61 (3H, multiplet);
7.80 (1H, doublet, J = 7.5 Hz).

### 74(d) 4-(1-Hydroxy-2,2-dimethylpropyl)-2-propyl-1-{4-[2-(tetrazol-5-yl)phenyl]phenyl}methylimidazole5-carboxamide

A mixture of 0.70 g of 4-(1-hydroxy-2,2-dimethylpropyl)-2-propyl-1-{4-[2-(tetrazol-5-yl)phenyl]-phenyl}methylimidazole-5-carbonitrile [prepared as described in step (c) above] in 14 ml of 1 N aqueous sodium hydroxide and 7 ml of ethanol was heated under reflux for 2 hours. At the end of this time, the ethanol in the reaction mixture was removed by evaporation under reduced pressure, and ethyl acetate and 14 ml of 1 N aqueous hydrochloric acid were added to the residue. The ethyl acetate layer was separated, dried over anhydrous magnesium sulphate and concentrated by evaporation under reduced pressure, to give 0.45 g of the title compound as a powder, melting at 174 - 176°C.

Nuclear Magnetic Resonance Spectrum (hexadeuterated dimethyl sulphoxide) δ ppm:
0.83 (3H, triplet, J = 7.5 Hz);
0.88 (9H, singlet);
1.44 - 1.63 (2H, multiplet);
2.46 (2H, triplet, J = 7.5 Hz);
4.45 (1H, singlet);
5.39 (1H, doublet, J = 16 Hz);
5.77 (1H, doublet, J = 16 Hz);
6.20 (1H, doublet, J = 4.5 Hz);
6.91 (2H, doublet, J = 8.5 Hz);
7.04 (2H, doublet, J = 8.5 Hz);
7.47 - 7.63 (4H, multiplet);

### EXAMPLE 75

### 2-Butyl-4-(1-hydroxy-2,2-dimethylpropyl)-1-{4-[2-(tetrazol-5-yl)phenyl]phenyl}methylimidazole-5-carboxamide (Compound No. 5-99)

### 75(a) 2-Butyl-4-pivaloyl-1-{4-[2-(trityltetrazol-5-yl)phenyl]phenyl}methylimidazole-5-carbonitrile

Following a procedure similar to that described in Example 74(a), but using 2.04 g of 2-butyl-4-pivaloylimidazole-5-carbonitrile (prepared as described in Preparation 40), 5.6 g of 4-[2-(trityltetrazol-5-yl)-phenyl]benzyl bromide and 1.06 g of potassium t-butoxide, 5.43 g of the title compound were obtained as crystals, melting at 103 - 105°C.

Nuclear Magnetic Resonance Spectrum (CDCl₃) δ ppm:
0.88 (3H, triplet, J = 7.5 Hz);
1.32 (2H, sextet, J = 7.5 Hz);
1.41 (9H, singlet);
1.66 (2H, quintet, J = 7.5 Hz);
2.53 (2H, triplet, J = 7.5 Hz);
5.09 (2H, singlet);
6.91 - 7.50 (22H, multiplet);
7.96 (1H, doublet, J = 7.5 Hz).

### 75(b) 2-Butyl-4-(1-hydroxy-2,2-dimethylpropyl)-1-{4-[2-(trityltetrazol-5-yl)phenyl]phenyl}methylimidazole-5-carbonitrile

Following a procedure similar to that described in Example 74(b), but using 4.03 g of 2-butyl-4-pivaloyl-1-{4-[2-(trityltetrazol-5-yl)phenyl]phenyl}methyl-imidazole-5-carbonitrile [prepared as described in step (a) above] and 0.22 g of sodium borohydride, 3.79 g of the title compound was obtained as crystals, melting at 134 - 135°C.

Nuclear Magnetic Resonance Spectrum (CDCl₃) δ ppm:
0.85 (3H, triplet, J = 7.5 Hz);
0.99 (9H, singlet);
1.27 (2H, sextet, J = 7.5 Hz);
2.52 - 2.67 (2H, multiplet);
2.51 (2H, triplet, J = 7.5 Hz);
2.74 (1H, doublet, J = 7.5 Hz);
4.45 (1H, doublet, J = 7.5 Hz);
5.04 (2H, singlet);
6.85 - 7.53 (22H, multiplet);
7.95 (1H, doublet, J = 7.5 Hz).

### 75(c) 2-Butyl-4-(1-Hydroxy-2,2-dimethylpropyl)-1-{4-[2-(tetrazol-5-yl )phenyl]phenyl}methylimidazole5-carbonitrile

Following a procedure similar to that described in Example 74(c), but using 1.00 g of 2-butyl-4-(1-hydroxy2,2-dimethylpropyl)-1-{4-[2-(trityltetrazol-5-yl)- phenyl]phenyl}methylimidazole-5-carbonitrile [prepared as described in step (b) above] in 75% v/v aqueous acetic acid, 0.65 g of the title compound was obtained as a glass.

Nuclear Magnetic Resonance Spectrum (CDCl₃) δ ppm:
0.91 (3H, triplet, J = 7.5 Hz);
0.96 (9H, singlet);
1.28 - 1.42 (2H, multiplet);
1.58 - 1.74 (2H, multiplet);
2.69 (2H, triplet, J = 7.5 Hz);
4.40 (1H, singlet);
5.21 (2H, singlet);
7.10 - 7.32 (4H, multiplet);
7.43 - 7.65 (3H, multiplet);
8.06 (1H, doublet, J = 8 Hz).

### 75(d) 2-Butyl-4-(1-hydroxy-2,2-dimethylpropyl)-1-{4-[2-(tetrazol-5-yl)phenyl]phenylmethylimidazole-5-carboxamide

Following a procedure similar to that described in Example 74(d), but using 0.34 g of 2-butyl-4-(1-hydroxy2,2-dimethylpropyl)-1-{4-[2-(tetrazol-5-yl)phenyl]-phenyl}methylimidazole-5-carbonitrile [prepared as described in step (c) above] in a 1 N aqueous solution of sodium hydroxide, 0.30 g of the title compound was obtained as a powder, melting at 157 - 160°C.

Nuclear Magnetic Resonance Spectrum (hexadeuterated dimethyl sulphoxide) δ ppm:
0.79 (3H, triplet, J = 7.5 Hz);
0.88 (9H, singlet);
1.16 - 1.30 (2H, multiplet);
1.39 - 1.54 (2H, multiplet);
2.59 (2H, triplet, J = 7.5 Hz);
4.51 (1H, singlet);
5.46 (1H, doublet, J = 16 Hz);
5.73 (1H, doublet, J = 16 Hz);
6.21 (1H, doublet, J = 4.5 Hz);
6.97 (2H, doublet, J = 8.5 Hz);
7.06 (2H, doublet, J = 8.5 Hz);
7.51 - 7.70 (4H, multiplet).

### EXAMPLE 76

### 4-(1-Hydroxy-2-methylpropyl)-2-propyl-1-{4-[2-(tetrazol-5-yl)phenyl]phenyl}methylimidazole-5-carboxamide (Compound No. 5-36)

### 76(a) 4 -Isobutyryl-2-propyl-1-{4-[2-(trityltetrazol-5- yl)phenyl]phenyl}methylimidazole-5-carbonitrile

Following a procedure similar to that described in Example 74(a), but using 0.97 g of 4-isobutyryl-2-propylimidazole-5-carbonitrile (prepared as described in Preparation 39), 2.90 g of 4-[2-(trityltetrazol-5-yl)- phenyl]benzyl bromide and 0.56 g of potassium t-butoxide, 1.90 g of the title compound was obtained as crystals, melting at 133 - 134°C.

Nuclear Magnetic Resonance Spectrum (CDCl₃) δ ppm:
0.91 (3H, triplet, J = 7.5 Hz);
1.22 (6H, doublet, J = 6.5 Hz);
1.69 (2H, sextet, J = 7.5 Hz);
2.54 (2H, triplet, J = 7.5 Hz);
3.64 (1H, quintet, J = 6.5 Hz);
5.12 (2H, singlet);
6.7 - 8.0 (23H, multiplet).

### 76(b) 4-(1-Hydroxy-2-methylpropyl)-2-propyl-1-{4-[2-(trityltetrazol-5-yl)phenyl]phenyl}methyl-imidazole-5-carbonitrile

Following a procedure similar to that described in Example 74(b), but using 1.60 g of 4-isobutyryl-2-propyl-1-{4-[2-(trityltetrazol-5-yl)phenyl]phenyl}-methylimidazole-5-carbonitrile [prepared as described in step (a) above] and 0.13 g of sodium borohydride, 1.50 g of the title compound was obtained as crystals, melting at 154 - 155°C.

Nuclear Magnetic Resonance Spectrum (CDCl₃) δ ppm:
0.87 (3H, triplet, J = 7.5 Hz);
0.94 (3H, doublet, J = 6.5 Hz);
1.00 (3H, doublet, J = 6.5 Hz);
1.66 (2H, sextet, J = 7.5 Hz);
2.12 (1H, sextet, J = 6.5 Hz);
2.50 (2H, triplet, J = 7.5 Hz);
4.54 (1H, doublet, J = 6 Hz);
5.04 (2H, singlet);
6.85 - 6.95 (6H, multiplet);
7.14 (2H, doublet, J = 8.5 Hz);
7.23 - 7.53 (14H, multiplet);
7.94 (1H, doublet, J = 7.5 Hz).

### 76(c) 4-(1-Hydroxy-2-methylpropyl)-2-propyl-1-{4-[2-(tetrazol-5-yl )phenyl]phenyl}methylimidazole5-carbonitrile

Following a procedure similar to that described in Example 74(c), but using 1.36 g of 4-(1-hydroxy-2-methylpropyl)-2-propyl-1-{4-[2-(trityltetrazol-5-yl)phenyl]phenyl}methylimidazole-5-carbonitrile [prepared as described in step (b) above] in 75% v/v aqueous acetic acid, 0.87 g of the title compound was obtained as a glass.

Nuclear Magnetic Resonance Spectrum (CDCl₃) δ ppm:
0.77 (3H, doublet, J = 6.5 Hz);
0.81 (3H, triplet, J = 7.5 Hz);
0.93 (3H, doublet, J = 6.5 Hz);
1.54 (2H, sextet, J = 7.5 Hz);
1.92 - 2.07 (1H, multiplet);
2.55 (2H, triplet, J = 7.5 Hz);
4.33 (1H, doublet, J = 7.5 Hz);
5.12 (2H, singlet);
6.96 - 6.99 (4H, multiplet);
7.35 - 7.69 (3H, multiplet);
7.71 (1H, doublet, J = 7.5 Hz).

### 76(d) 4-(1-Hydroxy-2-methylpropyl)-2-propyl-1-{4-[2-(tetrazol-5-yl)phenyl]phenyl}methylimidazole5-carboxamide

Following a procedure similar to that described in Example 74(d), but using 0.90 g of 4-(1-hydroxy-2-methylpropyl)-2-propyl-1-{4-[2-(tetrazol-5-yl)-phenyl]phenyl}methylimidazole-5-carbonitrile [prepared as described in step (c) above] in a 1 N aqueous solution of sodium hydroxide, 0.64 g of the title compound was obtained as a powder, melting at 153 - 157°C.

Nuclear Magnetic Resonance Spectrum (hexadeuterated dimethyl sulphoxide) δ ppm:
0.69 (3H, doublet, J = 6.5 Hz);
0.81 (3H, triplet, J = 6.5 Hz);
0.99 (3H, triplet, J = 6.5 Hz);
1.49 (2H, sextet, J = 7.5 Hz);
2.05 (1H, quintet, J= 6.5 Hz);
2.68 (2H, triplet, J = 7.5 Hz);
4.45 (1H, doublet, J = 7.5 Hz);
5.55 (1H, doublet, J = 16.5 Hz);
5.70 (1H, doublet, J = 16.5 Hz);
7.02 (2H, doublet, J = 8.5 Hz);
7.08 (2H, doublet, J = 8.5 Hz);
7.51 - 7.71 (4H, multiplet);

### EXAMPLE 77

### 2-Butyl-4-(1-hydroxy-2-methylpropyl)-1-{4-[2-(tetrazol-5-yl)phenyl]phenyl}methylimidazole-5-carboxamide (Compound No. 5-98)

### 77(a) 2-Butyl-4-isobutyryl-1-{4-[2-(trityltetrazol-5-yl)phenyl]phenyl}methylimidazole-5-carbonitrile

Following a procedure similar to that described in Example 74(a), but using 1.42 g of 2-butyl-4-isobutyrylimidazole-5-carbonitrile (prepared as described in Preparation 27), 4.49 g of 4-[2-(trityltetrazol-5-yl)- phenyl]benzyl bromide and 0.76 g of potassium t-butoxide, 3.04 g of the title compound was obtained as crystals, melting at 115 - 116°C.

Nuclear Magnetic Resonance Spectrum (CDCl₃) δ ppm:
0.87 (3H, triplet, J = 7.5 Hz);
1.22 (6H, doublet, J= 6.5 Hz);
1.31 (2H, sextet, J= 7.5 Hz);
1.63 (2H, quintet, J = 7.5 Hz);
2.57 (2H, triplet, J = 7.5 Hz);
3.64 (1H, septet, J= 7.5 Hz);
5.11 (2H, singlet);
6.90 - 7.52 (22H, multiplet);
7.96 (1H, doublet, J = 9 Hz).

### 77(b) 2-Butyl-4-(1-hydroxy-2-methylpropyl)-1-{4-[2-(trityltetrazol-5-yl)phenyl]phenyl}methyl-imidazole-5-carbonitrile

Following a procedure similar to that described in Example 74(b), but using 2.00 g of 2-butyl-4-isobutyryll-{4-[2-(trityltetrazol-5-yl)phenyl]phenyl}methylimidazole-5-carbonitrile [prepared as described in step (a) above] and 0.22 g of sodium borohydride, 1.68 g of the title compound was obtained as crystals, melting at 127 - 128°C.

Nuclear Magnetic Resonance Spectrum (CDCl₃) δ ppm:
0.85 (3H, triplet, J = 7.5 Hz);
0.93 (3H, doublet, J = 6.5 Hz);
1.00 (3H, doublet, J = 6.5 Hz);
1.26 (2H, sextet, J = 7.5 Hz);
1.59 (2H, quintet, J = 7.5 Hz);
2.13 (1H, sextet, J = 6.5 Hz);
2.52 (2H, triplet, J = 7.5 Hz);
4.53 (1H, doublet, J = 6 Hz);
5.04 (2H, singlet);
6.85 - 7.52 (22H, multiplet);
7.95 (1H, doublet, J = 9 Hz).

### 77(c) 2-Butyl-4-(1-Hydroxy-2-methylpropyl)-1-{4-[2-(tetrazol-5-yl)phenyl]phenyl}methylimidazole5-carbonitrile

Following a procedure similar to that described in Example 74(c), but using 1.29 g of 2-butyl-4-(1-hydroxy2-methylpropyl)-1-{4-[2-(trityltetrazol-5-yl)phenyl]-phenyl}methylimidazole-5-carbonitrile [prepared as described in step (b) above] in 75% v/v aqueous acetic acid, 0.83 g of the title compound was obtained as a glass.

Nuclear Magnetic Resonance Spectrum (CDCl₃) δ ppm:
0.81 (3H, doublet, J = 6.5 Hz);
0.83 (3H, triplet, J = 7.5 Hz);
0.95 (3H, doublet, J = 6.5 Hz);
1.26 (2H, sextet, J = 7.5 Hz);
1.54 (2H, quintet, J= 7.5 Hz);
1.97 - 2.09 (1H, multiplet);
2.59 (2H, triplet, J = 7.5 Hz);
4.37 (1H, doublet, J = 6.5 Hz);
5.14 (2H, singlet);
6.98 (2H, doublet, J = 8.5 Hz);
7.05 (2H, doublet, J = 8.5 Hz);
7.32 - 7.60 (3H, multiplet);
7.77 (1H, doublet, J = 7.5 Hz).

### 77(d) 2-Butyl-4-(1-hydroxy-2-methylpropyl)-1-{4-[2-(tetrazol-5-yl)phenyl]phenyl}methylimidazole5-carboxamide

Following a procedure similar to that described in Example 74(d), but using 0.34 g of 2-butyl-4-(1-hydroxy2-methylpropyl)-1-{4-[2-(tetrazol-5-yl)phenyl]-phenyl}methylimidazole-5-carbonitrile [prepared as described in step (c) above] in a 1 N aqueous solution of sodium hydroxide, 0.24 g of the title compound was obtained as a powder, melting at 155 - 157°C.

Nuclear Magnetic Resonance Spectrum (hexadeuterated dimethyl sulphoxide) δ ppm:
0.69 (3H, doublet, J = 6.5 Hz);
0.79 (3H, triplet, J = 7.5 Hz);
0.93 (3H, doublet, J = 6.5 Hz);
1.22 (2H, sextet, J = 7.5 Hz);
1.45 (2H, quintet, J= 7.5 Hz);
2.00 - 2.12 (1H, multiplet);
2.65 (2H, triplet, J = 7.5 Hz);
4.41 (1H, doublet, J = 8 Hz);
5.53 (1H, doublet, J = 16 Hz);
5.71 (1H, doublet, J = 16 Hz);
7.00 (2H, doublet, J = 8.5 Hz);
7.07 (2H, doublet, J = 8.5 Hz);
7.50 - 7.71 (4H, multiplet).

### PREPARATION 1

### 2-Butylimidazole-4,5-dicarbonitrile

A suspension of 51.4 g of diaminomaleonitrile and 85.6 g of trimethyl orthovalerate in 300 ml of acetonitrile was stirred in an oil bath kept at 85°C for 6 hours. At the end of this time, the reaction mixture was concentrated by evaporation under reduced pressure, and the concentrate was purified by short column chromatography through silica gel, using a 1 : 1 by volume mixture of ethyl acetate and hexane as the eluent, to give 99 g of 1-amino-2-N-(1-methoxypentylidene)aminosuccinonitrile. The whole of this compound was dissolved in 300 ml of xylene, and the resulting solution was stirred in an oil bath kept at 150°C for 8 hours, after which the reaction mixture was concentrated to half its original volume and allowed to stand at room temperature. The crystals which precipitated were collected by filtration and washed with a small amount of xylene, to give 55.2 g of the title compound, melting at 109 - 111°C.

### PREPARATION 2

### 2-Butylimidazole-4,5-dicarboxylic acid

A solution of 100 g of 2-butylimidazole-4,5-dicarbonitrile (prepared as described in Preparation 1) in 1 litre of 6 N aqueous hydrochloric acid was heated under reflux for 7 hours, and then the reaction mixture was allowed to stand overnight at room temperature. At the end of this time, the crystals which precipitated were collected by filtration and washed with water and with a small amount of acetone, to give 84 g of the title compound, melting at 261 - 263°C.

### PREPARATION 3

### Diethyl 2-butylimidazole-4,5-dicarboxylate

Dry hydrogen chloride was bubbled through a suspension of 40 g of 2-butylimidazole-4,5-dicarboxylic acid (prepared as described in Preparation 2) in 600 ml of ethanol at room temperature, whilst stirring, for 2 hours to yield a solution. This solution was allowed to stand at room temperature for 18 hours, after which the reaction mixture was concentrated by evaporation under reduced pressure. The concentrate was then mixed with ethyl acetate and with an aqueous solution of sodium hydrogencarbonate and neutralized by adding powdery sodium hydrogencarbonate. The ethyl acetate layer was separated and dried over anhydrous magnesium sulphate, and the solvent was removed by distillation under reduced pressure. The resulting crystalline residue was triturated with a mixture of diisopropyl ether and hexane, and collected by filtration, to give 43 g of the title compound, melting at 82 - 84°C.

### PREPARATION 4

### Dimethyl 2-butylimidazole-4,5-dicarboxylate

A procedure similar to that described in Preparation 3 was repeated, using 40 g of 2-butyl-imidazole-4,5-dicarboxylic acid, and except that methanol was used instead of ethanol, to give 41.6 g of the title compound as crystals, melting at 88°C.

### PREPARATION 5

### 4-Acetyl-2-butyl-5-cyanoimidazole

### 5(i) 2-Butyl-1-tritylimidazole-4,5-dicarbonitrile

1.25 g of sodium hydride (as a 55% w/w dispersion in mineral oil) were added, whilst ice-cooling, to a solution of 5 g of 2-butylimidazole-4,5-dicarbonitrile (prepared as described in Preparation 1) in 50 ml of N,N-dimethylformamide, and the resulting mixture was stirred for 15 minutes. 10 g of trityl chloride were then added, and the reaction mixture was stirred at 50 °C for 6 hours. At the end of this time, it was mixed with ethyl acetate and water, and the product was extracted with ethyl acetate. The extract was dried over anhydrous magnesium sulphate, and the solvent was removed by distillation under reduced pressure. The resulting residue was purified by column chromatography through silica gel, using a 1 : 5 by volume mixture of ethyl acetate and hexane as the eluent, to give 9.83 g of the title compound as a syrup, which solidified on being allowed to stand. The solid melted at 144 - 147°C (with decomposition and colouration at 94 - 98°C).

Nuclear Magnetic Resonance Spectrum (CDCl₃), δ ppm:
0.60 (3H, triplet, J = 7 Hz);
0.5 - 1.4 (4H, multiplet);
2.03 (2H, triplet, J = 7 Hz);
7.0 - 7.6 (15H, multiplet).

### 5 (ii) 4-Acetyl-2-butyl-5-cyano-1-tritylimidazole

11.1 ml of a 2 M solution of methylmagnesium iodide in diethyl ether was slowly added dropwise at room temperature, in an atmosphere of nitrogen, to a solution of 4.5 g of 2-butyl-1-tritylimidazole-4,5-dicarbonitrile [prepared as described in step (i) above] in 45 ml of tetrahydrofuran, and the resulting mixture was stirred at room temperature for 3 hours. At the end of this time, a saturated aqueous solution of ammonium chloride was added dropwise, whilst ice-cooling, to the mixture. The tetrahydofuran layer was separated, washed with a saturated aqueous solution of sodium chloride and concentrated by evaporation under reduced pressure to give a concentrate. The aqueous layer was once again extracted with a small amount of ethyl acetate. The extract was washed with a saturated aqueous solution of sodium chloride, dried and concentrated by evaporation under reduced pressure. The resulting extract was combined with the above concentrate, and the resulting crude product was purified by column chromatography through silica gel, using a 3 : 1 by volume mixture of hexane and ethyl acetate as the eluent, and the product was crystallized from a mixture of ethyl acetate and hexane, to give 1.46 g of the title compound, melting at 159 - 160°C (with decomposition).

Nuclear Magnetic Resonance Spectrum (CDCl₃), δ ppm:
0.60 (3H, triplet, J = 7 Hz);
0.5 - 1.5 (4H, multiplet);
2.08 (2H, triplet, J = 7 Hz);
2.58 (3H, singlet);
7.1 - 7.6 (15H, multiplet).

### 5 (iii) 4-Acetyl-2-butyl-5-cyanoimidazole

A suspension of 1.78 g of 4-acetyl-2-butyl-5-cyanol-tritylimidazole [prepared as described in step (ii) above] in 80% v/v aqueous acetic acid was stirred at 60°C for 1 hour. The solution thus obtained was concentrated to dryness by evaporation under reduced pressure. The resulting residue was purified by column chromatography through silica gel, using a 3 : 1 by volume mixture of hexane and ethyl acetate as the eluent, to give 0.66 g of the title compound as a colourless solid, melting at 77 - 78°C.

Nuclear Magnetic Resonance Spectrum (CDCl₃), δ ppm:
0.93 (3H, triplet, J = 7 Hz);
1.0 - 2.1 (4H, multiplet);
2.72 (3H, singlet);
2.89 (2H, triplet, J = 7 Hz).

### PREPARATION 6

### 4-Benzoyl-2-butyl-5-cyanoimidazole

### 6(i) 4-Benzoyl-2-butyl-5-cyano-1-tritylimidazole

Following a procedure similar to that described in Preparation 5 (ii), 10.3 g of the title compound were obtained as an amorphous solid by reacting a solution of 10 g of 2-butyl-1-tritylimidazole-4,5-dinitrile [prepared as described in Preparation 5(i)] in 100 ml of tetrahydrofuran with 25 ml of a 2 M solution of phenylmagnesium iodide in diethyl ether.

Nuclear Magnetic Resonance Spectrum (CDCl₃), δ ppm:
0.67 (3H, triplet, J = 7 Hz);
0.5 - 1.5 (4H, multiplet);
2.11 (2H, triplet, J = 7 Hz);
7.1 - 8.0 (20H, multiplet).

### 6 (ii) 4-Benzoyl-2-butyl-5-cyanoimidazole

A suspension of 10.3 g of 4-benzoyl-2-butyl-5-cyano-1-tritylimidazole [prepared as described in step (i) above] in 80% v/v aqueous acetic acid was stirred at 60°C for 5 hours. At the end of this time, the solution thus obtained was concentrated by evaporation under reduced pressure, and the concentrate was purified by column chromatography through silica gel, using a 2 : 1 by volume mixture of hexane and ethyl acetate as the eluent. The resulting oily product was dissolved in carbon tetrachloride and the solution was allowed to stand at room temperature, to precipitate crystals, which were collected by filtration to give 4.46 g of the title compound, melting at 121 - 122°C.

Nuclear Magnetic Resonance Spectrum (CDCl₃), δ ppm:
0.90 (3H, triplet, J = 7 Hz) ;
1.0 - 2.3 (4H, multiplet);
2.85 (2H, triplet, J = 7 Hz);
7.2 - 8.0 (5H, multiplet);
11.0 - 12.1 (1H, broad).

### PREPARATION 10

### 2-Propylimidazole-4,5-dicarbonitrile

Following a procedure similar to that described in Preparation 1, but using 16.0 g of diaminomaleonitrile and 24 g of trimethyl orthobutyrate, 18.7 g of the title compound were obtained as crystals, melting at 141 - 144°C.

### PREPARATION 11

### 2-Propylimidazole-4,5-dicarboxylic acid

Following a procedure similar to that described in Preparation 2, but using 18.2 g of 2-propylimidazole-4,5-dicarbonitrile (prepared as described in Preparation 10), 9.95 g of the title compound were obtained as crystals, melting at 261 - 263°C.

### PREPARATION 12

### Diethyl 2-propylimidazole-4,5-dicarboxylate

Following a procedure similar to that described in Preparation 3, but using 10.0 g of 2-propylimidazole-4,5-dicarboxylic acid (prepared as described in Preparation 11), 9.55 g of the title compound were obtained as crystals, melting at 81 - 83°C.

### PREPARATION 14

### 2-Propyl-1-tritylimidazole-4,5-dicarbonitrile

Following a procedure similar to that described in Preparation 5(i), but using 7.8 g of 2-propylimidazole4,5-dicarbonitrile (prepared as described in Preparation 10), 2.14 g of sodium hydride (as a 55% w/w dispersion in mineral oil) and 17.1 g of trityl chloride, 14.6 g of the title compound were obtained as crystals, melting at 107°C (with decomposition and with a yellow colouration at 102°C).

Nuclear Magnetic Resonance Spectrum (CDCl₃), δ ppm:
0.52 (3H, triplet, J = 7 Hz);
1.07 - 1.21 (2H, multiplet);
2.03 (2H, triplet, J = 8 Hz);
7.19 - 7.48 (15H, multiplet).

### PREPARATION 15

### 2-Butyl-5-cyano-4-propionyl-1-tritylimidazole

14 ml of a 3 M solution of ethylmagnesium bromide in diethyl ether were added dropwise at 10°C under an atmosphere of nitrogen to a solution of 8.33 g of 2-butyl-1-tritylimidazole-4,5-dicarbonitrile [prepared as described in Preparation 5(i)] in 83 ml of tetrahydrofuran, and the resulting mixture was stirred at room temperature for 3 hours. At the end of this time, a mixture of a saturated aqueous solution of ammonium chloride and ethyl acetate was added to the reaction mixture, whilst ice-cooling. The ethyl acetate layer was separated, washed with water and dried over anhydrous magnesium sulphate. The solvent was then removed by distillation under reduced pressure. The crystalline product thus obtained was washed with diisopropyl ether, to give 4.56 g of the title compound, melting at 140 - 143°C (softening at 83°C).

Nuclear Magnetic Resonance Spectrum (CDCl₃), δ ppm:
0.61 (3H, triplet, J = 7 Hz);
0.84 - 1.14 (4H, multiplet);
1.18 (3H, triplet, J = 8 Hz);
2.08 (2H, triplet, J = 7 Hz);
3.03 (2H, quartet, J = 7 Hz);
7.22 - 7.42 (15H, multiplet).

### PREPARATION 16

### 5-Cyano-4-propionyl-2-propyl-1-tritylimidazole

Following a procedure similar to that described in Preparation 15, but using 8.05 g of 2-propyl-1-trityl-imidazole-4,5-dicarbonitrile (prepared as described in Preparation 14) and 14 ml of a 3 M solution of ethylmagnesium bromide in diethyl ether, 7.03 g of the title compound were obtained as crystals, melting at 96°C (softening at 87°C).

Nuclear Magnetic Resonance Spectrum (CDCl₃), δ ppm:
0.52 (3H, triplet, J = 7 Hz);
1.05 - 1.3 (2H, multiplet);
1.18 (3H, triplet, J = 7 Hz);
2.05 (2H, triplet, J = 7 Hz);
3.03 (2H, quartet, J = 7 Hz);
7.20 - 7.40 (15H, multiplet).

### PREPARATION 23

### Ethyl 4-(1-hydroxyethyl)-2-propylimidazole-5-carboxylate

### 23(i) 4-Acetyl-2-propylimidazole-5-carbonitrile

194 ml of a 1 M solution of methylmagnesium bromide in tetrahydrofuran were added dropwise at a temperature of 10°C to 15°C and under an atmosphere of nitrogen to a solution of 10 g of 2-propylimidazole-4,5-dicarbonitrile (prepared as described in Preparation 10) in 100 ml of tetrahydrofuran, and the resulting mixture was stirred at a temperature of 10°C to 15°C for 30 minutes. The reaction mixture was then cooled, and 200 ml of ethyl acetate and 100 ml of a saturated aqueous solution of ammonium chloride were added to it. The mixture was then acidified by adding an aqueous solution of potassium bisulphate. The organic layer was separated and dried over anhydrous magnesium sulphate, and the solvent was removed by distillation under reduced pressure. The residue was subjected to column chromatography through silica gel, using a 1 : 1 by volume mixture of ethyl acetate and hexane as the eluent, to give 9.18 g of the title compound as crystals, melting at 93 - 95°C.

Nuclear Magnetic Resonance Spectrum (CDCl₃), δ ppm:
0.99 (3H, triplet, J = 7.5 Hz);
1.83 (2H, sextet, 7.5 Hz);
2.71 (3H, singlet);
2.82 (2H, triplet, J = 8 Hz).

### 23(ii) Ethyl 4-acetyl-2-propylimidazole-5-carboxylate

A mixture of 4.0 g of 4-acetyl-2-propylimidazole-5- carbonitrile [prepared as described in step (i) above] and 60 ml of 6 N aqueous hydrochloric acid was heated under reflux, with stirring, for 8 hours. The reaction mixture was then concentrated by evaporation under reduced pressure, and the concentrate was dissolved in ethanol, after which it was again concentrated in the same way. The residue was dissolved in ethanol and the solvent was again distilled off. After this sequence of dissolution and concentration had been carried out for a total of five times, the residue was dissolved in 60 ml of ethanol. A stream of hydrogen chloride was bubbled through the resulting solution at room temperature for 20 minutes, and then the solution was allowed to stand at room temperature for 16 hours. It was then concentrated by evaporation under reduced pressure. The concentrate was dissolved in a mixture of ethyl acetate and an aqueous solution of sodium hydrogencarbonate, and the solution was neutralized by adding sodium hydrogencarbonate. The ethyl acetate layer was separated and dried over anhydrous magnesium sulphate. The solvent was removed by distillation under reduced pressure, and the resulting residue was purified by column chromatography through silica gel, using a 1 : 1 by volume mixture of ethyl acetate and hexane as the eluent, to give 3.07 g of the title compound as crystals, melting at 76 - 78°C.

Nuclear Magnetic Resonance Spectrum (CDCl₃), δ ppm:
0.96 (3H, triplet, J = 7.5 Hz);
1.39 (3H, triplet, J = 7 Hz);
1.82 (2H, sextet, J = 7.5 Hz);
2.75 (3H, singlet);
2.80 (2H, triplet, J = 7.5 Hz);
4.44 (2H, quartet, J = 7 Hz).

### 23 (iii) Ethyl 4- (1-hydroxyethyl) -2-propylimidazole-5-carboxylate

125 mg of sodium borohydride were added to a solution of 1.50 g of ethyl 4-acetyl-2-propylimidazole5-carboxylate [prepared as described in step (ii) above] in 15 ml of ethanol, and the resulting mixture was stirred at room temperature for 30 minutes. 2 ml of acetone were added, and the mixture was stirred for a further 10 minutes. The reaction mixture was then concentrated by evaporation under reduced pressure, and the concentrate was dissolved in methanol. The solution was again concentrated by evaporation under reduced pressure, and the residue was purified by column chromatography through silica gel, using 1 : 20 and 1 : 10 by volume mixtures of methylene chloride and methanol as the eluent, to give 1.32 g of the title compound as crystals, melting at 151 - 153°C.

Nuclear Magnetic Resonance Spectrum (CDCl₃ + hexadeuterated dimethyl sulphoxide), δ ppm:
0.95 (3H, triplet, J = 7.5 Hz);
1.38 (3H, triplet, J = 7 Hz);
1.48 (3H, doublet, J = 6.5 Hz);
1.74 (2H, sextet, J = 7.5 Hz);
2.67 (2H, triplet, J = 8 Hz);
4.34 (2H, quartet, J = 7 Hz);
5.28 (1H, quartet, J = 6.5 Hz).

### PREPARATION 24

### Ethyl 2-butyl-4-(1-hydroxyethyl)imidazole-5-carboxylate

### 24 (i) 4-Acetyl-2-butylimidazole-5-carbonitrile

Following a procedure similar to that described in Preparation 23(i), but using 10 g of 2-butylimidazole-4,5-dicarbonitrile (prepared as described in Preparation 1), 9.15 g of the title compound were obtained as crystals, melting at 77 - 78°C.

Nuclear Magnetic Resonance Spectrum (CDCl₃), δ ppm:
0.93 (3H, triplet, J = 7 Hz);
1.0 - 2.1 (4H, multiplet);
2.72 (3H, singlet);
2.89 (2H, triplet, J = 7 Hz).

### 24 (ii) Ethyl 4-acetyl-2-butylimidazole-5-carboxylate

Following a procedure similar to that described in Preparation 23(ii), but using 1.00 g of 4-acetyl-2-butylimidazole-5-carbonitrile [prepared as described in step (i) above], 0.92 g of the title compound was obtained as a viscous oil.

Nuclear Magnetic Resonance Spectrum (CDCl₃), δ ppm:
0.88 (3H, triplet, J = 7 Hz);
1.1 - 2.1 (4H, multiplet);
1.33 (3H, triplet, J = 7 Hz);
2.74 (3H, singlet);
2.82 (2H, triplet, J = 7.5 Hz);
4.38 (2H, quartet, J = 7 Hz).

### 24 (iii) Ethyl 2-butyl-4-(1-hydroxyethyl)imidazole-5-carboxylate

Following a procedure similar to that described in Preparation 23 (iii), but using 0.64 g of ethyl 4-acetyl2-butylimidazole-5-carboxylate [prepared as described in step (ii) above], 0.55 g of the title compound was obtained as crystals, melting at 149°C.

Nuclear Magnetic Resonance Spectrum (CDCl₃ + hexadeuterated dimethyl sulphoxide), δ ppm:
0.91 (3H, triplet, J = 7.5 Hz);
1.37 (3H, triplet, J = 7 Hz);
1.3 - 1.42 (2H, multiplet);
1.50 (3H, doublet, J = 6.5 Hz);
1.69 (2H, quintet, J = 7.5 Hz);
2.69 (2H, triplet, J = 8 Hz);
4.34 (2H, quartet, J = 7 Hz);
5.26 (1H, quartet, J = 6.5 Hz).

### PREPARATION 25

### 2-Butyl-4-propionylimidazole-5-carbonitrile

Following a procedure similar to that described in Preparation 24 (i), but using ethylmagnesium bromide instead of methylmagnesium bromide, the title compound, melting at 84 - 85°C, was obtained in a 51.9% yield.

Nuclear Magnetic Resonance Spectrum (CDCl₃), δ ppm:
0.95 (3H, triplet, J = 7 Hz);
1.0 - 2.2 (4H, multiplet);
1.28 (3H, triplet, J = 7.0 Hz);
2.88 (2H, triplet, J = 7 Hz);
3.15 (2H, quartet, J = 7 Hz).

### PREPARATION 26

### 2-Butyl-4-butyrylimidazole-5-carbonitrile

Following a procedure similar to that described in Preparation 24(i), but using propylmagnesium bromide instead of methylmagnesium bromide, the title compound, melting at 91 - 92°C, was obtained in a 57.2% yield.

Nuclear Magnetic Resonance Spectrum (CDCl₃), δ ppm:
1.02 (3H, triplet, J = 7.5 Hz);
1.11 (3H, triplet, J = 7.5 Hz);
1.3 - 1.6 (2H, multiplet);
1.7 - 2.0 (4H, multiplet);
2.88 (2H, triplet, J = 8 Hz);
3.13 (2H, triplet, J = 7.5 Hz).

### PREPARATION 27

### 2-Butyl-4-isobutyrylimidazole-5-carbonitrile

Following a procedure similar to that described in Preparation 24 (i), but using isopropylmagnesium bromide instead of methylmagnesium bromide, the title compound, melting at 88 - 89°C, was obtained in a 36.2% yield.

Nuclear Magnetic Resonance Spectrum (CDCl₃), δ ppm:
0.94 (3H, triplet, J = 7 Hz);
1.0 - 2.1 (4H, multiplet);
1.30 (6H, doublet, J = 7 Hz);
2.91 (2H, triplet, J = 7 Hz);
3.71 (1H, septet, J = 7 Hz).

### PREPARATION 28

### 4-Butyryl-2-propylimidazole-5-carbonitrile

Following a procedure similar to that described in Preparation 24(i), but using 2-propylimidazole-4,5-dicarbonitrile (prepared as described in Preparation 10) and propylmagnesium bromide, the title compound, melting at 94 - 95°C, was obtained in a 49.8% yield.

Nuclear Magnetic Resonance Spectrum (CDCl₃), δ ppm:
1.00 (3H, triplet, J = 7.5 Hz);
1.04 (3H, triplet, J = 7.5 Hz);
1.7 - 1.9 (4H, multiplet);
2.79 (2H, triplet, J = 7.5 Hz);
3.06 (2H, triplet, J = 7.5 Hz).

### PREPARATION 34

### 2-Ethylimidazole-4,5-dicarbonitrile

Following a procedure similar to that described in Preparation 1, but using 53.3 g of diaminomaleonitrile and 91.3 g of triethyl orthopropionate, 59.5 g of the title compound were obtained as crystals, melting at 179 - 181°C.

### PREPARATION 35

### 2-Ethylimidazole-4,5-dicarboxylic acid

Following a procedure similar to that described in Preparation 2, but using 45.0 g of 2-ethylimidazole-4,5-dicarbonitrile (prepared as described in Preparation 34), 31.2 g of the title compound were obtained as crystals, melting at 265 - 268°C.

### PREPARATION 36

### Diethyl 2-ethylimidazole-4,5-dicarboxylate

Following a procedure similar to that described in Preparation 3, but using 35.0 g of 2-ethylimidazole-4,5-dicarboxylic acid (prepared as described in Preparation 35), 38.7 g of the title compound were obtained as crystals, melting at 83 - 84°C.

### PREPARATION 38

### N-t-Butyl-4'-bromomethylbiphenyl-2-carboxamide

### 38 (i) N-t-Butyl-4'-methylbiphenyl-2-carboxamide

5.7 ml of oxalyl chloride were added dropwise, whilst ice-cooling, to a solution of 6.91 g of 4'-methylbiphenyl-2-carboxylic acid in 70 ml of methylene chloride, and the mixture was stirred at room temperature for 2 hours. The mixture was then concentrated by evaporation under reduced pressure, and the residue was dissolved in 70 ml of tetrahydrofuran. A solution of 7.5 ml of t-butylamine in 50 ml of tetrahydrofuran was added dropwise, whilst ice-cooling to the solution, and the mixture was stirred at room temperature for 10 minutes. At the end of this time, the reaction mixture was diluted with water and ethyl acetate. The ethyl acetate layer was separated, washed with aqueous sodium hydrogencarbonate and then with aqueous sodium chloride, dried over anhydrous sodium sulphate and concentrated by evaporation under reduced pressure, to give 7.48 g of the title compound as crystals, melting at 105 - 106.5°C (after recrystallization from ethyl acetate and hexane).

Nuclear Magnetic Resonance Spectrum (CDCl₃) δ ppm:
1.12 (9H, singlet);
2.41 (3H, singlet);
5.04 (1H, broad singlet);
7.2 - 7.5 (7H, multiplet);
7.71 (1H, doublet, J = 8 Hz).

### 38 (ii) N-t-Butyl-4'-bromomethylbiphenyl-2-carboxamide

4.39 g of N-bromosuccinimide and 50 mg of benzoyl peroxide were added to a solution of 6.00 g of N-t-butyl-4'-methylbiphenyl-2-carboxamide [prepared as described in Preparation 38(i)] in 90 ml of carbon tetrachloride, and the mixture was heated under reflux for 4 hours. At the end of this time, the reaction mixture was cooled to room temperature, washed with water, dried over anhydrous magnesium sulphate and concentrated by evaporation under reduced pressure. The residue was purified by column chromatography through silica gel, using a 1 : 4 by volume mixture of ethyl acetate and hexane as the eluent, to give 7.04 g of the title compound as crystals, melting at 124 - 126°C.

Nuclear Magnetic Resonance Spectrum (CDCl₃) δ ppm:
1.14 (9H, singlet);
4.55 (2H, singlet);
4.99 (1H, broad singlet);
7.30 - 7.72 (8H, multiplet).

### PREPARATION 39

### 4-Isobutyryl-2-propylimidazole-5-carbonitrile

Following a procedure similar to that described in Preparation 23(i), but using 8.24 g of 2-propyl-imidazole-4,5-dicarbonitrile (prepared as described in Preparation 10) and 103 ml of a 2 M solution of isopropylmagnesium iodide in diethyl ether, 45.0 g of the title compound were obtained as crystals, melting at 90.5 - 91°C.

Nuclear Magnetic Resonance Spectrum (CDCl₃) δ ppm:
1.01 (3H, triplet, J = 7.5 Hz);
1.29 (6H, doublet, J = 6.5 Hz);
1.82 (2H, sextet, J = 7.5 Hz);
2.81 (2H, triplet, J = 7.5 Hz);
3.66 (1H, septet, J = 6.5 Hz).

### PREPARATION 40

### 2-Butyl-4-pivaloylimidazole-5-carbonitrile

A solution of 10.4 g of 2-butylimidazole-4,5-dicarbonitrile (prepared as described in Preparation 1) in 150 ml of methylene chloride was added dropwise in an atmosphere of nitrogen at 10 - 15°C to 100 ml of a 2 M solution of t-butylmagnesium chloride in diethyl ether, and the mixture was stirred at the same temperature for 1 hour. 200 ml of ethyl acetate and 100 ml of aqueous potassium hydrogensulphate were then added dropwise to the reaction mixture, and the mixture was stirred at room temperature for 20 minutes. At end of this time, insoluble materials were removed by filtration, and the organic layer was separated, dried over anhydrous magnesium sulphate and concentrated by evaporation under reduced pressure. The residue was purified by column chromatography through silica gel, using a 1 : 3 by volume mixture of ethyl acetate and hexane as the eluent, to give 7.95 g of the title compound as crystals, melting at 135 - 137°C.

Nuclear Magnetic Resonance Spectrum (CDCl₃) δ ppm:
0.95 (3H, triplet, J = 7.5 Hz);
1.42 (2H, septet, J = 7.5 Hz);
1.46 (9H, singlet);
1.75 (2H, quintet, J = 7.5 Hz);
2.79 (2H, triplet, J = 7.5 Hz).

### PREPARATION 41

### 2-Propyl-4-pivaloylimidazole-5-carbonitrile

Following a procedure similar to that described in Preparation 40, but using 3.2 g of 2-propylimidazole-4,5-dicarbonitrile (prepared as described in Preparation 10) and 33 ml of a 2 M solution of t-butylmagnesium chloride in diethyl ether, 2.35 g of the title compound were obtained as crystals, melting at 176 - 178°C.

Nuclear Magnetic Resonance Spectrum (CDCl₃) δ ppm:
0.93 (3H, triplet, J = 7.5 Hz);
1.36 (9H, singlet);
1.75 (2H, sextet, J = 7.5 Hz);
2.68 (2H, triplet, J = 7.5 Hz).

## Claims (Claims for the following Contracting State(s): GB, DE, FR, IT, CH, DK, BE, NL, SE, LU, AT, MC, PT)

1. A compound of formula (I): 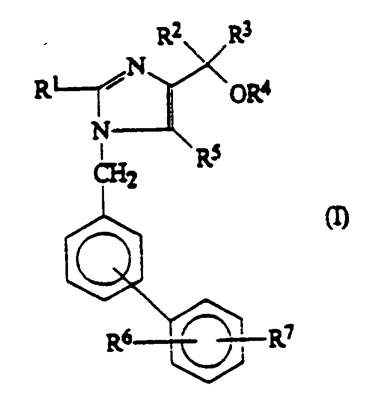 in which:
R¹ represents an alkyl group having from 2 to 5 carbon atoms;
R² and R³ each represent a hydrogen atom or one of R² and R³ represents a hydrogen atom, and the other represents an alkyl group having from 1 to 4 carbon atoms;
R⁴ represents a hydrogen atom, a methyl group, an ethyl group or an alkanoyl group having from 1 to 5 carbon atoms;
R⁵ represents a group of formula -COOR^{5a} or a group of formula -CONR⁸R⁹, in which:
R^{5a} represents
a hydrogen atom,
a methyl, ethyl or benzyl group,
an alkanoyloxymethyl group, in which the alkanoyl part has from 1 to 5 carbon atoms,
a 1-(alkanoyloxy)ethyl group, in which the alkanoyl part has from 1 to 5 carbon atoms,
an alkoxycarbonyloxymethyl group, in which the alkoxy part has from 1 to 4 carbon atoms,
a 1-(alkoxycarbonyloxy)ethyl group, in which the alkoxy part has from 1 to 4 carbon atoms,
a cycloalkanoyloxyalkyl group in which the cycloalkyl part has 5 or 6 carbon atoms and the alkyl part has 1 or 2 carbon atoms,
a cycloalkoxycarbonyloxyalkyl group in which the cycloalkyl part has 5 or 6 carbon atoms and the alkyl part has 1 or 2 carbon atoms,
a [5-(phenyl- or methyl-)-2-oxo-1,3-dioxolen-4-yl]methyl group, or
a phthalidyl group;
R⁸ and R⁹ are the same or different and each represents a hydrogen atom, a methyl group, an ethyl group, a methoxycarbonylmethyl group, an ethoxy carbonylmethyl group or a carboxymethyl group; or R⁸ and R⁹ together represent a tetramethylene, pentamethylene, 1-carboxytetramethylene or 1-carboxypentamethylene group;
R⁶ represents a hydrogen atom, or it represents a methyl group, an methoxy group, a fluorine atom or a chlorine atom at the 6-position of the benzene ring;
R⁷ represents a carboxy group or a tetrazol-5-yl group at the 2-position of the benzene ring; and
the benzene ring which bears the substituents represented by R⁶ and R⁷ is at the 4-position of the benzyl group to which it is attached;
and pharmaceutically acceptable salts and esters thereof.

2. A compound according to Claim 1, in which:
R¹ represents an ethyl, propyl or butyl group;
R² represents a hydrogen atom or a methyl group;
R³ represents a hydrogen atom;
R⁴ represents a hydrogen atom or a methyl group;
R⁵ represents a group of formula -COOR^{5a}, in which R^{5a} represents a hydrogen atom, a pivaloyloxymethyl group, an ethoxycarbonyloxymethyl group, a 1-(ethoxy-carbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group, or a phthalidyl group;
R⁶ represents a hydrogen atom;
R⁷ represents a carboxy group or a tetrazol-5-yl group at the 2-position of the benzene ring; and
the benzene ring which bears the substituents represented by R⁶ and R⁷ is at the 4-position of the benzyl group to which it is attached;
and pharmaceutically acceptable salts and esters thereof.

3. A compound according to Claim 1, in which:
R¹ represents an ethyl, propyl or butyl group;
R² represents a isopropyl group or a t-butyl group;
R³ represents a hydrogen atom;
R⁴ represents a hydrogen atom or a methyl group;
R⁵ represents a group of formula -CONR⁸R⁹, in which R⁸ and R⁹ are the same or different and each represents a hydrogen atom, a methyl group, a methoxycarbonylmethyl group, an ethoxycarbonylmethyl group or a carboxymethyl group;
R⁶ represents a hydrogen atom;
R⁷ represents a carboxy group or a tetrazol-5-yl group at the 2-position of the benzene ring; and
the benzene ring which bears the substituents represented by R⁶ and R⁷ is at the 4-position of the benzyl group to which it is attached;
and pharmaceutically acceptable salts and esters thereof.

4. 4-(1-hydroxyethyl)-2-propyl-1-{4-[2-(tetrazol-5-yl)-phenyl]phenyl}methylimidazole-5-carboxylic acid;
pivaloyloxymethyl 4-(1-hydroxyethyl)-2-propyl-1-{4-[2-(tetrazol-5-yl)phenyl]phenyl}methylimidazole-5-carboxylate;
(5-methyl-2-oxo-1, 3-dioxolen-4-yl)methyl 4-(1-hydroxy-ethyl)-2-propyl-1-{4-[2-(tetrazol-5-yl)phenyl]-phenyl}methylimidazole-5-carboxylate;
and pharmaceutically acceptable salts thereof.

5. 4-(1-hydroxyethyl)-2-butyl-1-{4-[2-(tetrazol-5-yl)- phenyl]phenyl}methylimidazole-5-carboxylic acid;
pivaloyloxymethyl 4-(1-hydroxyethyl)-2-butyl-1-{4-[2-(tetrazol-5-yl)phenyl]phenyl}methylimidazole-5-carboxylate;
(5-methyl-2-oxo-1, 3-dioxolen-4-yl)methyl 4-(1-hydroxy-ethyl)-2-butyl-1-{4- [2-(tetrazol-5-yl)phenyl]-phenyl}methylimidazole-5-carboxylate;
and pharmaceutically acceptable salts thereof.

6. 4-(1-hydroxymethyl)-2-propyl-1-{4-[2-(tetrazol-5-yl)- phenyl]phenyl}methylimidazole-5-carboxylic acid;
pivaloyloxymethyl 4-(1-hydroxymethyl)-2-propyl-1-{4-[2-(tetrazol-5-yl)phenyl]phenyl}methylimidazole-5-carboxylate;
(5-methyl-2-oxo-1,3-dioxolen-4-yl)methyl 4-(1-hydroxy-methyl)-2-propyl-1-{4-[2-(tetrazol-5-yl)phenyl]-phenyl}methylimidazole-5-carboxylate;
and pharmaceutically acceptable salts thereof.

7. 4-(1-hydroxymethyl)-2-butyl-1-{4-[2-(tetrazol-5-yl)-phenyl]phenyl}methylimidazole-5-carboxylic acid;
pivaloyloxymethyl 4-(1-hydroxymethyl)-2-butyl-1-{4-[2-(tetrazol-5-yl)phenyl]phenyl}methylimidazole-5-carboxylate;
(5-methyl-2-oxo-1,3-dioxolen-4-yl)methyl 4-(1-hydroxy-methyl) -2-butyl-1-{4-[2-(tetrazol-5-yl)phenyl]-phenyl}methylimidazole-5-carboxylate;
and pharmaceutically acceptable salts thereof.

8. A pharmaceutical composition for the treatment or prophylaxis of hypertension, which comprises an anti-hypertensive agent in admixture with a pharmaceutically acceptable carrier or diluent, in which the anti-hypertensive agent is at least one compound of formula (I) or a pharmaceutically acceptable salt or ester thereof, as claimed in any one of Claims 1 to 7.

9. Compounds of formula (I) and pharmaceutically acceptable salts and esters thereof, as claimed in any one of Claims 1 to 7, for use in therapy.

10. The use of compounds of formula (I) and pharmaceutically acceptable salts and esters thereof, as claimed in any one of Claims 1 to 7, for the manufacture of a medicament for the treatment or prophylaxis of hypertension.

11. A process for preparing a compound according to any one of Claims 1 to 7, which comprises the steps:
reacting a compound of formula (II): 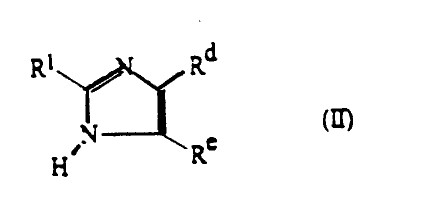 [in which:
R¹ is as defined above and R^{d} represents a group of formula 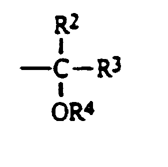 wherein R², R³ and R⁴ are as defined in Claim 1,
or R^{d} represents a group of formula -COOR^{f} wherein R^{f} represents a carboxy-protecting group, R^{d} represents a group of formula -COR², wherein R² is as defined above, or R^{d} represents a cyano group; and
R^{e} represents a cyano group, a carboxy group or a group of formula -COOR^{f} wherein R^{f} is as defined above;
with a compound of formula (III): 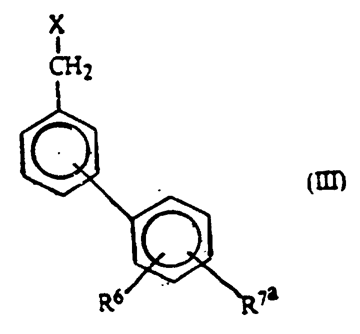 in which: R⁶ is as defined above; R^{7a} represents a protected carboxy group, a cyano group, a protected tetrazol-5-yl group, a carbamoyl group or an alkylcarbamoyl group; and X represents a halogen atom;
to give a compound of formula (IV): 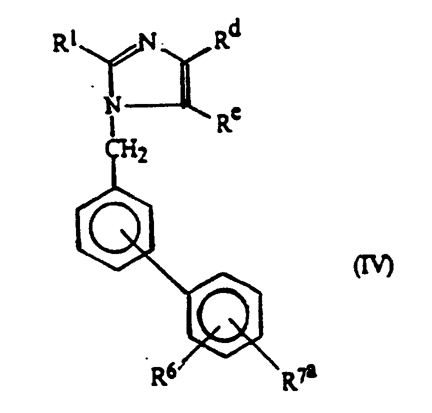 wherein R^{d}, R^{e}, R¹, R⁶ and R^{7a} are as defined above; and
in any order, removing protecting groups, and, if necessary, converting said group R^{d} to a group of formula 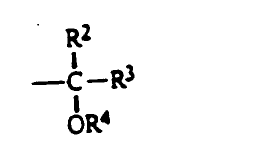 wherein R², R³ and R⁴ are as defined above,
and, if necessary, converting said group R^{e} to a group R⁵, converting said group R^{7a} to a group R⁷, or alkylating or acylating a hydroxy group in R⁴, to give a compound of formula (I); and
optionally salifying or esterifying the product.

## Claims (Claims for the following Contracting State(s): ES, GR)

1. A process for preparing a compound of formula (I): 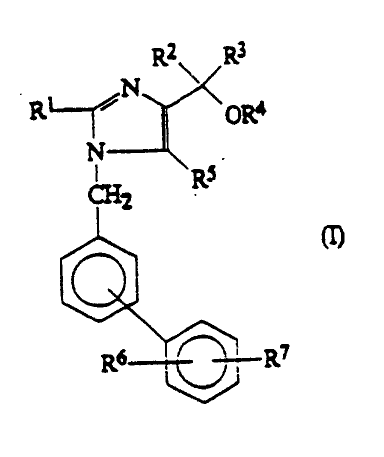 in which:
R¹ represents an alkyl group having from 2 to 5 carbon atoms;
R² and R³ each represent a hydrogen atom or one of R² and R³ represents a hydrogen atom and the other represents an alkyl group having from 1 to 4 carbon atoms;
R⁴ represents a hydrogen atom, a methyl group, an ethyl group or an alkanoyl group having from 1 to 5 carbon atoms;
R⁵ represents a group of formula -COOR^{5a} or a group of formula -CONR⁸R⁹, in which:
R^{5a} represents
a hydrogen atom,
a methyl, ethyl or benzyl group,
an alkanoyloxymethyl group, in which the alkanoyl part has from 1 to 5 carbon atoms,
a 1-(alkanoyloxy)ethyl group, in which the alkanoyl part has from 1 to 5 carbon atoms,
an alkoxycarbonyloxymethyl group, in which the alkoxy part has from 1 to 4 carbon atoms,
a 1-(alkoxycarbonyloxy)ethyl group, in which the alkoxy part has from 1 to 4 carbon atoms,
a cycloalkanoyloxyalkyl group in which the cycloalkyl part has 5 or 6 carbon atoms and the alkyl part has 1 or 2 carbon atoms,
a cycloalkoxycarbonyloxyalkyl group in which the cycloalkyl part has 5 or 6 carbon atoms and the alkyl part has 1 or 2 carbon atoms,
a [5-(phenyl- or methyl-)-2-oxo-1,3-dioxolen-4-yl]methyl group, or
a phthalidyl group;
R⁸ and R⁹ are the same or different and each represents a hydrogen atom, a methyl group, an ethyl group, a methoxycarbonylmethyl group, an ethoxycarbonylmethyl group or a carboxymethyl group; or R⁸ and R⁹ together represent a tetramethylene, pentamethylene, 1-carboxytetramethylene or 1-carboxypentamethylene group;
R⁶ represents a hydrogen atom, or it represents a methyl group, an methoxy group, a fluorine atom or a chlorine atom at the 6-position of the benzene ring;
R⁷ represents a carboxy group or a tetrazol-5-yl group at the 2-position of the benzene ring; and
the benzene ring which bears the substituents represented by R⁶ and R⁷ is at the 4-position of the benzyl group to which it is attached;
and pharmaceutically acceptable salts and esters thereof;
which process comprises the steps:
reacting a compound of formula (II): 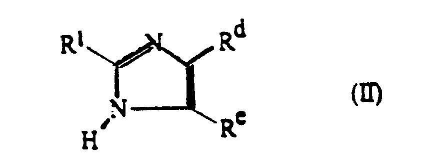 [in which:
R¹ is as defined above and R^{d} represents a group of formula 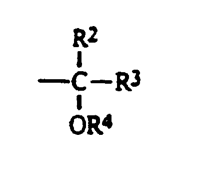 wherein R², R³ and R⁴ are as defined in Claim 1,
or R^{d} represents a group of formula -COOR^{f} wherein R^{f} represents a carboxy-protecting group, R^{d} represents a group of formula -COR², wherein R² is as defined above, or R^{d} represents a cyano group; and
R^{e} represents a cyano group, a carboxy group or a group of formula -COOR^{f} wherein R^{f} is as defined above;
with a compound of formula (III): 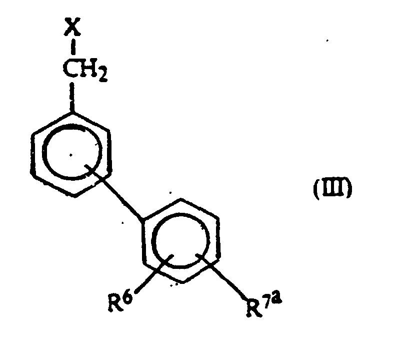 in which: R⁶ is as defined above; R^{7a} represents a protected carboxy group, a cyano group, a protected tetrazol-5-yl group, a carbamoyl group or an alkylcarbamoyl group; and X represents a halogen atom;
to give a compound of formula (IV): 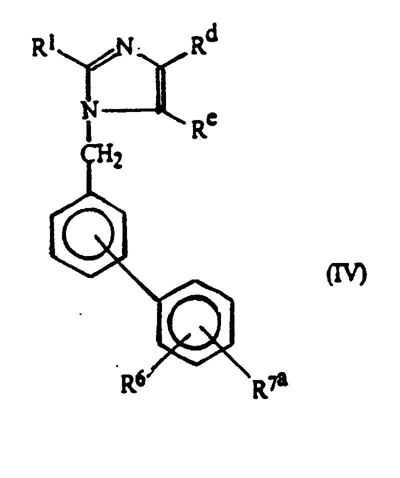 wherein R^{d}, R^{e}, R¹, R⁶ and R^{7a} are as defined above; and
in any order, removing protecting groups, and, if necessary, converting said group R^{d} to a group of formula 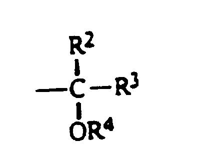 wherein R², R³ and R⁴ are as defined above,
and, if necessary, converting said group R^{e} to a group R⁵, converting said group R^{7a} to a group R⁷, or alkylating or acylating a hydroxy group in R⁴, to give a compound of formula (I); and optionally salifying or esterifying the product.

2. A process according to Claim 1, in which the reagents and reaction conditions are so chosen as to prepare a compound of formula (I) or a salt or ester thereof, in which:
R¹ represents an ethyl, propyl or butyl group;
R² represents a hydrogen atom or a methyl group;
R³ represents a hydrogen atom;
R⁴ represents a hydrogen atom or a methyl group;
R⁵ represents a group of formula -COOR^{5a}, in which R^{5a} represents a hydrogen atom, a pivaloyloxymethyl group, an ethoxycarbonyloxymethyl group, a 1-(ethoxy-carbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group, or a phthalidyl group;
R⁶ represents a hydrogen atom;
R⁷ represents a carboxy group or a tetrazol-5-yl group at the 2-position of the benzene ring; and
the benzene ring which bears the substituents represented by R⁶ and R⁷ is at the 4-position of the benzyl group to which it is attached.

3. A process according to Claim 1, in which the reagents and reaction conditions are so chosen as to prepare a compound of formula (I) or a salt or ester thereof, in which:
R¹ represents an ethyl, propyl or butyl group;
R² represents an isopropyl group or a t-butyl group;
R³ represents a hydrogen atom;
R⁴ represents a hydrogen atom or a methyl group;
R⁵ represents a group of formula -CONR^{B}R⁹, in which R⁸ and R⁹ are the same or different and each represents a hydrogen atom, a methyl group, a methoxycarbonylmethyl group, an ethoxycarbonylmethyl group or a carboxymethyl group;
R⁶ represents a hydrogen atom;
R⁷ represents a carboxy group or a tetrazol-5-yl group at the 2-position of the benzene ring; and
the benzene ring which bears the substituents represented by R⁶ and R⁷ is at the 4-position of the benzyl group to which it is attached.

4. A process according to Claim 1, in which the reagents and reaction conditions are so chosen as to prepare:
4-(1-hydroxyethyl)-2-propyl-1-{4-[2-(tetrazol-5-yl)-phenyl]phenyl}methylimidazole-5-carboxylic acid;
pivaloyloxymethyl 4-(1-hydroxyethyl)-2-propyl-1-{4-[2-(tetrazol-5-yl)phenyl]phenyl}methylimidazole-5-carboxylate;
(5-methyl-2-oxo-1,3-dioxolen-4-yl)methyl 4-(1-hydroxy-ethyl)-2-propyl-1-{4-[2-(tetrazol-5-yl)phenyl]-phenyl}methylimidazole-5-carboxylate;
or a pharmaceutically acceptable salt thereof.

5. A process according to Claim 1, in which the reagents and reaction conditions are so chosen as to prepare:
4-(1-hydroxyethyl)-2-butyl-1-{4-[2-(tetrazol-5-yl)- phenyl]phenyl}methylimidazole-5-carboxylic acid;
pivaloyloxymethyl 4-(1-hydroxyethyl)-2-butyl-1-{4-[2-(tetrazol-5-yl)phenyl]phenyl}methylimidazole-5-carboxylate;
(5-methyl-2-oxo-1,3-dioxolen-4-yl)methyl 4-(1-hydroxyethyl)-2-butyl-1-{4-[2-(tetrazol-5-yl)phenyl]-phenyl}methylimidazole-5-carboxylate;
and pharmaceutically acceptable salts thereof.

6. A process according to Claim 1, in which the reagents and reaction conditions are so chosen as to prepare:
4-(1-hydroxymethyl)-2-propyl-1-{4-[2-(tetrazol-5-yl)- phenyl]phenyl}methylimidazole-5-carboxylic acid;
pivaloyloxymethyl 4-(1-hydroxymethyl)-2-propyl-1-{4-[2-(tetrazol-5-yl)phenyl]phenyl}methylimidazole-5-carboxylate;
(5-methyl-2-oxo-1,3-dioxolen-4-yl)methyl 4-(1-hydroxymethyl)-2-propyl-1-{4- [2- (tetrazol-5-yl)phenyl]-phenyl}methylimidazole-5-carboxylate;
and pharmaceutically acceptable salts thereof.

7. A process according to Claim 1, in which the reagents and reaction conditions are so chosen as to prepare:
4-(1-hydroxymethyl)-2-butyl-1-{4-[2-(tetrazol-5-yl)- phenyl]phenyl}methylimidazole-5-carboxylic acid;
pivaloyloxymethyl 4-(1-hydroxymethyl)-2-butyl-1-{4-[2-(tetrazol-5-yl)phenyl]phenyl}methylimidazole-5-carboxylate;
(5-methyl-2-oxo-1, 3-dioxolen-4-yl)methyl 4-(1-hydroxymethyl)-2-butyl-1-{4-[2-(tetrazol-5-yl)phenyl]-phenyl}methylimidazole-5-carboxylate;
and pharmaceutically acceptable salts thereof.

8. A process for preparing a pharmaceutical composition for the treatment or prophylaxis of hypertension, which comprises mixing an anti-hypertensive agent with a pharmaceutically acceptable carrier or diluent, in which the anti-hypertensive agent is at least one compound of formula (I) or a pharmaceutically acceptable salt or ester thereof, as defined in any one of Claims 1 to 7.

9. The use of compounds of formula (I) and pharmaceutically acceptable salts and esters thereof, as defined in any one of Claims 1 to 7 for the manufacture of a medicament for the treatment or prophylaxis of hypertension.

## Patentansprüche (Patentansprüche für folgende(n) Vertragsstaat(en): GB, DE, FR, IT, CH, DK, BE, NL, SE, LU, AT, MC, PT)

1. Verbindung der Formel (I): 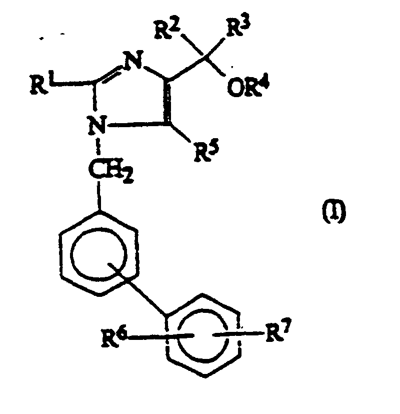 in der
R¹ eine Alkylgruppe mit 2 bis 5 Kohlenstoffatomen darstellt,
R² und R³ jeweils ein Wasserstoffatom bedeuten oder eines von R² und R³ ein Wasserstoffatom darstellt, und das andere eine Alkylgruppe mit 1 bis 4 Kohlenstoffatomen darstellt,
R⁴ ein Wasserstoffatom, eine Methylgruppe, eine Ethylgruppe oder eine Alkanoylgruppe mit 1 bis 5 Kohlenstoffatomen darstellt,
R⁵ eine Gruppe der Formel -COOR^{5a} oder eine Gruppe der Formel -CONR⁸R⁹ darstellt, in der
R^{5a} darstellt:
ein Wasserstoffatom,
eine Methyl-, Ethyl- oder Benzylgruppe,
eine Alkanoyloxymethylgruppe, in der der Alkanoylteil 1 bis 5 Kohlenstoffatome aufweist,
eine 1-(Alkanoyloxy)-ethylgruppe, in der der Alkanoylteil 1 bis 5 Kohlenstoffatome aufweist,
eine Alkoxycarbonyloxymethylgruppe, in der der Alkoxyteil 1 bis 4 Kohlenstoffatome aufweist,
eine 1-(Alkoxycarbonyloxy)-ethylgruppe, in der der Alkoxyteil 1 bis 4 Kohlenstoffatome aufweist,
eine Cycloalkanoyloxyalkylgruppe, in der der Cycloalkylteil 5 oder 6 Kohlenstoffatome hat und der Alkylteil 1 oder 2 Kohlenstoffatome hat,
eine Cycloalkoxycarbonyloxyalkylgruppe, in der der Cycloalkylteil 5 oder 6 Kohlenstoffatome hat und der Alkylteil 1 oder 2 Kohlenstoffatome hat,
eine [5-(Phenyl- oder Methyl-)-2-oxo-1,3-dioxolen-4-yl]-methylgruppe oder eine Phthalidylgruppe,
R⁸ und R⁹ gleich oder verschieden sind und jeweils ein Wasserstoffatom, eine Methylgruppe, eine Ethylgruppe, eine Methoxycarbonylmethylgruppe, eine Ethoxycarbonylmethylgruppe oder eine Carboxymethylgruppe darstellen oder R⁸ und R⁹ zusammen eine Tetramethylen-, Pentamethylen-, 1-Carboxytetramethylen- oder 1-Carboxypentamethylengruppe darstellen,
R⁶ ein Wasserstoffatom darstellt oder eine Methylgruppe, eine Methoxygruppe, ein Fluoratom oder ein Chloratom in 6-Stellung des Benzolrings darstellt,
R⁷ eine Carboxygruppe oder eine Tetrazol-5-yl-Gruppe in 2-Stellung des Benzolrings darstellt, und
der Benzolring, welcher die durch R⁶ und R⁷ dargestellten Substituenten trägt, in 4-Stellung der Benzylgruppe ist, an die er gebunden ist,
und pharmazeutisch geeignete Salze und Ester dieser Verbindung.

2. Verbindung nach Anspruch 1, in der:
R¹ eine Ethyl-, Propyl- oder Butylgruppe darstellt,
R² ein Wasserstoffatom oder eine Methylgruppe darstellt,
R³ ein Wasserstoffatom darstellt,
R⁴ ein Wasserstoffatom oder eine Methylgruppe darstellt,
R⁵ eine Gruppe der Formel -COOR^{5a} bedeutet, in welcher R^{5a} ein Wasserstoffatom, eine Pivaloyloxymethylgruppe, eine Ethoxycarbonyloxymethylgruppe, eine 1-(Ethoxycarbonyloxy)-ethylgruppe, eine Isopropoxycarbonyloxymethylgruppe, eine 1-(Isopropoxycarbonyloxy)-ethylgruppe, eine (5-Methyl-2-oxo-1,3-dioxolen-4-yl)methylgruppe oder eine Phthalidylgruppe darstellt,
R⁶ ein Wasserstoffatom darstellt,
R⁷ eine Carboxygruppe oder eine Tetrazol-5-ylgruppe in 2-Stellung des Benzolrings darstellt und
der Benzolring, der die durch R⁶ und R⁷ dargestellten Substituenten trägt, in 4-Stellung der Benzylgruppe ist, an die er gebunden ist,
und pharmazeutisch geeignete Salze und Ester dieser Verbindung.

3. Verbindung nach Anspruch 1, in der
R¹ eine Ethyl-, Propyl- oder Butylgruppe darstellt,
R² eine Isopropylgruppe oder eine t-Butylgruppe darstellt,
R³ ein Wasserstoffatom darstellt,
R⁴ ein Wasserstoffatom oder eine Methylgruppe darstellt,
R⁵ eine Gruppe der Formel -CONR⁸R⁹ darstellt, in der R⁸ und R⁹ gleich oder verschieden sind und jeweils ein Wasserstoffatom, eine Methylgruppe, eine Methoxycarbonylmethylgruppe, eine Ethoxycarbonylmethylgruppe oder eine Carboxymethylgruppe bedeuten,
R⁶ ein Wasserstoffatom darstellt,
R⁷ eine Carboxygruppe oder eine Tetrazol-5-yl-Gruppe in 2-Stellung des Benzolrings darstellt und
der Benzolring, der die durch R⁶ und R⁷ dargestellten Substituenten trägt, in 4-Stellung der Benzylgruppe ist, an die er gebunden ist,
und pharmazeutisch geeignete Salze und Ester dieser Verbindung.

4. 4-(1-Hydroxyethyl)-2-propyl-1-{4-[2-(tetrazol-5-yl)-phenyl)phenyl}methylimidazol-5-carbonsäure;
Pivaloyloxymethyl-4-(1-hydroxyethyl) -2-propyl-1-{4-[2-(tetrazol-5-yl)phenyl]phenyl}methylimidazol-5-carboxylat;
(5-Methyl-2-oxo-1,3-dioxolen-4-yl)methyl-4-(1-hydroxyethyl)-2-propyl-1-{4- [2-(tetrazol-5-yl)phenyl]-phenyl}methylimidazol-5-carboxylat
und pharmazeutisch geeignete Salze davon.

5. 4-(1-Hydroxyethyl)-2-butyl-1-{4-[2-(tetrazol-5-yl)-phenyl]phenyl}methylimidazol-5-carbonsäure;
Pivaloyloxymethyl-4-(1-hydroxyethyl)-2-butyl-1-{4-[2-(tetrazol-5-yl)phenyl]phenyl}methylimidazol-5-carboxylat;
(5-Methyl-2-oxo-1,3-dioxolen-4-yl)methyl-4-(1-hydroxyethyl)-2-butyl-1-{4-[2-(tetrazol-5-yl)phenyl]-phenyl}methylimidazol-5-carboxylat
und pharmazeutisch geeignete Salze davon.

6. 4-(1-Hydroxymethyl)-2-propyl-1-{4-[2-(tetrazol-5-yl) -phenyl]phenyl}methylimidazol-5-carbonsäure;
Pivaloyloxymethyl-4-(1-hydroxymethyl)-2-propyl-1-{4-[2-(tetrazol-5-yl)phenyl]phenyl}methylimidazol-5-carboxylat;
(5-Methyl-2-oxo-1, 3-dioxolen-4-yl)methyl-4-(1-hydroxymethyl)-2-propyl-1-{4-[2-(tetrazol-5-yl)phenyl] -phenyl}methylimidazol-5-carboxylat
und pharmazeutisch geeignete Salze davon.

7. 4-(1-Hydroxymethyl)-2-butyl-1-{4-[2-(tetrazol-5-yl) -phenyl]phenyl}methylimidazol-5-carbonsäure;
Pivaloyloxymethyl-4-(1-hydroxymethyl)-2-butyl-1-{4-[2-(tetrazol-5-yl)phenyl]phenyl}methylimidazol-5-carboxylat;
(5-Methyl-2-oxo-1,3-dioxolen-4-yl)methyl-4-(1-hydroxymethyl)-2-butyl-1-{4- [2-(tetrazol-5-yl)phenyl] -phenyl}methylimidazol-5-carboxylat
und pharmazeutisch geeignete Salze davon.

8. Arzneimittelzusammensetzung zur Behandlung oder Prophylaxe von Bluthochdruck, die ein antihypertensives Mittel im Gemisch mit einem pharmazeutisch geeigneten Träger oder Verdünnungsmittel enthält, wobei das antihypertensive Mittel mindestens eine Verbindung der Formel (I) oder ein pharmazeutisch geeignetes Salz oder ein pharmazeutisch geeigneter Ester davon gemäß einem der Ansprüche 1 bis 7 ist.

9. Verbindung der Formel (I) und pharmazeutisch geeignete Salze und Ester davon gemäß einem der Ansprüche 1 bis 7 zur therapeutischen Verwendung.

10. Verwendung von Verbindungen der Formel (I) und pharmazeutisch geeigneter Salze und Ester davon gemäß einem der Ansprüche 1 bis 7 zur Herstellung eines Arzneimittels zur Behandlung oder Prophylaxe von Bluthochdruck.

11. Verfahren zur Herstellung einer Verbindung nach einem der Ansprüche 1 bis 7, welches folgende Stufen umfaßt:
Umsetzen einer Verbindung der Formel (II): 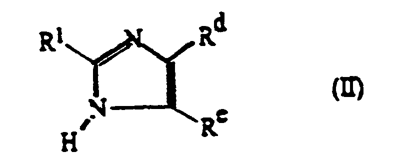 [in der:
R¹ wie vorstehend definiert ist und R^{d} eine Gruppe der Formel 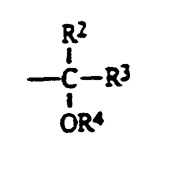 darstellt, worin R², R³ und R⁴ wie in Anspruch 1 definiert sind,
oder R^{d} eine Gruppe der Formel -COOR^{f} darstellt, worin R^{f} eine Carboxy-Schutzgruppe bedeutet, R^{d} eine Gruppe der Formel -COR² bedeutet, worin R² wie vorstehend definiert ist oder R^{d} eine Cyangruppe darstellt und
R^{e} eine Cyangruppe, eine Carboxygruppe oder eine Gruppe der Formel -COOR^{f} darstellt, worin R^{f} wie vorstehend definiert ist,
mit einer Verbindung der Formel (III) 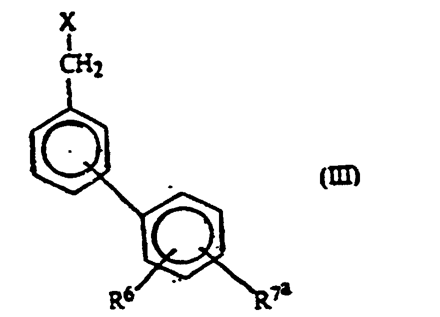 in der R⁶ wie vorstehend definiert ist, R^{7a} eine geschützte Carboxygruppe, eine Cyangruppe, eine geschützte Tetrazol-5-yl-Gruppe, eine Carbamoylgruppe oder eine Alkylcarbamoylgruppe darstellt und X ein Halogenatom bedeutet,
unter Bildung einer Verbindung der Formel (IV) : 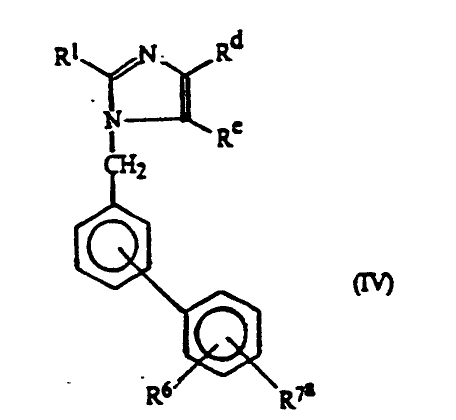 worin R^{d}, R^{e}, R¹, R⁶ und R^{7a} wie vorstehend definiert sind, und
Entfernen der Schutzgruppen in beliebiger Reihenfolge,
und erforderlichenfalls Umwandeln der Gruppe R^{d} in eine Gruppe der Formel 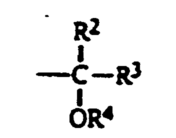 worin R², R³ und R⁴ wie vorstehend definiert sind,
und erforderlichenfalls Umwandeln der Gruppe R^{e} in eine Gruppe R⁵, Umwandeln der Gruppe R^{7a} in eine Gruppe R⁷ oder Alkylieren oder Acylieren einer Hydroxygruppe in R⁴, unter Bildung einer Verbindung der Formel (I), und
gegebenenfalls Salzbildung oder Verestern des Produkts.

## Patentansprüche (Patentansprüche für folgende(n) Vertragsstaat(en): GR, ES)

1. Verfahren zur Herstellung einer Verbindung der Formel (I): 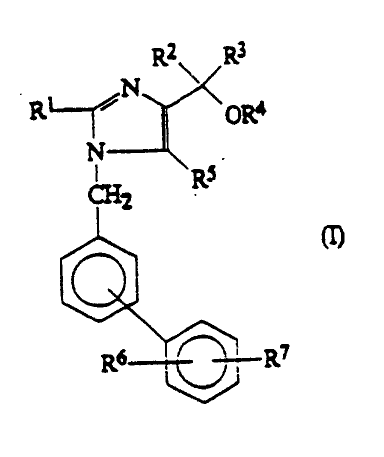 in der
R¹ eine Alkylgruppe. mit 2 bis 5 Kohlenstoffatomen darstellt,
R² und R³ jeweils ein Wasserstoffatom bedeuten oder eines von R² und R³ ein Wasserstoffatom darstellt, und das andere eine Alkylgruppe mit 1 bis 4 Kohlenstoffatomen darstellt,
R⁴ ein Wasserstoffatom, eine Methylgruppe, eine Ethylgruppe oder eine Alkanoylgruppe mit 1 bis 5 Kohlenstoffatomen darstellt,
R⁵ eine Gruppe der Formel -COOR^{5a} oder eine Gruppe der Formel -CONR⁸R⁹ darstellt, in der
R^{5a} darstellt:
ein Wasserstoffatom,
eine Methyl-, Ethyl- oder Benzylgruppe,
eine Alkanoyloxymethylgruppe, in der der Alkanoylteil 1 bis 5 Kohlenstoffatome aufweist,
eine 1-(Alkanoyloxy)-ethylgruppe, in der der Alkanoylteil 1 bis 5 Kohlenstoffatome aufweist,
eine Alkoxycarbonyloxymethylgruppe, in der der Alkoxyteil 1 bis 4 Kohlenstoffatome aufweist,
eine 1-(Alkoxycarbonyloxy) -ethylgruppe, in der der Alkoxyteil 1 bis 4 Kohlenstoffatome aufweist,
eine Cycloalkanoyloxyalkylgruppe, in der der Cycloalkylteil 5 oder 6 Kohlenstoffatome hat und der Alkylteil 1 oder 2 Kohlenstoffatome hat,
eine Cycloalkoxycarbonyloxyalkylgruppe, in der der Cycloalkylteil 5 oder 6 Kohlenstoffatome hat und der Alkylteil 1 oder 2 Kohlenstoffatome hat,
eine [5-(Phenyl- oder Methyl-)-2-oxo-1,3-dioxolen-4-yl]-methylgruppe oder eine Phthalidylgruppe,
R⁸ und R⁹ gleich oder verschieden sind und jeweils ein Wasserstoffatom, eine Methylgruppe, eine Ethylgruppe, eine Methoxycarbonylmethylgruppe, eine Ethoxycarbonylmethylgruppe oder eine Carboxymethylgruppe darstellen oder R⁸ und R⁹ zusammen eine Tetramethylen-, Pentamethylen-, 1-Carboxytetramethylen oder 1-Carboxypentamethylengruppe darstellen,
R⁶ ein Wasserstoffatom darstellt oder eine Methylgruppe, eine Methoxygruppe, ein Fluoratom oder ein Chloratom in 6-Stellung des Benzolrings darstellt,
R⁷ eine Carboxygruppe oder eine Tetrazol-5-yl-Gruppe in 2-Stellung des Benzolrings darstellt, und
der Benzolring, welcher die durch R⁶ und R⁷ dargestellten Substituenten trägt, in 4-Stellung der Benzylgruppe ist, an die er gebunden ist,
und von pharmazeutisch geeigneten Salzen und Estern dieser Verbindung,
welches folgende Stufen umfaßt:
Umsetzen einer Verbindung der Formel (II); 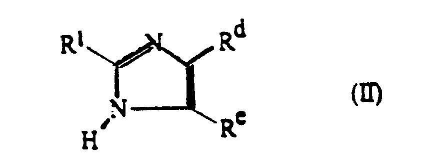 [in der:
R¹ wie vorstehend definiert ist und R^{d} eine Gruppe der Formel 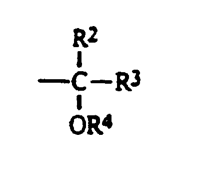 darstellt, worin R², R³ und R⁴ wie in Anspruch 1 definiert sind,
oder R^{d} eine Gruppe der Formel -COOR^{f} darstellt, worin R^{f} eine Carboxy-Schutzgruppe bedeutet, R^{d} eine Gruppe der Formel -COR² bedeutet, worin R² wie vorstehend definiert ist oder R^{d} eine Cyangruppe darstellt und
R^{e} eine Cyangruppe, eine Carboxygruppe oder eine Gruppe der Formel -COOR^{f} darstellt, worin R^{f} wie vorstehend definiert ist,
mit einer Verbindung der Formel (III) 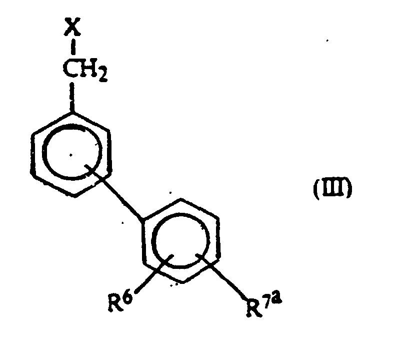 in der R⁶ wie vorstehend definiert ist, R^{7a} eine geschützte Carboxygruppe, eine Cyangruppe, eine geschützte Tetrazol-5-yl-Gruppe, eine Carbamoylgruppe oder eine Alkylcarbamoylgruppe darstellt und X ein Halogenatom bedeutet,
unter Bildung einer Verbindung der Formel (IV) : 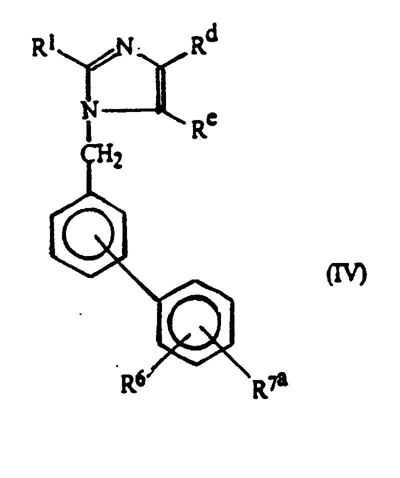 worin R^{d}, R^{e}, R¹, R⁶ und R^{7a} wie vorstehend definiert sind, und Entfernen der Schutzgruppen in beliebiger Reihenfolge, und erforderlichenfalls Umwandeln der Gruppe R^{d} in eine Gruppe der Formel 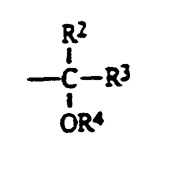 worin R², R³ und R⁴ wie vorstehend definiert sind,
und erforderlichenfalls Umwandeln der Gruppe R^{e} in eine Gruppe R⁵, Umwandeln der Gruppe R^{7a} in eine Gruppe R⁷ oder Alkylieren oder Acylieren einer Hydroxygruppe in R⁴, unter Bildung einer Verbindung der Formel (I), und
gegebenenfalls Salzbildung oder Verestern des Produkts.

2. Verfahren nach Anspruch 1, bei dem die Reagenzien und Reaktionsbedingungen so gewählt werden, daß eine Verbindung der Formel (I) oder ein Salz oder Ester davon gebildet wird, worin
R¹ eine Ethyl-, Propyl- oder Butylgruppe darstellt,
R² ein Wasserstoffatom oder eine Methylgruppe darstellt,
R³ ein Wasserstoffatom darstellt,
R⁴ ein Wasserstoffatom oder eine Methylgruppe darstellt,
R⁵ eine Gruppe der Formel -COOR^{5a} bedeutet, in welcher R^{5a} ein Wasserstoffatom, eine Pivaloyloxymethylgruppe, eine Ethoxycarbonyloxymethylgruppe, eine 1-(Ethoxycarbonyloxy) -ethylgruppe, eine Isopropoxycarbonyloxymethylgruppe, eine 1-(Isopropoxycarbonyloxy) -ethylgruppe, eine (5-Methyl-2-oxo-1,3-dioxolen-4-yl)methylgruppe oder eine Phthalidylgruppe darstellt,
R⁶ ein Wasserstoffatom darstellt,
R⁷ eine Carboxygruppe oder eine Tetrazol-5-ylgruppe in 2-Stellung des Benzolrings darstellt und
der Benzolring, der die durch R⁶ und R⁷ dargestellten Substituenten trägt, in 4-Stellung der Benzylgruppe ist, an die er gebunden ist.

3. Verfahren nach Anspruch 1, bei dem die Reagenzien und Reaktionsbedingungen so gewählt werden, daß eine Verbindung der Formel (I) oder ein Salz oder Ester davon gebildet wird, worin
R¹ eine Ethyl-, Propyl- oder Butylgruppe darstellt,
R² eine Isopropylgruppe oder eine t-Butylgruppe darstellt,
R³ ein Wasserstoffatom darstellt,
R⁴ ein Wasserstoffatom oder eine Methylgruppe darstellt,
R⁵ eine Gruppe der Formel -CONR⁸R⁹ darstellt, in der R⁸ und R⁹ gleich oder verschieden sind und jeweils ein Wasserstoffatom, eine Methylgruppe, eine Methoxycarbonylmethylgruppe, eine Ethoxycarbonylmethylgruppe oder eine Carboxymethylgruppe bedeuten,
R⁶ ein Wasserstoffatom darstellt,
R⁷ eine Carboxygruppe oder eine Tetrazol-5-yl-Gruppe in 2-Stellung des Benzolrings darstellt und
der Benzolring, der die durch R⁶ und R⁷ dargestellten Substituenten trägt, in 4-Stellung der Benzylgruppe ist, an die er gebunden ist.

4. Verfahren nach Anspruch 1, bei dem die Reagenzien und Reaktionsbedingungen so gewählt werden, daß hergestellt wird:
4-(1-Hydroxyethyl)-2-propyl-1-{4-[2-(tetrazol-5-yl)-phenyl]phenyl}methylimidazol-5-carbonsäure;
Pivaloyloxymethyl-4-(1-hydroxyethyl)-2-propyl-1-{4-[2-(tetrazol-5-yl)phenyl]phenyl}methylimidazol-5-carboxylat;
(5-Methyl-2-oxo-1,3-dioxolen-4-yl)methyl-4-(1-hydroxyethyl)-2-propyl-1-{4-[2-(tetrazol-5-yl)phenyl]-phenyl}methylimidazol-5-carboxylat
oder ein pharmazeutisch geeignetes Salz davon.

5. Verfahren nach Anspruch 1, bei dem die Reagenzien und Reaktionsbedingungen so gewählt werden, daß hergestellt wird:
4-(1-Hydroxyethyl)-2-butyl-1-{4-[2-(tetrazol-5-yl)-phenyl]-phenyl}methylimidazol-5-carbonsäure;
Pivaloyloxymethyl-4-(1-hydroxyethyl)-2-butyl-1-{4-[2-(tetrazol-5-yl)phenyl]phenyl}methylimidazol-5-carboxylat;
(5-Methyl-2-oxo-1,3-dioxolen-4-yl)methyl-4-(1-hydroxyethyl)-2-butyl-1-{4-[2-(tetrazol-5-yl)phenyl]-phenyl}methylimidazol-5-carboxylat
und pharmazeutisch geeignete Salze davon.

6. Verfahren nach Anspruch 1, bei dem die Reagenzien und Reaktionsbedingungen so gewählt werden, daß hergestellt wird:
4-(1-Hydroxymethyl)-2-propyl-1-{4-[2-(tetrazol-5-yl)-phenyl]phenyl}methylimidazol-5-carbonsäure;
Pivaloyloxymethyl-4-(1-hydroxymethyl)-2-propyl-1-{4-[2-(tetrazol-5-yl)phenyl]phenyl}methylimidazol-5-carboxylat;
(5-Methyl-2-oxo-1,3-dioxolen-4-yl)methyl-4-(1-hydroxymethyl)-2-propyl-1-{4-[2-(tetrazol-5-yl)phenyl]-phenyl}methylimidazol-5-carboxylat
und pharmazeutisch geeignete Salze davon.

7. Verfahren nach Anspruch 1, bei dem die Reagenzien und Reaktionsbedingungen so gewählt werden, daß hergestellt wird:
4-(1-Hydroxymethyl)-2-butyl-1-{4-[2-(tetrazol-5-yl)-phenyl]-phenyl}methylimidazol-5-carbonsäure;
Pivaloyloxymethyl-4-(1-hydroxymethyl)-2-butyl-1-{4-[2-(tetrazol-5-yl)phenyl]phenyl}methylimidazol-5-carboxylat;
(5-Methyl-2-oxo-1, 3-dioxolen-4-yl)methyl-4-(1-hydroxymethyl)-2-butyl-1-{4-[2-(tetrazol-5-yl)phenyl]-phenyl}methylimidazol-5-carboxylat
und pharmazeutisch geeignete Salze davon.

8. verfahren zur Herstellung einer Arzneimittelzusammensetzung zur Behandlung oder Prophylaxe von Bluthochdruck, welches das Vermischen eines antihypertensiven Mittels mit einem pharmazeutisch geeigneten Träger oder Verdünnungsmittel umfaßt, wobei das antihypertensive Mittel mindestens eine Verbindung der Formel (I) oder ein pharmazeutisch geeignetes Salz oder ein pharmazeutisch geeigneter Ester davon gemäß der Definition in einem der Ansprüche 1 bis 7 ist.

9. Verwendung von Verbindungen der Formel (I) und pharmazeutisch geeigneter Salze und Ester davon gemäß der Definition in einem der Anspruche 1 bis 7 zur Herstellung eines Arzneimittels zur Behandlung oder Prophylaxe von Bluthochdruck.

## Revendications (Revendications pour l'(les) Etat(s) contractant(s) suivant(s): GB, DE, FR, IT, CH, DK, BE, NL, SE, LU, AT, MC, PT)

1. Composé de formule (I) : 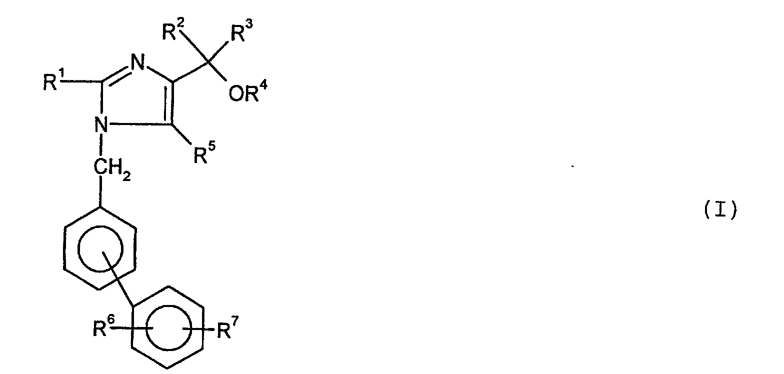 dans laquelle :
R¹ représente un groupe alkyle ayant de 2 à 5 atomes de carbone ;
R² et R³ représentent chacun un atome d'hydrogène ou bien l'un de R² et R³ représente un atome d'hydrogène et
l'autre représente un groupe alkyle ayant de 1 à 4 atomes de carbone ;
R⁴ représente un atome d'hydrogène, un groupe méthyle, un groupe éthyle ou un groupe alcanoyle ayant de 1 à 5 atomes de carbone ;
R⁵ représente un groupe de formule -COOR^{5a} ou un groupe de formule -CONR⁸R⁹, où :
R^{5a} représente :
un atome d'hydrogène,
un groupe méthyle, éthyle ou benzyle,
un groupe alcanoyloxyméthyle dans lequel le fragment alcanoyle a de 1 à 5 atomes de carbone,
un groupe 1-(alcanoyloxy)éthyle dans lequel le fragment alcanoyle a de 1 à 5 atomes de carbone,
un groupe alcoxycarbonyloxyméthyle dans lequel le fragment alcoxy a de 1 à 4 atomes de carbone,
un groupe 1-(alcoxycarbonyloxy)éthyle dans lequel le fragment alcoxy a de 1 à 4 atomes de carbone,
un groupe cycloalcanoyloxyalkyle dans lequel le fragment cycloalkyle a 5 ou 6 atomes de carbone et le fragment alkyle a 1 ou 2 atomes de carbone,
un groupe cycloalcoxycarbonyloxyalkyle dans lequel le fragment cycloalkyle a 5 ou 6 atomes de carbone et le fragment alkyle a 1 ou 2 atomes de carbone,
un groupe [5-(phényl- ou méthyl-)-2-oxo-1,3-dioxolén-4-yl]méthyle, ou
un groupe phtalidyle ;
R⁸ et R⁹ sont identiques ou différents et représentent chacun un atome d'hydrogène, un groupe méthyle, un groupe éthyle, un groupe méthoxycarbonylméthyle, un groupe éthoxycarbonylméthyle ou un groupe carboxyméthyle ; ou bien R⁸ et R⁹ représentent ensemble un groupe tétraméthylène, pentaméthylène, 1-carboxytétraméthylène ou 1-carboxypentaméthylène;
R⁶ représente un atome d'hydrogène, ou représente un groupe méthyle, un groupe méthoxy, un atome de fluor ou un atome de chlore en position 6 du cycle benzène ;
R⁷ représente un groupe carboxy ou un groupe tétrazol-5-yle en position 2 du cycle benzène ; et
le cycle benzène qui porte les substituants représentés par R⁶ ou R⁷ est en position 4 du groupe benzyle auquel il est rattaché ;
et ses sels et esters acceptables en pharmacie.

2. Composé selon la revendication 1, dans lequel :
R¹ représente un groupe éthyle, propyle ou butyle ;
R² représente un atome d'hydrogène ou un groupe méthyle ;
R³ représente un atome d'hydrogène ;
R⁴ représente un atome d'hydrogène ou un groupe méthyle ;
R⁵ représente un groupe de formule -COOR^{5a} dans laquelle R^{5a} représente un atome d'hydrogène, un groupe pivaloyloxyméthyle, un groupe éthoxycarbonyloxyméthyle,
un groupe 1-(éthoxycarbonyloxy)éthyle, un groupe isopropoxycarbonyloxyméthyle, un groupe 1-(isopropoxycarbonyloxy)éthyle, un groupe (5-méthyl-2-oxo-1,3-dioxolén-4-yl)méthyle ou un groupe phtalidyle ;
R⁶ représente un atome d'hydrogène ;
R⁷ représente un groupe carboxy ou un groupe tétrazol-5-yle en position 2 du cycle benzène ; et
le cycle benzène qui porte les substituants représentés par R⁶ et R⁷ est en position 4 du groupe benzyle auquel il est rattaché,
et ses sels et esters acceptables en pharmacie.

3. Composé selon la revendication 1, dans lequel :
R¹ représente un groupe éthyle, propyle ou butyle ;
R² représente un groupe isopropyle ou un groupe t-butyle ;
R³ représente un atome d'hydrogène ;
R⁴ représente un atome d'hydrogène ou un groupe méthyle ;
R⁵ représente un groupe de formule -CONR⁸R⁹ dans laquelle R⁸ et R⁹ sont identiques ou différents et représentent chacun un atome d'hydrogène, un groupe méthyle, un groupe méthoxycarbonylméthyle, un groupe éthoxycarbonylméthyle ou un groupe carboxyméthyle ;
R⁶ représente un atome d'hydrogène ;
R⁷ représente un groupe carboxy ou un groupe tétrazol-5-yle en position 2 du cycle benzène ; et
le cycle benzène qui porte les substituants représentés par R⁶ et R⁷ est en position 4 du groupe benzyle auquel il est rattaché,
et ses sels et esters acceptables en pharmacie.

4. Acide 4-(1-hydroxyéthyl)-2-propyl-1-{4-[2-(tétrazol-5-yl)phényl]phényl}méthylimidazole-5-carboxylique ;
4-(1-hydroxyéthyl)-2-propyl-1-{4-[2-(tétrazol-5-yl)phényl]phényl}méthylimidazole-5-carboxylate de pivaloyloxyméthyle ;
4-(1-hydroxyéthyl)-2-propyl-1-{4-[2-(tétrazol-5-yl)phényl]phényl}méthylimidazole-5-carboxylate de (5-méthyl-2-oxo-1,3-dioxolén-4-yl)méthyle;
et leurs sels acceptables en pharmacie.

5. Acide 4-(1-hydroxyéthyl)-2-butyl-1-{4-[2-(tétrazol-5-yl)phényl]phényl}méthylimidazole-5-carboxylique;
4-(1-hydroxyéthyl)-2-butyl-1-{4-[2-(tétrazol-5-yl)phényl]phényl}méthylimidazole-5-carboxylate de pivaloyloxyméthyle ;
4-(1-hydroxyéthyl)-2-butyl-1-{4-[2-(tétrazol-5-yl)phényl]phényl}méthylimidazole-5-carboxylate de (5-méthyl-2-oxo-1,3-dioxolén-4-yl)méthyle ;
et leurs sels acceptables en pharmacie.

6. Acide 4-(1-hydroxyméthyl)-2-propyl-1-{4-[2-(tétrazol-5-yl)phényl]phényl}méthylimidazole-5-carboxylique;
4-(1-hydroxyméthyl)-2-propyl-1-{4-[2-(tétrazol-5-yl)phényl]phényl}méthylimidazole-5-carboxylate de pivaloyloxyméthyle ;
4-(1-hydroxyméthyl)-2-propyl-1-{4-[2-(tétrazol-5-yl)phényl]phényl}méthylimidazole-5-carboxylate de (5-méthyl-2-oxo-1,3-dioxolén-4-yl)méthyle;
et leurs sels acceptables en pharmacie.

7. Acide 4-(1-hydroxyméthyl)-2-butyl-1-{4-[2-(tétrazol-5-yl)phényl]phényl}méthylimidazole-5-carboxylique ;
4-(1-hydroxyméthyl)-2-butyl-1-{4-[2-(tétrazol-5-yl)phényl]phényl}méthylimidazole-5-carboxylate de pivaloyloxyméthyle ;
4-(1-hydroxyméthyl)-2-butyl-1-{4-[2-(tétrazol-5-yl)phényl]phényl}méthylimidazole-5-carboxylate de (5-méthyl-2-oxo-1,3-dioxolén-4-yl)méthyle ;
et leurs sels acceptables en pharmacie.

8. Composition pharmaceutique pour le traitement ou la prophylaxie de l'hypertension, qui comprend un agent antihypertenseur en mélange avec un véhicule ou diluant acceptable en pharmacie, dans laquelle l'agent antihypertenseur est au moins un composé de formule (I) ou un de ses sels ou esters acceptables en pharmacie, tel que revendiqué dans l'une quelconque des revendications 1 à 7.

9. Composés de formule (I) et leurs sels et esters acceptables en pharmacie, tels que revendiqués dans l'une quelconque des revendications 1 à 7, à utiliser en thérapie.

10. Utilisation des composés de formule (I) et de leurs sels et esters acceptables en pharmacie, tels que revendiqués dans l'une quelconque des revendications 1 à 7, pour la fabrication d'un médicament destiné au traitement ou à la prophylaxie de l'hypertension.

11. Procédé pour préparer un composé selon l'une quelconque des revendications 1 à 7, qui comprend les étapes consistant à :
faire réagir un composé de formule (II) : 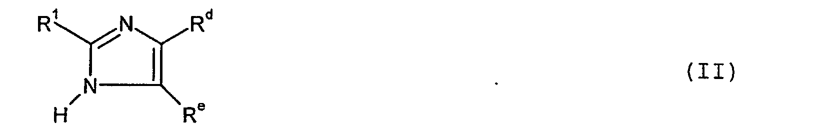 [dans laquelle :
R¹ est tel que défini ci-dessus et R^{d} représente un groupe de formule : 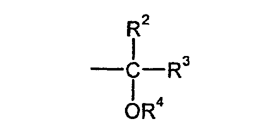 dans laquelle R², R³ et R⁴ sont tels que définis dans la revendication 1,
ou bien R^{d} représente un groupe de formule -COOR^{f} dans laquelle R^{f} représente un groupe carboxy-protecteur, R^{d} représente un groupe de formule -COR² où R² est tel que défini ci-dessus, ou bien R^{d} représente un groupe cyano ; et
R^{e} représente un groupe cyano, un groupe carboxy ou un groupe de formule -COOR^{f} dans laquelle R^{f} est tel que défini ci-dessus ;
avec un composé de formule (III) : 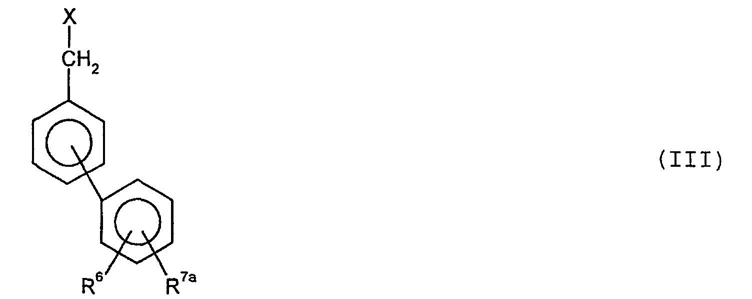 dans laquelle : R⁶ est tel que défini ci-dessus ; R^{7a} représente un groupe carboxy protégé, un groupe cyano, un groupe tétrazol-5-yle protégé, un groupe carbamoyle ou un groupe alkylcarbamoyle ; et X représente un atome d'halogène ;
pour donner un composé de formule (IV) : 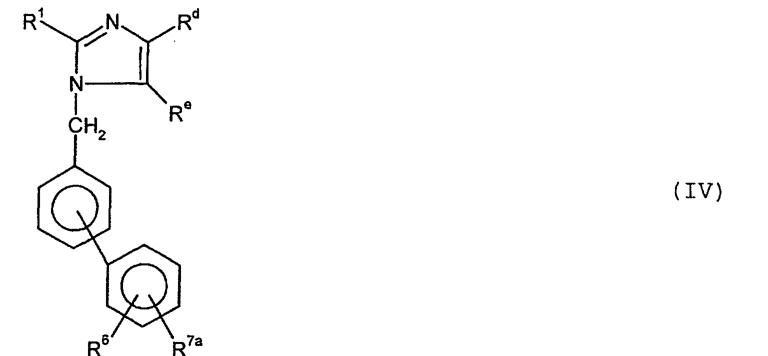 dans laquelle R^{d}, R^{e}, R¹, R⁶ et R^{7a} sont tels que définis ci-dessus ; et
dans un ordre quelconque, éliminer les groupes protecteurs et, si nécessaire, convertir ledit groupe R^{d} en un groupe de formule : 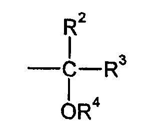 dans laquelle R², R³ et R⁴ sont tels que définis ci-dessus,
et, si nécessaire, convertir ledit groupe R^{e} en un groupe R⁵, convertir ledit groupe R^{7a} en un groupe R⁷, ou alkyler ou acyler un groupe hydroxy dans R⁴, pour donner un composé de formule (I) ; et éventuellement, salifier ou estérifier le produit.

## Revendications (Revendications pour l'(les) Etat(s) contractant(s) suivant(s): ES, GR)

1. Procédé pour préparer un composé de formule (I) : 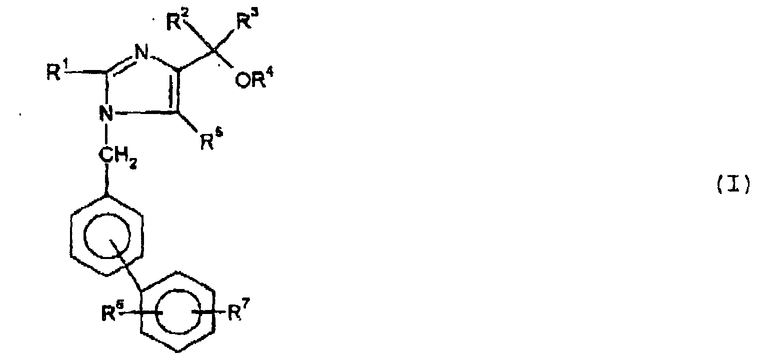 dans laquelle :
R¹ représente un groupe alkyle ayant de 2 à 5 atomes de carbone ;
R² et R³ représentent chacun un atome d'hydrogène ou bien l'un de R² et R³ représente un atome d'hydrogène et l'autre représente un groupe alkyle ayant de 1 à 4 atomes de carbone ;
R⁴ représente un atome d'hydrogène, un groupe méthyle, un groupe éthyle ou un groupe alcanoyle ayant de 1 à 5 atomes de carbone ;
R⁵ représente un groupe de formule -COOR^{5a} ou un groupe de formule -CONR⁸R⁹, où :
R^{5a} représente :
un atome d'hydrogène,
un groupe méthyle, éthyle ou benzyle,
un groupe alcanoyloxyméthyle dans lequel le fragment alcanoyle a de 1 à 5 atomes de carbone,
un groupe 1-(alcanoyloxy)éthyle dans lequel le fragment alcanoyle a de 1 à 5 atomes de carbone,
un groupe alcoxycarbonyloxyméthyle dans lequel le fragment alcoxy a de 1 à 4 atomes de carbone,
un groupe 1-(alcoxycarbonyloxy)éthyle dans lequel le fragment alcoxy a de 1 à 4 atomes de carbone,
un groupe cycloalcanoyloxyalkyle dans lequel le fragment cycloalkyle a 5 ou 6 atomes de carbone et le fragment alkyle a 1 ou 2 atomes de carbone,
un groupe cycloalcoxycarbonyloxyalkyle dans lequel le fragment cycloalkyle a 5 ou 6 atomes de carbone et le fragment alkyle a 1 ou 2 atomes de carbone,
un groupe [5-(phényl- ou méthyl-)-2-oxo-1,3-dioxolén-4-yl]méthyle, ou
un groupe phtalidyle ;
R⁸ et R⁹ sont identiques ou différents et représentent chacun un atome d'hydrogène, un groupe méthyle, un groupe éthyle, un groupe méthoxycarbonylméthyle, un groupe éthoxycarbonylméthyle ou un groupe carboxyméthyle ; ou bien R⁸ et R⁹ représentent ensemble un groupe tétraméthylène, pentaméthylène, 1-carboxytétraméthylène ou 1-carboxypentaméthylène ;
R⁶ représente un atome d'hydrogène, ou représente un groupe méthyle, un groupe méthoxy, un atome de fluor ou un atome de chlore en position 6 du cycle benzène ;
R⁷ représente un groupe carboxy ou un groupe tétrazol-5-yle en position 2 du cycle benzène ; et
le cycle benzène qui porte les substituants représentés par R⁶ ou R⁷ est en position 4 du groupe benzyle auquel il est rattaché ;
et ses sels et esters acceptables en pharmacie ;
lequel procédé comprend les étapes consistant à : faire réagir un composé de formule (II) : 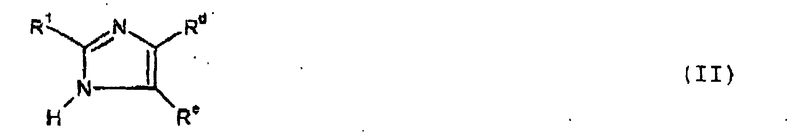 [dans laquelle :
R¹ est tel que défini ci-dessus et R^{d} représente un groupe de formule : 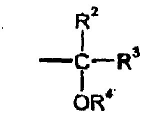 dans laquelle R², R³ et R⁴ sont tels que définis dans la revendication 1,
ou bien R^{d} représente un groupe de formule -COOR^{f} dans laquelle R^{f} représente *un* groupe carboxy-protecteur, R^{d} représente un groupe de formule -COR² où R² est tel que défini ci-dessus, ou bien R^{d} représente un groupe cyano ; et
R^{e} représente un groupe cyano, un groupe carboxy ou un groupe de formule -COOR^{f} dans laquelle R^{f} est tel que défini ci-dessus ;
avec un composé de formule (III) : 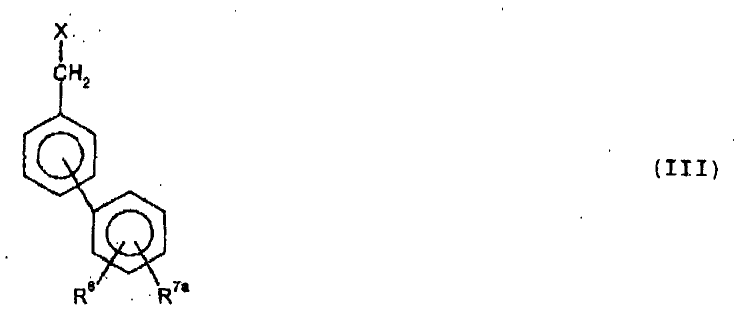 dans laquelle : R⁶ est tel que défini ci-dessus ; R^{7a} représente un groupe carboxy protégé, un groupe cyano, un groupe tétrazol-5-yle protégé, un groupe carbamoyle ou un groupe alkylcarbamoyle ; et X représente un atome d'halogène ;
pour donner un composé de formule (IV) : 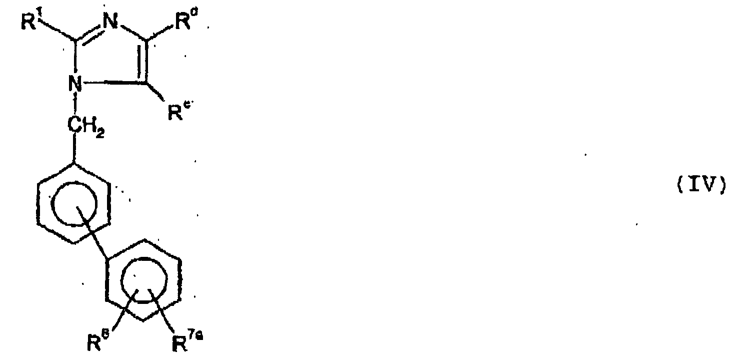 dans laquelle R^{d}, R^{e}, R¹, R⁶ et R^{7a} sont tels que définis ci-dessus : et
dans un ordre quelconque, éliminer les groupes protecteurs et, si nécessaire, convertir ledit groupe R^{d} en un groupe de formule : 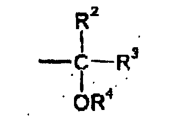 dans laquelle R², R³ et R⁴ sont tels que définis ci-dessus,
et, si nécessaire, convertir ledit groupe R^{e} en un groupe R⁵, convertir ledit groupe R^{7a} en un groupe R⁷, ou alkyler ou acyler un groupe hydroxy dans R⁴, pour donner un composé de formule (I) ; et
éventuellement, salifier ou estérifier le produit.

2. Procédé selon la revendication 1, dans lequel les réactifs et conditions réactionnelles sont choisis de façon à préparer un composé de formule (I) ou un de ses sels ou esters, dans lequel :
R¹ représente un groupe éthyle, propyle ou butyle ;
R² représente un atome d'hydrogène ou un groupe méthyle ;
R³ représente un atome d'hydrogène ;
R⁴ représente un atome d'hydrogène ou un groupe méthyle ;
R⁵ représente un groupe de formule -COOR^{5a} dans laquelle R^{5a} représente un atome d'hydrogène, un groupe pivaloyloxyméthyle, un groupe éthoxycarbonyloxyméthyle, un groupe 1-(éthoxycarbonyloxy)éthyle, un groupe isopropoxycarbonyloxyméthyle, un groupe 1-(isopropoxycarbonyloxy)éthyle, un groupe (5-méthyl-2-oxo-1,3-dioxolén-4-yl)méthyle ou un groupe phtalidyle ;
R⁶ représente un atome d'hydrogène ;
R⁷ représente un groupe carboxy ou un groupe tétrazol-5-yle en position 2 du cycle benzène ; et
le cycle benzène qui porte les substituants représentés par R⁶ et R⁷ est en position 4 du groupe benzyle auquel il est rattaché.

3. Procédé selon la revendication 1, dans lequel les réactifs et conditions réactionnelles sont choisis de façon à préparer un composé de formule (I) ou un de ses sels ou esters, dans lequel :
R¹ représente un groupe éthyle, propyle ou butyle ;
R² représente un groupe isopropyle ou un groupe t-butyle ;
R³ représente un atome d'hydrogène ;
R⁴ représente un, atome d'hydrogène ou un groupe méthyle ;
R⁵ représente un groupe de formule -CONR⁸R⁹ dans laquelle R⁸ et R⁹ sont identiques ou différents et représentent chacun un atome d'hydrogène, un groupe méthyle, un groupe méthoxycarbonylméthyle, un groupe éthoxycarbonylméthyle ou un groupe carboxyméthyle ;
R⁶ représente un atome d'hydrogène ;
R⁷ représente un groupe carboxy ou un groupe tétrazol-5-yle en position 2 du cycle benzène ; et
le cycle benzène qui porte les substituants représentés par R⁶ et R⁷ est en position 4 du groupe benzyle auquel il est rattaché.

4. Procédé selon la revendication 1, dans lequel les réactifs et conditions réactionnelles sont choisis de façon à préparer l'un des composés suivants :
acide 4-(1-hydroxyéthyl)-2-propyl-1-{4-[2-(tétrazol-5-yl)phényl]phényl}méthylimidazole-5-carboxylique ;
4-(1-hydroxyéthyl)-2-propyl-1-{4-[2-(tétrazol-5-yl)phényl]phényl}méthylimidazole-5-carboxylate de pivaloyloxyméthyle ;
4-(1-hydroxyéthyl)-2-propyl-1-{4-[2-(tétrazol-5-yl)phényl]phényl}méthylimidazole-5-carboxylate de (5-méthyl-2-oxo-1,3-dioxolén-4-yl)méthyle ;
ou un de leurs sels acceptables en pharmacie.

5. Procédé selon la revendication 1, dans lequel les réactifs et conditions réactionnelles sont choisis de façon à préparer l'un des composés suivants :
acide 4-(1-hydroxyéthyl)-2-butyl-1-{4-[2-(tétrazol-5-yl)phényl]phényl]méthylimidazole-5-carboxylique ;
4- (1-hydroxyéthyl)-2-butyl-1-{4-[2-(tétrazol-5-yl)phényl]phényl}méthylimidazole-5-carboxylate de pivaloyloxyméthyle ;
4- (1-hydroxyéthyl)-2-butyl-1-{4-[2-(tétrazol-5-yl)phényl]phényl}méthylimidazole-5-carboxylate de (5-méthyl-2-oxo-1,3-dioxolén-4-yl)méthyle ;
et leurs sels acceptables en pharmacie.

6. Procédé selon la revendication 1, dans lequel, les réactifs et conditions réactionnelles sont choisis de façon à préparer l'un des composés suivants :
acide 4-(1-hydroxyméthyl)-2-propyl-1-{4-[2-(tétrazol-5-yl)phényl]phényl}méthylimidazole-5-carboxylique ;
4-(1-hydroxyméthyl)-2-propyl-1-{4-[2-(tétrazol-5-yl) phényl]phényl}méthylimidazole-5-carboxylate de pivaloyloxyméthyle ;
4-(1-hydroxyméthyl)-2-propyl-1-{4-[2-(tétrazol-5-yl)phényl]phényl}méthylimidazole-5-carboxylate de (5-méthyl-2-oxo-1,3-dioxolén-4-yl)méthyle ;
et leurs sels acceptables en pharmacie.

7. Procédé selon la revendication 1, dans lequel les réactifs et conditions réactionnelles sont choisis de façon à préparer l'un des composés suivants :
acide 4-(1-hydroxyméthyl)-2-butyl-1-{4-[2-(tétrazol-5-yl)phényl]phényl}méthylimidazole-5-carboxylique ;
4-(1-hydroxyméthyl)-2-butyl-1-{4-[2-(tétrazol-5-yl)phényl]phényl}méthylimidazole-5-carboxylate de pivaloyloxyméthyle ;
4- (1-hydroxyméthyl)-2-butyl-1-{4-[2-(tétrazol-5-yl)phényl]phényl}méthylimidazole-5-carboxylate de (5-méthyl-2-oxo-1, 3-dioxolén-4-yl)méthyle ;
et leurs sels acceptables en pharmacie.

8. Procédé pour préparer une composition pharmaceutique pour le traitement où la prophylaxie de l'hypertension, qui comprend le mélange d'un agent antihypertenseur avec un véhicule ou diluant acceptable en pharmacie, dans laquelle l'agent antihypertenseur est au moins un composé de formule (I) ou un de ses sels ou esters acceptables en pharmacie, tel que revendiqué dans l'une quelconque des revendications 1 à 7.

9. Utilisation de composés de formule (I) et leurs sels et esters acceptables en pharmacie, tels que revendiqués dans l'une quelconque des revendications 1 à 7, pour la fabrication d'un médicament destiné au traitement ou à la prophylaxie de l'hypertension.
